(19) Europäisches Patentamt
European Patent Office
Office européen des brevets

(11) EP 1 297 834 A1

(12) **EUROPEAN PATENT APPLICATION**
published in accordance with Art. 158(3) EPC

(43) Date of publication:
**02.04.2003 Bulletin 2003/14**

(21) Application number: **01936939.6**

(22) Date of filing: **11.06.2001**

(51) Int Cl.7: **A61K 31/41**, A61K 31/42, A61K 31/422, A61K 31/44, A61K 31/4409, A61K 31/4433, A61K 31/4709, A61K 31/472, A61K 31/4965, A61K 31/497, A61K 31/505, A61K 31/506, A61P 31/18, A61P 43/00, C07B 61/00
// (C07D213/79, 217:18, 239:26, 241:12, 249:02, 401:06, 403:06, 405:06, 413:06, 417:06)

(86) International application number:
**PCT/JP01/04886**

(87) International publication number:
**WO 01/095905 (20.12.2001 Gazette 2001/51)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **14.06.2000 JP 2000178805**
**19.06.2000 JP 2000183173**

(71) Applicant: **SHIONOGI & CO., LTD.**
**Osaka-shi, Osaka 541-0045 (JP)**

(72) Inventors:
- **KIYAMA, Ryuichi, c/o Shionogi & Co. Ltd.**
  **Osaka-shi, Osaka 553-0002 (JP)**
- **KAWASUJI, Takashi, c/o Shionogi & Co. Ltd.**
  **Osaka-shi, Osaka 553-0002 (JP)**

(74) Representative: **VOSSIUS & PARTNER**
**Siebertstrasse 4**
**81675 München (DE)**

(54) **INHIBITOR FOR ENZYME HAVING TWO DIVALENT METAL IONS AS ACTIVE CENTERS**

(57) A pharmaceutical composition for use as an inhibitor of an enzyme having two divalent metal ions as an active center was found.

It is possible to inhibit the activity of an enzyme by a compound capable of chelating both of the two divalent metal ions.



Printed by Jouve, 75001 PARIS (FR)

## Description

TECHNICAL FIELD

The present invention relates to a pharmaceutical composition for use as an inhibitor of an enzyme, specifically to a pharmaceutical composition for use as an inhibitor of an enzyme having two divalent metal ions as an active center, and more specifically to a pharmaceutical composition for use as an inhibitor of enzymes which catalyze nucleic acid related reactions, a pharmaceutical composition for use as an integrase inhibitor and a pharmaceutical composition for use as an HIV integrase inhibitor.

BACKGROUND ART

At an early stage in searching for an enzyme inhibitor, a random screening method or a method for designing an inhibitor by utilizing the data relating to the 3-D structure of the enzyme is utilized. The significance especially of the latter method is increasing in these days as a result of the recent advance in the technologies of the structural biology (X-ray crystal structure analysis, NMR analysis and the like). However, when no 3-D structure of a target enzyme is characterized, there is practically no clue with regard to the structure to the discovery of its inhibitor and a random screening method is only way to search for the inhibitors.

On the other hand, some enzymes have common characteristics that two divalent metal ions are present as an active center. For example, enzymes which catalyze nucleic acid related reactions such as DNA polymerases and integrases are considered to have two divalent metal ions as an active center, and two metal ions were identified in the active center of an ASV integrase by X-ray crystal structure analysis (G. Bujacz, J. Biol. Chem., Vol.272, 18161 (1997), PDB Code: 1vsj, 1vsh). Recently, extensive attempts were made for treating diseases such as HIV by inhibiting the enzyme possessed by a virus. For example, an HIV integrase has extensively been reported with regard to its inhibitors, but no search for an inhibitor utilizing the structural data positively has been made.

Thus, as HIV integrase inhibitors, a compound represented by Formula:

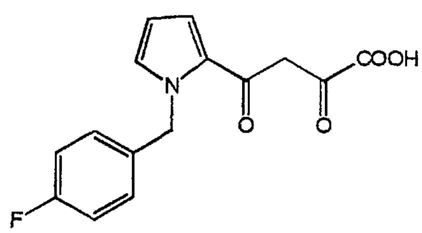

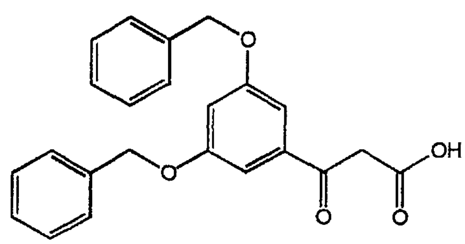

(WO99/62513, WO99/62897, WO99/62520, Science, Vol.287, 646-650, 1999), a compound represented by Formula:

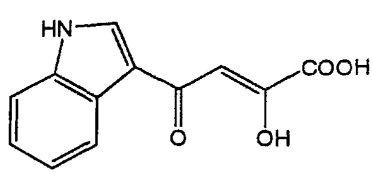

and a compound represented by Formula:

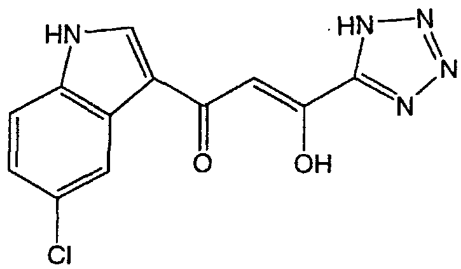

(WO99/50245) were known, but none of these references described the binding mode of each compound with an HIV integrase.

On the other hand, a reference Proc. Natl. Acad. Sci. USA, vol.96, 13040-13043 (1999) (hereinafter, referred to as Reference A) disclosed the crystal structure of an HIV integrase complexed with the compound (hereinafter, referred to as Compound A) which is one of the compounds described in WO99/50245 represented by Formula:

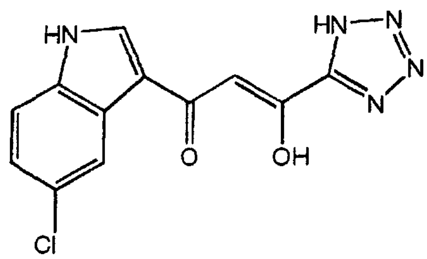

Reference A discloses that a hydrogen bond is formed between the oxygen atom in the 1,3-dioxo group in Compound A and the oxygen atom in the 152nd glutamic acid side chain which is one of the active centers of an HIV integrase, between the nitrogen atom in a tetrazolyl group of the compound and the nitrogen atom in the 156th lysine side chain, the nitrogen atom in the 159th lysine side chain, the oxygen atom in the 66th threonine side chain and the nitrogen atom in the 155th asparagine side chain in the enzyme, and between the nitrogen atom in the indolyl group in the compound and the nitrogen atom in the 148th glutamine side chain in the enzyme.

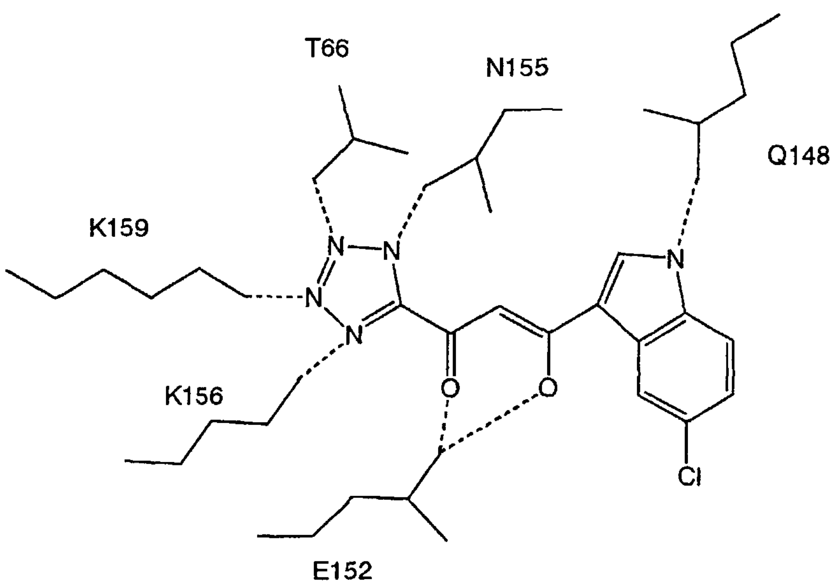

Reference A also disclosed that Compound A was bound to the HIV integrase while eliminating one of the two divalent metal ions possessed by an HIV integrase.

However, it involves several problems to adopt this binding mode as a true inhibitory mechanism.

An HIV integrase is believed to form a dimeric structure with the hydrophobic surfaces of the core domains facing each other and form a multimer structure with the active centers facing each other. The crystal in Reference A forms a single asymmetric unit in a configuration of three core domains (designated as A, B and C) aligning linearly.

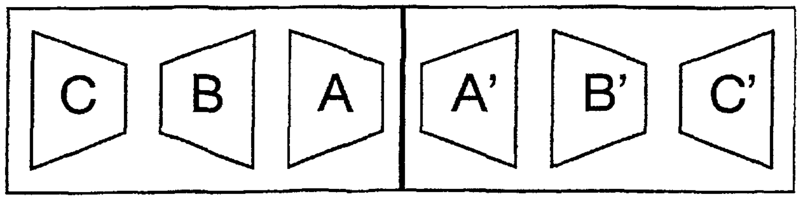


Among these, A and B are in the configuration in which the hydrophobic surfaces face each other while B and C are in the configuration in which the active centers face each other, possibly reflecting the actual multimerization state of the integrase. However, the described in Reference A was not found at the active centers between B and C, and was identified only between A and A' (present in the adjacent asymmetric unit). Since this contact surface between monomers A and A' is a multimerization site which is generated as a result of a crystallographic environment and which is not existing naturally in a solution, the binding mode disclosed in Reference A is difficult to be considered to reflect the actual binding mode between the HIV integrase and Compound A. Also because of the experimental fact that the binding mode disclosed there cannot explain the structure-activity relationship discussed below, this binding mode appears only in a certain crystal structure but was not considered to reflect the enzyme inhibitory mechanism under a physiological condition.

On the other hand, Polyhedron, 16, 1279, 1997 disclosed that a compound represented by Formula:

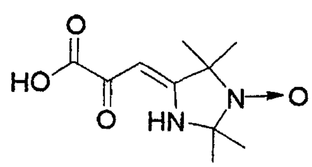

is bound to two copper ions ($Cu^{2+}$) in a manner described below, but this reference related to the analysis of the interaction between the compound and the metal ions with no discussion being made with regard to the binding mode between the compound and an enzyme.

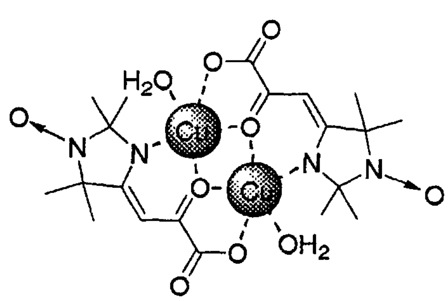

DISCLOSURE OF THE INVENTION

The present invention provides a pharmaceutical composition for use as an inhibitor of an enzyme, specifically a pharmaceutical composition for use as an inhibitor of an enzyme having two divalent metal ions as an active center, and more particularly a pharmaceutical composition for use as an inhibitor of enzymes which catalyze nucleic acid related reactions, an integrase inhibitor and an HIV integrase inhibitor.

The present inventors discovered, as detailed below, a pharmaceutical composition for use as an inhibitor of an enzyme having two divalent metal ions as an active center.

Thus, the present invention is:

(1) a pharmaceutical composition for use as an inhibitor of an enzyme having two divalent metal ions as an active center, which comprises a compound capable of chelating both of the two divalent metal ions at the same time, a prodrug thereof, a pharmaceutically acceptable salt thereof or a solvate thereof as an active ingredient;

(2) a pharmaceutical composition for use as an inhibitor according to the above-mentioned (1), wherein the compound is capable of chelating both of the two divalent metal ions present in the active center of an enzyme at the same time;

(3) a pharmaceutical composition for use as an inhibitor according to the above-mentioned (1), wherein the compound is capable of chelating both of the two divalent metal ions at the same time and has an inhibitory activity on an enzyme having two divalent metal ions as an active center;

(4) a pharmaceutical composition for use as an inhibitor according to any of the above-mentioned (1) to (3), wherein the compound has atom $A^1$ capable of coordinating to one divalent metal ion, atom $A^3$ capable of coordinating to the other divalent metal ion and atom $A^2$ capable of coordinating to the both divalent metal ions at the same time;

(5) a pharmaceutical composition for use as an inhibitor according to the above-mentioned (4), wherein atom A1, atom A2 and atom A3 are each independently, a nitrogen atom capable of coordinating to a divalent metal ion, oxygen atom capable of coordinating to a divalent metal ion or sulfur atom capable of coordinating to a divalent metal ion;

(6) a pharmaceutical composition for use as an inhibitor according to the above-mentioned (4) or (5), wherein the distance between atom $A^1$ and atom $A^2$ (distance $A^1$-$A^2$) is 2.4 to 3.6 angstrom, the distance between atom $A^2$ and atom $A^3$ (distance $A^2$-$A^3$) is 2.4 to 3.6 angstrom and the angle defined by atom $A^1$, atom $A^2$ and atom $A^3$ (angle $A^1$-$A^2$-$A^3$) is 110 to 180°;

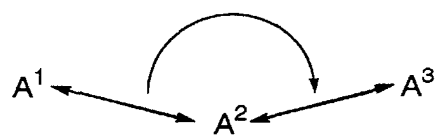


(7) a pharmaceutical composition for use as an inhibitor according to any of the above-mentioned (1) to (6), wherein the compound is capable of forming a 5- or 6-membered chelate ring by chelating one divalent metal ion and also capable of forming a 5- or 6-membered chelate ring by chelating the other divalent metal ion;

(8) a pharmaceutical composition for use as an inhibitor according to the above-mentioned (7), wherein the compound has a substructure represented by Formula (I):

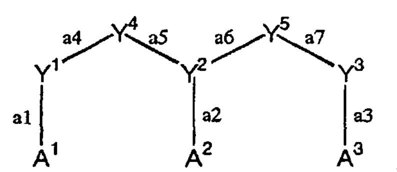

(I)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
atom $Y^4$ and atom $Y^5$ are each independently a carbon atom or nitrogen atom,
bond a1 to bond a7 are each independently a single bond or a double bond,
one of bond a2, bond a5 and bond a6 is a double bond, and the other two are single bonds,
bond a1 and bond a3 to bond a7 may each independently constitute a part of the ring,
provided that any adjacent bonds of bond a1 to bond a7 are not double bonds at the same time;

(9) a pharmaceutical composition for use as an inhibitor according to the above-mentioned (7), wherein the compound has a substructure represented by Formula (II):

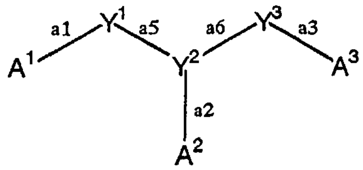

(II)

wherein atom $A^1$, atom $A^2$ and atom $A^3$ are each independently, a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
bond a1 to bond a3, bond a5 and bond a6 are each independently a single bond or a double bond,
one of bond a2, bond a5 and bond a6 is a double bond, and the other two are single bonds,
bond a1, bond a3, bond a5 and bond a6 may each independently constitute a part of the ring,
provided that any adjacent bonds of bond a1 to bond a3, bond a5 and bond a6 are not double bonds at the same time;
(10) a pharmaceutical composition for use as an inhibitor according to the above-mentioned (7), wherein the compound has a substructure represented by Formula (III):

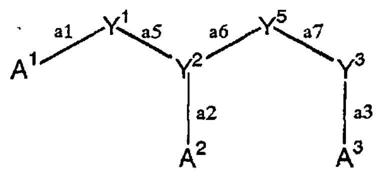

(III)

wherein atom $A^1$, atom $A^2$ and atom $A^3$ are each independently, a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
atom $Y^5$ is a carbon atom or nitrogen atom,
bond a1 to bond a3 and bond a5 to bond a7 are each independently a single bond or a double bond,
one of bond a2, bond a5 and bond a6 is a double bond, and the other two are single bonds,
bond a1, bond a3 and bond a5 to bond a7 may each independently constitute a part of the ring,
provided that any adjacent bonds of bond a1 to bond a3 and bond a5 to bond a7 are not double bonds at the same time;
(11) a pharmaceutical composition for use as an inhibitor according to the above-mentioned (7), wherein the compound has a substructure represented by Formula (IV):

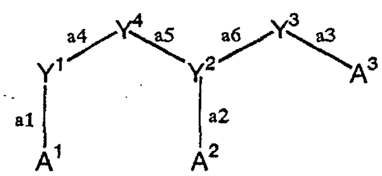

(IV)

wherein atom $A^1$, atom $A^2$ and atom $A^3$ are each independently, a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
atom $Y^4$ is a carbon atom or nitrogen atom,
bond a1 to bond a6 are each independently a single bond or a double bond,

one of bond a2, bond a5 and bond a6 is a double bond, and the other two are single bonds,
bond a1 and bond a3 to bond a6 may each independently constitute a part of the ring,
provided that any adjacent bonds of bond a1 to bond a6 are not double bonds at the same time;

(12) a pharmaceutical composition for use as an inhibitor according to any of the above-mentioned (8) to (11), wherein the structure containing a substructure represented by Formula:

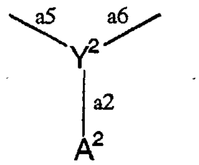

is a structure represented by Formula:

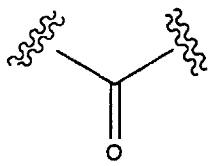
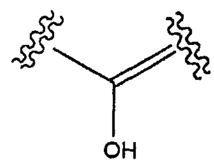
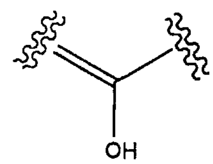
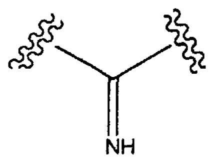
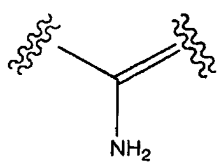
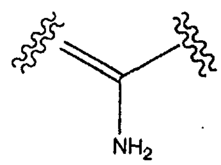
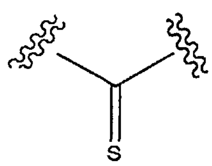
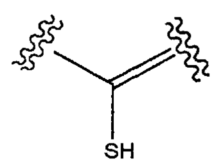
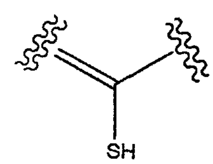
;

(13) a pharmaceutical composition for use as an inhibitor according to the above-mentioned (1), wherein the compound chelates two divalent metal ions and forms a complex when the compound contacts with a divalent metal salt;

(14) a pharmaceutical composition for use as an inhibitor according to the above-mentioned (1), wherein the compound chelates two divalent metal ions and forms a complex when the compound contacts with a base and a divalent metal salt;

(15) a pharmaceutical composition for use as an inhibitor according to the above-mentioned (1), wherein the compound chelates two divalent metal ions and forms a complex when the compound contacts with two equivalents or more of a base and one equivalent or more of a divalent metal salt;

(16) a pharmaceutical composition for use as an inhibitor according to any of the above mentioned (13) to (15), wherein the complex is a complex in which the compounds and the metal ions are present in-the ratio of 2:2;

(17) a pharmaceutical composition for use as an inhibitor according to any of the above-mentioned (13) to (16), wherein the complex can be identified by the change in an elemental analysis, mass spectroscopy, X-ray crystal analysis and/or UV spectrum;

(18) a pharmaceutical composition for use as an inhibitor according to any of the above-mentioned (1) to (17), wherein the distance between the two divalent metal ions is 3.2 to 4.5 angstrom;

(19) a pharmaceutical composition for use as an inhibitor according to any of the above-mentioned (1) to (18), wherein each of the two divalent metal ions is $Mg^{2+}$ or $Mn^{2+}$;

(20) a pharmaceutical composition for use as an inhibitor according to any of the above-mentioned (1) to (19),

wherein the molecular weight of the compound is 700 or less;
(21) a pharmaceutical composition for use as an inhibitor according to any of the above-mentioned (1) to (20), wherein the enzyme having two divalent metal ions as an active center is enzyme which catalyzes nucleic acid related reactions;
(22) a pharmaceutical composition for use as an inhibitor according to the above-mentioned (21), wherein the enzyme which catalyzes nucleic acid related reactions is an HIV integrase;
(23) a pharmaceutical composition for use as an HIV integrase inhibitor which comprises a compound having a substructure according to any of the above-mentioned (8) to (12) and having moiety T which satisfies the following criteria:

(1) the distance between the center of moiety T and the position of atom $A^2$ (distance T-$A^2$) is 6.0 to 11.0 angstrom,
(2) the angle defined by the center of moiety T, the position of atom $A^2$ and the position of atom $A^3$ (angle T-$A^2$-$A^3$) is 5.0 to 30.0°, and
(3) the torsional angle defined by the center of moiety T, position of atom $A^2$, position of atom $A^3$ and position of atom $A^1$ (torsional angle T-$A^2$-$A^3$-$A^1$) is 30.0 to 100.0°,
a prodrug thereof, a pharmaceutically acceptable salt thereof or a solvate thereof;

(24) a pharmaceutical composition for use as an HIV integrase inhibitor which comprises a compound having a substructure according to any of the above-mentioned (8) to (12) and having moiety T which satisfies the following criteria:

(1) the distance between the center of moiety T and the position of atom $A^3$ (distance T-$A^3$) is 3.0 to 8.0 angstrom,
(2) the angle defined by the center of moiety T, the position of atom $A^3$ and the position of atom $A^2$ (angle T-$A^3$-$A^2$) is 130.0 to 165.0°, and
(3) the torsional angle defined by the center of moiety T, position of atom $A^3$, position of atom $A^2$ and position of Atom $A^1$ (torsional angle T-$A^3$-$A^2$-$A^1$) is 240.0 to 320.0°,
a prodrug thereof, a pharmaceutically acceptable salt thereof or a solvate thereof;

(25) a pharmaceutical composition for use as an HIV integrase inhibitor which comprises a compound having a substructure according to any of the above-mentioned (8) to (12) having moiety T which satisfies the following criteria:

(1) the distance between the center of moiety T and the position of divalent metal ion $M^2$ (distance T-$M^2$) is 3.5 to 8.5 angstrom,
(2) the angle defined by the center of moiety T, the position of divalent metal ion $M^2$ and the position of atom $A^2$ (angle T-$M^2$-$A^2$) is 110.0 to 140.0°, and
(3) the torsional angle defined by the center of moiety T, position of divalent metal ion $M^2$, position of atom $A^2$ and position of divalent metal ion $M^1$ (torsional angle T-$M^2$-$A^2$-$M^1$) is 130.0 to 165.0°,
a prodrug thereof, a pharmaceutically acceptable salt thereof or a solvate thereof;

(26) a pharmaceutical composition for use as an HIV integrase inhibitor according to any of the above-mentioned (23) to (25), wherein moiety T is a group whose volume is 50 cubic angstrom or more;
(27) a pharmaceutical composition for use as an HIV integrase inhibitor according to any of the above-mentioned (23) to (25), wherein moiety T is a group represented by Formula:

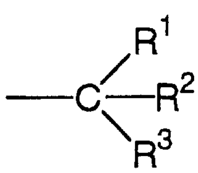

wherein 1) $R^1$, $R^2$ and $R^3$ are hydrogen, optionally substituted alkyl, optionally substituted alkenyl or optionally substituted alkynyl, and two of $R^1$, $R^2$ and $R^3$ are optionally substituted alkyl, optionally substituted alkenyl or optionally substituted alkynyl, 2) $R^1$ is hydrogen, optionally substituted alkyl, optionally substituted alkenyl or op-

tionally substituted alkynyl, and $R^2$ and $R^3$ are taken together to form an optionally substituted 3- to 8-membered ring consisting of hydrogen atoms, carbon atoms, nitrogen atoms, sulfur atoms and oxygen atoms, or 3) $R^1$ and $R^2$ are taken together with $R^3$ to form an optionally substituted 3- to 8-membered ring consisting of hydrogen atoms, carbon atoms, nitrogen atoms, sulfur atoms and oxygen atoms, or a group represented by Formula:

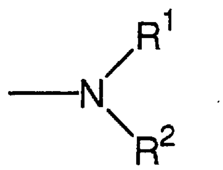

wherein 1) $R^1$ and $R^2$ are optionally substituted alkyl, optionally substituted alkenyl or optionally substituted alkynyl, or 2) $R^2$ and $R^3$ are taken together to form an optionally substituted 3- to 8-membered ring consisting of hydrogen atoms, carbon atoms, nitrogen atoms, sulfur atoms and oxygen atoms;
(28) a pharmaceutical composition for use as an HIV integrase inhibitor according to any of the above-mentioned (23) to (25), wherein moiety T is an optionally substituted carbocyclic group, an optionally substituted heterocyclic group, an optionally substituted branched alkyl or an optionally substituted branched alkenyl;
(29) a pharmaceutical composition for use as an HIV integrase inhibitor according to any of the above-mentioned (23) to (25), wherein moiety T is optionally substituted phenyl;
(30) a method for inhibiting an enzyme having two divalent metal ions as an active center, which comprises bringing a compound capable of chelating both of the two divalent metal ions at the same time, a prodrug thereof, a pharmaceutically acceptable salt thereof or a solvate thereof into contact with the enzyme;
(31) a method for inhibiting an enzyme having two divalent metal ions as an active center, which comprises bringing a compound capable of chelating both of the two divalent metal ions present in the active center of the enzyme at the same time, a prodrug thereof, a pharmaceutically acceptable salt thereof or a solvate thereof into contact with the enzyme;
(32) a method for inhibiting an enzyme having two divalent metal ions as an active center, which comprises bringing a compound which is capable of chelating both of the two divalent metal ions at the same time and which has an inhibitory activity on the enzyme having two divalent metal ions as an active center, a prodrug thereof, a pharmaceutically acceptable salt thereof or a solvate thereof into contact with the enzyme;
(33) a method for inhibiting an enzyme having two divalent metal ions as an active center, which comprises the following steps:

(1) a step for bringing a compound into contact with the enzyme, and
(2) a step for chelating the compound with both of the two divalent metal ion at the same time;

(34) a method for inhibiting an enzyme according to any of the above-mentioned (30) to (33), wherein the compound is a compound according to any of the above-mentioned (1) to (12);
(35) a method for inhibiting an enzyme according to any of the above-mentioned (30) to (33), wherein the compound is a compound according to any of the above-mentioned (13) to (17);
(36) a method for searching for an inhibitor of an enzyme having two divalent metal ions as an active center, which comprises using a search prerequisite that the distance between atom $A^1$ and atom $A^2$ (distance $A^1$-$A^2$) is 2.4 to 3.6 angstrom, the distance between atom $A^2$ and atom $A^3$ (distance $A^2$-$A^3$) is 2.4 to 3.6 angstrom and the angle defined by atom $A^1$, atom $A^2$ and atom $A^3$ (angle $A^1$-$A^2$-$A^3$) is 110 to 180°,

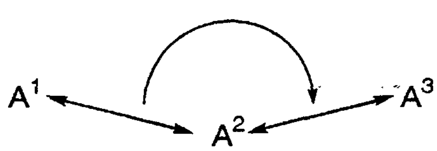

;

(37) a method for searching for an inhibitor of an enzyme having two divalent metal ions as an active center, which comprises using a substructure data according to any of the above-mentioned (8) to (12);
(38) a method for searching for an inhibitor according to the above-mentioned (37), which comprises using the

substructure data as a search prerequisite;
(39) a pharmaceutical composition for use as an inhibitor of an enzyme having two divalent metal ions as an active center which comprises a compound introduced by a searching method according to any of the above-mentioned (36) to (38), a prodrug thereof, a pharmaceutically acceptable salt thereof or a solvate thereof;
(40) a pharmaceutical composition for use as an HIV integrase inhibitor according to any of the above-mentioned (23) to (25), which comprises a compound represented by Formula:

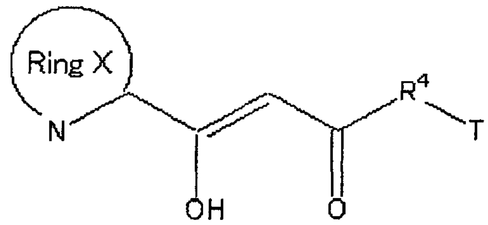

wherein $R^4$ is an organic residue, moiety T is an optionally substituted carbocyclic group, optionally substituted heterocyclic group, optionally substituted branched alkyl or optionally substituted branched alkenyl, ring X is an optionally substituted nitrogen-containing heterocyclic group, and the nitrogen atom (N) containing in ring X is an atom capable of coordinating to a divalent metal ion, a prodrug thereof, a pharmaceutically acceptable salt thereof or a solvate thereof as an active ingredient;
(41) a pharmaceutical composition for use as an HIV integrase inhibitor according to any of the above-mentioned (23) to (25), which comprises a compound represented by Formula:

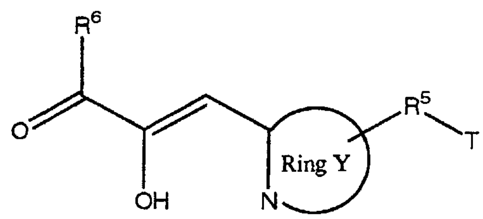

wherein $R^5$ and $R^6$ are each independently an organic residue, moiety T is an optionally substituted carbocyclic group, optionally substituted heterocyclic group, optionally substituted branched alkyl or optionally substituted branched alkenyl, ring Y is an optionally substituted nitrogen-containing heterocyclic group, and the nitrogen atom (N) containing in ring Y is an atom capable of coordinating to a divalent metal ion, a prodrug thereof, a pharmaceutically acceptable salt thereof or a solvate thereof or a compound represented by Formula:

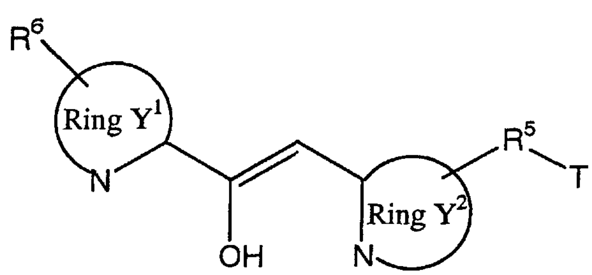

wherein $R^5$ and $R^6$ are each independently an organic residue, moiety T is an optionally substituted carbocyclic group, optionally substituted heterocyclic group, optionally substituted branched alkyl or optionally substituted branched alkenyl, each of ring $Y^1$ and ring $Y^2$ is an optionally substituted nitrogen-containing heterocyclic group, and the nitrogen atom (N) containing in ring $Y^1$ and ring $Y^2$ is an atom capable of coordinating to a divalent metal ion, a prodrug thereof, a pharmaceutically acceptable salt thereof or a solvate thereof as an active ingredient;
(42) a pharmaceutical composition for use as an HIV integrase inhibitor according to any of the above-mentioned (23) to (25), which comprises a compound represented by Formula:

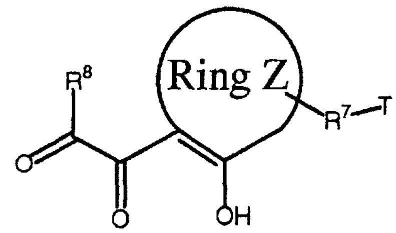

wherein $R^7$ and $R^8$ are each independently an organic residue, moiety T is an optionally substituted carbocyclic group, optionally substituted heterocyclic group, optionally substituted branched alkyl or optionally substituted branched alkenyl, ring Z is an optionally substituted carbocyclic ring or optionally substituted heterocyclic ring, a prodrug thereof, a pharmaceutically acceptable salt thereof or a solvate thereof or a compound represented by Formula:

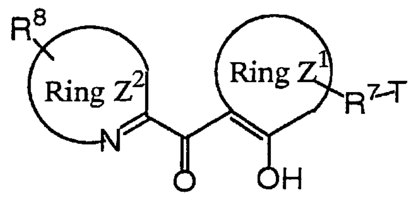

wherein $R^7$ and $R^8$ are each independently an organic residue, moiety T is an optionally substituted carbocyclic group, optionally substituted heterocyclic group, optionally substituted branched alkyl or optionally substituted branched alkenyl, ring $Z^1$ is an optionally substituted carbocyclic ring or optionally substituted heterocyclic ring, ring $Z^2$ is an optionally substituted nitrogen-containing heterocyclic ring, and the nitrogen atom (N) containing in ring $Z^2$ is an atom capable of coordinating to a divalent metal ion, a prodrug thereof, a pharmaceutically acceptable salt thereof or a solvate thereof as an active ingredient;
(43) a method for treating a disease related to an enzyme having two divalent metal ions as an active center which comprises administering a compound which can coordinate to both of two divalent metal ions, a prodrug thereof, a pharmaceutically acceptable salt thereof or a solvate thereof;
(44) use of a compound which can coordinate to both of two divalent metal ions for producing a therapeutic agent against a disease related to an enzyme having two divalent metal ions as an active center;
(45) a method for searching for an inhibitor of an enzyme having two divalent metal ions as an active center, which comprises the following steps:

(1) a step for selecting a compound which can coordinate to both of two divalent metal ions, and
(2) a step for determining the inhibitory activity of the selected compound on the enzyme;

(46) a method for searching for an inhibitor of an enzyme having two divalent metal ions as an active center, which comprises:

(1) a step for selecting a compound having a substructure according to the above-mentioned (8) to (12), and
(2) a step for determining the inhibitory activity of the selected compound on the enzyme;

(47) a method for searching for an inhibitor of an enzyme having two divalent metal ions as an active center, which comprises the following steps:

(1) a step for producing a compound having a substructure according to the above-mentioned (8) to (12), and
(2) a step for determining the inhibitory activity of the produced compound on the enzyme;

(48) a method for searching for an inhibitor of an enzyme having two divalent metal ions as an active center, which comprises the following steps:

(1) a step for selecting, among compounds, a compound having atom $A^1$, atom $A^2$ and atom $A^3$ which fulfill

the condition that the distance between atom $A^1$ and atom $A^2$ (distance $A^1$-$A^2$) is 2.4 to 3.6 angstrom, the distance between atom $A^2$ and atom $A^3$ (distance $A^2$-$A^3$) is 2.4 to 3.6 angstrom and the angle defined by atom $A^1$, atom $A^2$ and atom $A^3$ (angle $A^1$-$A^2$-$A^3$) is 110 to 180°, and
(2) a step for determining the inhibitory activity of the selected compound on the enzyme;

(49) a method for searching for an inhibitor of an enzyme having two divalent metal ions as an active center, which comprises the following steps:

(1) a step for selecting a compound which forms a complex in which the compound is chelating two divalent metal ions when the compound contacts with the divalent metal salt, and
(2) a step for determining the inhibitory activity of the selected compound on the enzyme; and

(50) a method for searching for an inhibitor of an enzyme having two divalent metal ions as an active center, which comprises the following steps:

(1) a step for bringing a compound into contact with a divalent metal salt,
(2) a step for ensuring that the compound forms a complex in which the compound is chelating two divalent metal ions, and
(3) a step for determining the inhibitory activity of the selected compound on the enzyme.

In more detail, the invention relates to the following.
The present invention relates to a pharmaceutical composition for use as an inhibitor of an enzyme, particularly a pharmaceutical composition for use as an inhibitor of an enzyme having two divalent metal ions as an active center.
An enzyme having two divalent metal ions as an active center means an enzyme having amino acid residues and two divalent metal ions as an active center. Thus, the enzyme catalyzes a reaction as a result of the coordination of the substrate to the two divalent metal ions, and may for example be a protein kinase or enzymes which catalyze nucleic acid related reactions, although it is not limited to a particular meaning.
For example, a protein kinase catalyzes the phosphorylation of a substrate by means of transferring the terminal phosphate of an ATP to the substrate protein. In the catalytic reaction, the oxygen atom in the phosphate in an ATP is considered to coordinate to two divalent metal ions which are the active center of the protein kinase. It is also known that the protein kinase active center has the site which recognizes the hydroxyl group in the substrate protein and the site which is bound to the ATP and divalent ions (such as $Mg^{2+}$) are present at the ATP-binding site. Also in the cases of an ATP dependent protein kinase and CAMP dependent protein kinase which are classified also as protein kinases, an X-ray crystal structure analysis revealed that two divalent metal ions are present in the active center, with the distances between these two metals being 3.97 angstrom and 3.85 angstrom, respectively.
For example, a nucleic acid-synthesizing enzyme (DNA dependent DNA polymerase, RNA dependent DNA polymerase (such as retrovirus reverse transcriptase), RNA dependent RNA polymerase (such as HCV polymerase), DNA dependent RNA polymerase (such as human polymerase)), nuclease (such as DNA nuclease, RNA nuclease), integrase, ligase and the like are exemplified as enzymes which catalyze nucleic acid related reactions. In many of the above enzymes, three amino acid residues (for example, acidic amino acid residues such as aspartic acid, glutamic acid, and the like) in the enzyme which is an active center forms a catalytic triad and coordinates to two divalent metal ions. In a catalytic reaction, a nucleic acid molecule as a substrate is considered to coordinate to two divalent metal ions as shown below.

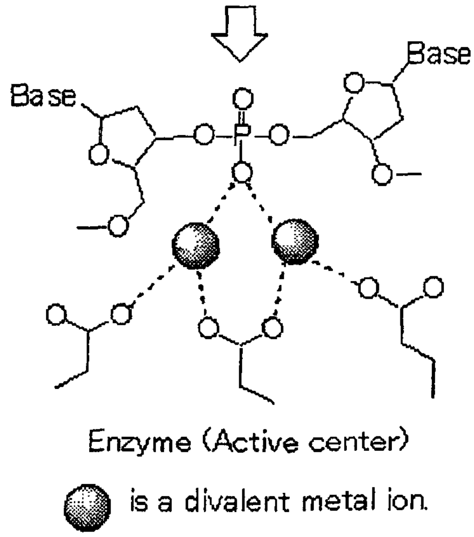


The figure shown above can be represented also as shown below when viewed in the direction of the arrow.

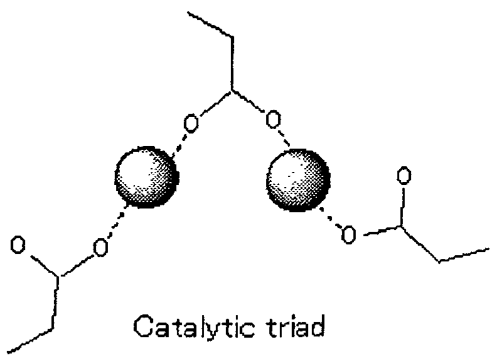


As shown above, the carboxylic groups in acidic amino acid residues such as aspartic acid or glutamic acid as an active center are positioned in a triangle. A substrate nucleic acid molecule coordinate to these two divalent metal ions from the opposite side of this triangle (i.e., from the upside of the figure).

Accordingly, by designing a compound capable of substituting this nucleic acid molecule it is possible to inhibit an enzyme having two divalent metal ions as an active center effectively. Thus, a compound which chelates these two divalent metal ions at the same time is an extremely effective inhibitor of an enzyme having two divalent metal ions as an active center.

In many of the enzymes which catalyze nucleic acid related reactions, two divalent metal ions are present as each active center, with the distance between such two divalent metal ions being about 3.2 to 4.5 angstrom, typically about 3.4 to 4.0 angstrom, in an interaction manner similar to that described above. Generally, magnesium ion ($Mg^{2+}$), manganese ion ($Mn^{2+}$), and the like are exemplified as a divalent metal ion, although it is not limited particularly.

As an enzyme having two divalent metal ions as an active center, an enzyme wherein the distance between two divalent metal ions is about 4.5 angstrom or less is preferred particularly. For example, the 3-D coordinates of the following enzymes having two metal ions wherein the distance between them is about 4.5 angstrom or less are registered in PDB database: serine/threonine phosphatase, aminopeptidase, methionineamnopeptidase, isomerase, RNA reductase, carbamoyl phosphate synthetase, glutathione synthetase, ligase, polymerase, farnesyl diphosphate synthase, carbon enzyme lyase, enolase, alkaline phosphatase, monophosphate, phosphotransferase, phosphorylase kinase, hydrolase, nucleoside diphosphate kinase, uridyl monophosphate/cytidyl monophosphate kinase, GTP-binding protein, CAMP-dependent protein kinase, pyrophosphatase, arginase, metallobetalactamase, metallotionine, urease, aldolase, phospholipase C, nuclease, integrase, carboxypeptidase, and phosphotriesterase.

Any of the enzymes listed above is an enzyme having two divalent metal ions as an active center. Some of these crystal structures may contain a metal species different from that associated naturally, such as aluminum, cobalt, nickel, cadmium and the like, due to the crystallization under a condition which is different from an in vivo condition.

However, the natural divalent metal ions are considered to be involved in an actual in vivo function as the active center.

The present invention provides a "two metal chelating model" shown below. Specifically, it is a model in which a compound capable of chelating both of two divalent ions prevents the interaction between an enzyme and a substrate (particularly, the interaction between the two divalent metal ions and the substrate molecule), thereby inhibiting the enzyme reaction.

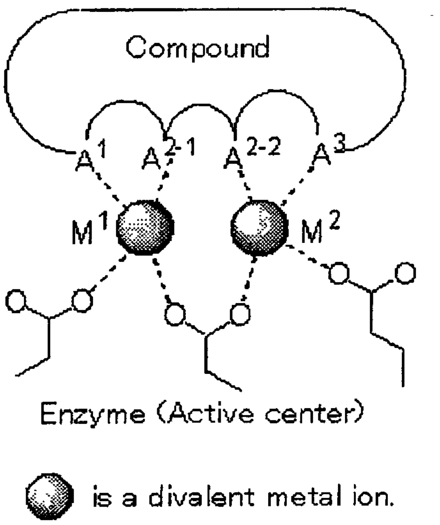


wherein atom $A^1$ and atom $A^{2-1}$ coordinate to divalent metal ion $M^1$ to form a chelate ring, and atom $A^{2-2}$ and $A^3$ coordinate to divalent metal ion $M^2$ to form a chelate ring.

The compound capable of coordinating to both of two divalent metal ions includes a compound, as is represented in the figure shown above, in which atom $A^1$ and atom $A^{2-2}$ coordinate to divalent metal ion $M^1$ to form a chelate ring, and atom $A^{2-1}$ and $A^3$ coordinate to divalent metal ion $M^2$ to form a chelate ring.

Especially as shown in the following figure, a compound having atom $A^1$ capable of coordinating one divalent metal ion, atom $A^3$ capable of coordinating the other divalent metal ion and atom $A^2$ capable of coordinating both divalent metal ions is preferred. While an enzyme forming a catalytic triad is exemplified here for convenience, the enzyme can otherwise be inhibited effectively as a result of the similar chelate ring formation.

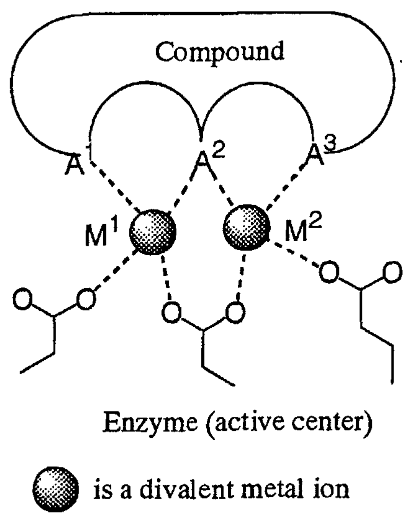


wherein atom $A^1$ and atom $A^2$ coordinate to divalent metal ion $M^1$ to form a chelate ring, atom $A^2$ and $A^3$ coordinate to divalent metal ion $M^2$ to form a chelate ring, and the divalent metal ion to which atom $A^1$ and atom $A^2$ is coordinating is designated as $M^1$, and the divalent metal ion to which atom $A^2$ and atom $A^3$ is coordinating is designated as $M^2$.

"Chelate" means the formation of a ring (chelate ring) as a result of the coordination of two or more atoms

(coordinating atoms) possessed by a compound (ligand) to a single metal ion.

In the "two metal chelating model" shown above, two chelate rings are formed. Thus, two atoms (atom $A^1$ and $A^2$) possessed by the compound coordinate to the left metal ion ($M^1$) in the figure shown above to form a chelate ring. Also to the right metal ion ($M^2$) in the figure shown above, two atoms (atom $A^2$ and $A^3$) possessed by the compound coordinate to form a chelate ring.

As a chelate ring, a chelate ring having 3 or more members are exemplified. A 5- or 6-membered chelate ring is preferred in view of the stability of the ring. Thus, a compound capable of forming a 5-membered and/or 6-membered chelate ring is preferred particularly as an inhibitor of an enzyme having two divalent metal ions as an active center.

As such a compound:

a compound having a substructure represented by Formula (I):

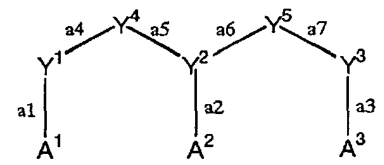

(I)

wherein atom $A^1$, atom $A^3$ and atom $A^4$ are each independently a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
bond a1 to bond a7 are each independently a single bond or a double bond,
one of bond a2, bond a5 and bond a6 is a double bond, and the other two are single bonds,
bond a1 and bond a3 to bond a7 may each independently constitute a part of the ring,
provided that any adjacent bonds of bond a1 to bond a7 are not double bonds at the same time,
a compound having a substructure represented by Formula (II):

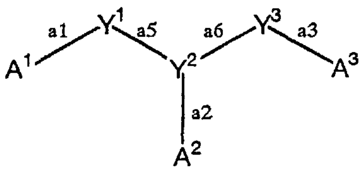

(II)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
bond a1 to bond a3, bond a5 and bond a6 are each independently a single bond or a double bond,
one of bond a2, bond a5 and bond a6 is a double bond, and the other two are single bonds,
bond a1, bond a3, bond a5 and bond a6 may each independently constitute a part of the ring,
provided that any adjacent bonds of bond a1 to bond a3, bond a5 and bond a6 are not double bonds at the same time,
a compound having a substructure represented by Formula (III):

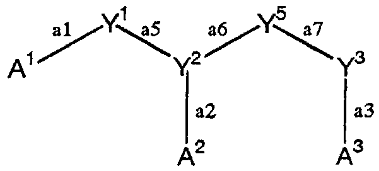

(III)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion, atom $Y^1$ and atom $Y^3$ are each independently a carbon atom, nitrogen atom or sulfur atom,
atom $Y^2$ is a carbon atom,
atom $Y^5$ is a carbon atom or nitrogen atom,
bond a1 to bond a3 and bond a5 to bond a7 are each independently a single bond or a double bond,
one of bond a2, bond a5 and bond a6 is a double bond, and the other two are single bonds,
bond a1, bond a3 and bond a5 to bond a7 may each independently constitute a part of the ring, provided that any adjacent bonds of bond a1 to bond a3 and bond a5 to bond a7 are not double bonds at the same time, and
a compound having a substructure represented by Formula (IV):

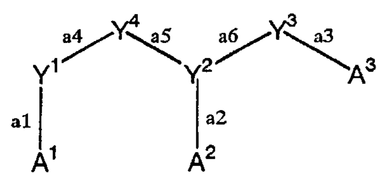

(IV)

wherein atom $A^1$, atom $A^2$ and atom $A^3$ are each independently, a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
atom $Y^5$ is a carbon atom or nitrogen atom,
bond a1 to bond a6 are each independently a single bond or a double bond,
one of bond a2, bond a5 and bond a6 is a double bond, and the other two are single bonds,
bond a1 and bond a3 to bond a6 may each independently constitute a part of the ring,
provided that any adjacent bonds of bond a1 to bond a6 are not double bonds at the same time are exemplified.

An atom capable of coordinating to a divalent metal means an atom having at least one lone electron-pair and capable of giving the electrons to a vacant p orbital or d orbital of the divalent metal ion under a physiological condition. As an example of the atom, an oxygen atom capable of coordinating to a divalent metal ion, nitrogen atom capable of coordinating to a divalent metal ion, sulfur atom capable of coordinating to a divalent metal ion and phosphorus atom capable of coordinating to a divalent metal ion are exemplified. Those preferred especially are an oxygen atom capable of coordinating to a divalent metal ion, nitrogen atom capable of coordinating to a divalent metal ion and sulfur atom capable of coordinating to a divalent metal ion. The binding mode of each atom capable of coordinating to a divalent metal ion to the adjacent atom (designated as Y) is represented as Y-O-Y and Y=O when the atom capable of coordinating to a divalent metal ion is an oxygen atom, Y-N-(-Y)-Y, Y-N=Y and Y≡N when the atom capable of coordinating to a divalent metal ion is a nitrogen atom, and Y-S-Y and Y=S when the atom capable of coordinating to a divalent metal ion is a sulfur atom, wherein Y is an adjacent atom, each of Y is an atom selected from the group consisting of hydrogen atom, carbon atom, nitrogen atom and sulfur atom. When the oxygen atom and the sulfur atom are the atoms which are the constituents of aromatic rings (such as an oxygen atom in a furan ring, sulfur atom in a thiophene ring), these atoms can not coordinate to the divalent metal ions. Also when a nitrogen atom to which a hydrogen atom is bound is a constituent of an aromatic ring and the hydrogen atom is not substituted by a metal ion under a physiological condition (such as in a case of a nitrogen atom in a pyrrole ring), the nitrogen atom can not coordinate to the divalent metal ions. Accordingly, such oxygen atom, sulfur atom and nitrogen atom are not included in the atoms capable of coordinating to the divalent metal ions. On the other hand, when a nitrogen atom to which a hydrogen atom is bound is a constituent of an aromatic ring but the hydrogen atom is substituted by a metal ion under

a physiological condition (such as in a case of a nitrogen atom in a tetrazole ring), the nitrogen atom can coordinate to the divalent metal ions. Such a nitrogen atom is an atom capable of coordinating to a divalent metal atom.

In the case of an atom capable of coordinating to both of two divalent metal ions (atom $A^2$), the binding mode of atom $A^2$ to the adjacent carbon atom (designated as $Y^2$) is represented preferably as C=O, C-OH, C=NH, C-$NH_2$, C=S and C-SH, since a simultaneous binding to both of the divalent metal ions is required. When a hydrogen atom is bound to atom $A^2$ (such as C-OH, C=NH, C-$NH_2$, C-SH), it is preferred especially that the hydrogen atom binding to atom $A^2$ can be substituted by a metal ion under a physiological condition.

Also since atom $A^2$ should coordinate to both of two divalent metal ions at the same time, an atom capable of being a monovalent anion is preferred particularly.

It should be discussed also in terms of the 3-D structure whether a compound can chelate with both of two divalent metal ions or not. Thus, atoms which cannot coordinate sterically to divalent metal ions are not included in the atoms capable of coordinating to divalent metal ions. Accordingly, in order to be a compound chelating both of two divalent metal ions, it is preferred that when said compound chelates the two divalent metal ions then the two divalent metal ions and the atom capable of coordinating the divalent metal ions are present substantially on the same plane.

Accordingly, it is preferred that the structure comprising a substructure represented by:

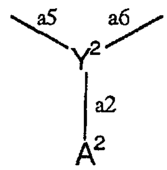

is a structure represented by:

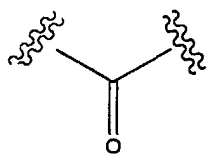
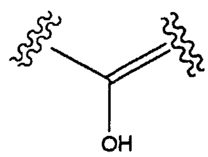
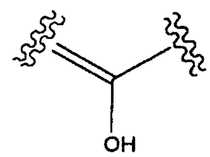
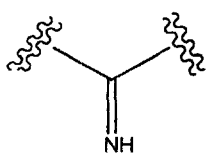
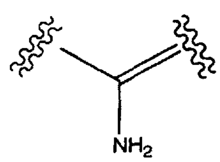
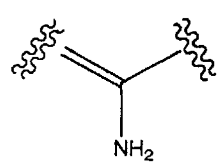
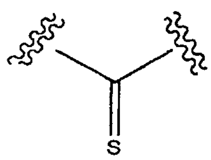
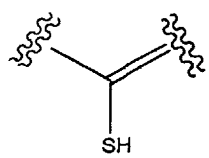
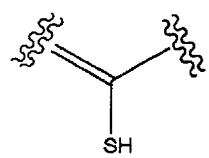
.

In such a case, atom $A^1$ to atom $A^3$ can be present substantially on the same plane, and a compound having such a substructure can form a structure which is advantageous to chelate with both of the divalent metal ions.

It is also possible that an atom capable of chelating a divalent metal may be an atom as a constituent of a moiety of the chain in the chemical structure of a compound, or may be an atom as a constituent of a moiety of the functional group, or may also be an atom as a constituent of a moiety of a ring. It is not possible that an atom capable of chelating both of two divalent metal ions (atom $A^2$) exists as a constituent of a moiety of a ring. Thus, bond a2 is not possible to be a constituent of a moiety of the ring.

The case where an atom capable of chelating a divalent metal is an atom as a constituent of a chain in the chemical structure of a compound, when exemplified using atom $A^3$, means a case where the structure comprising a

substructure represented by Formula:

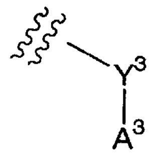

wherein atom $Y^3$ is a carbon atom, nitrogen atom or sulfur atom, and atom $A^3$ is a nitrogen atom capable of coordinating to a divalent metal ion, oxygen atom capable of coordinating to a divalent metal ion or sulfur atom capable of coordinating to a divalent metal ion is a group represented for example by Formula:

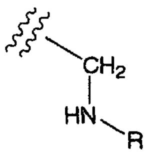 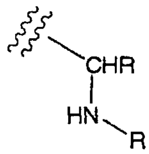 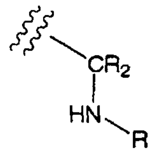 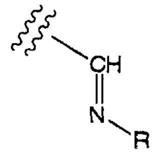 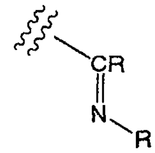 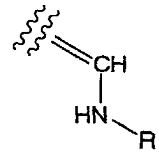

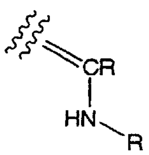 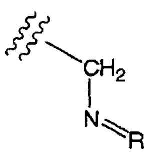 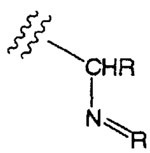 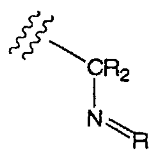 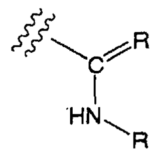 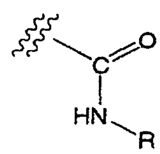

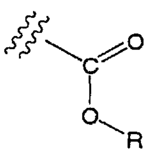 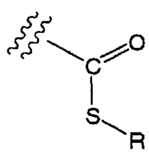 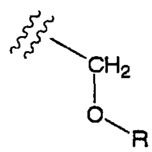 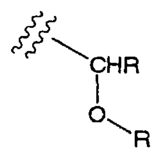 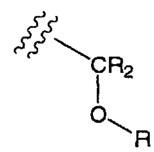 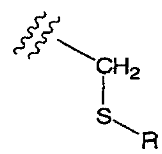

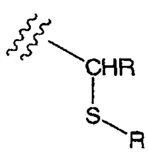 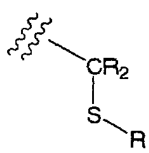 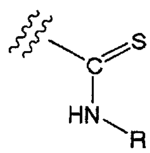 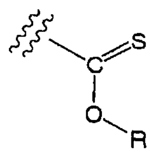 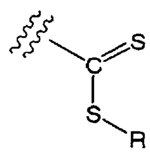 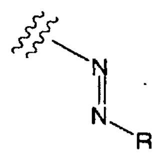

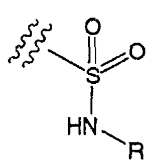 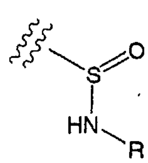

wherein substituent R is an organic residue.

The case where an atom capable of chelating a divalent metal is an atom as a constituent of a moiety of the functional group in the chemical structure of a compound, when exemplified using atom $A^3$, means a case where the structure comprising a substructure represented by Formula:

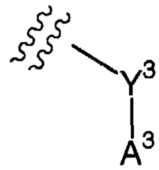

wherein atom $Y^3$ and atom $A^3$ are defined as described above is a group represented for example by Formula:

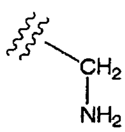
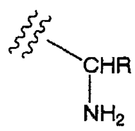
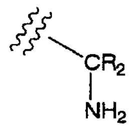
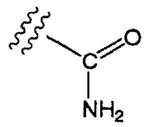
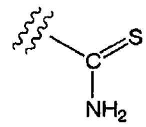
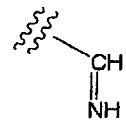

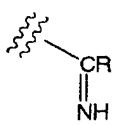
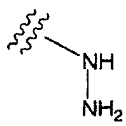
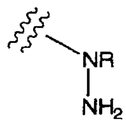
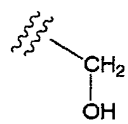
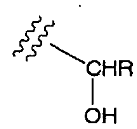
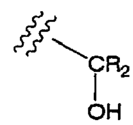

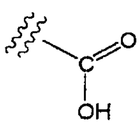
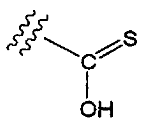
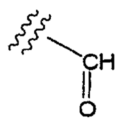
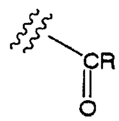
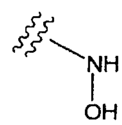
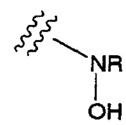

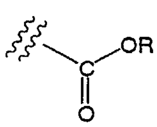
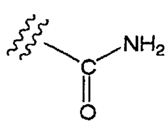
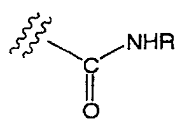
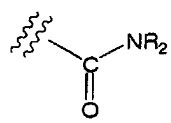
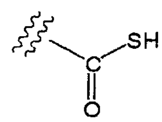
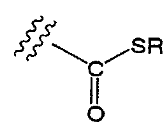

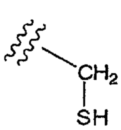
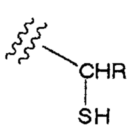
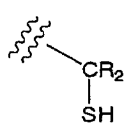
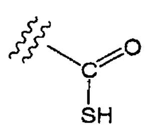
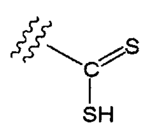
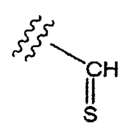

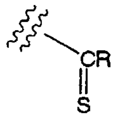 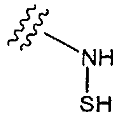 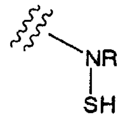 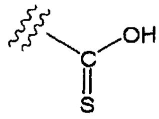 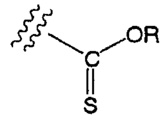 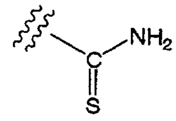

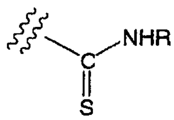 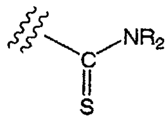 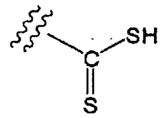 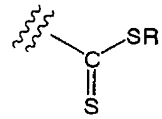

wherein R is each independently an organic residue.

The case where an atom capable of chelating a divalent metal is an atom as a constituent of a moiety of the a ring in the chemical structure of a compound, when exemplified using atom $A^3$, means a case where the structure comprising a substructure represented by Formula:

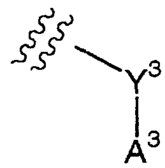

wherein atom $Y^3$ and atom $A^3$ are defined as described above is a group represented for example by Formula:

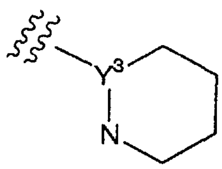 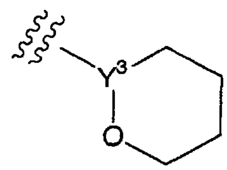 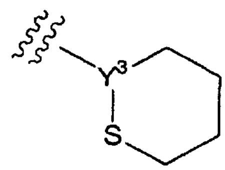

wherein the ring designated as a 6-membered ring for convenience is a heterocyclic ring having 3 or more members, typically, a group represented for example by Formula:

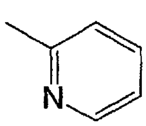 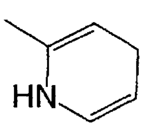 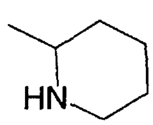 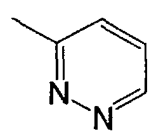 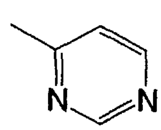

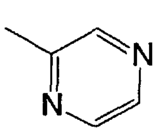 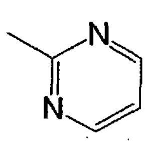 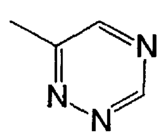 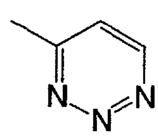 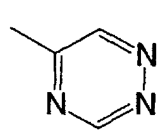

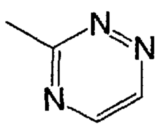 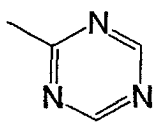 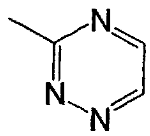 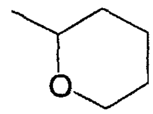 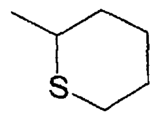

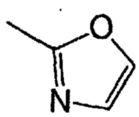 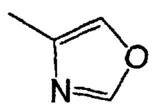 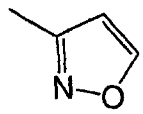 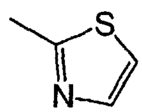 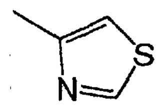

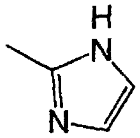 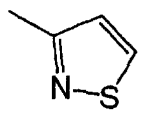 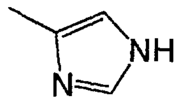 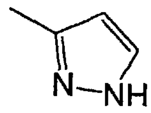 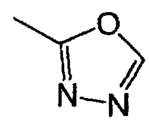

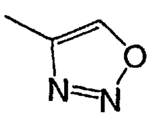 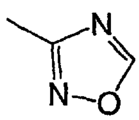 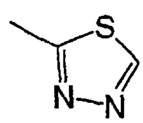 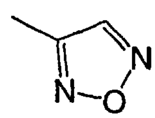 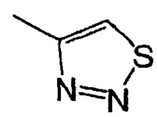

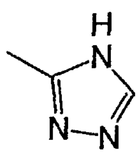 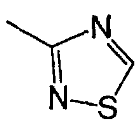 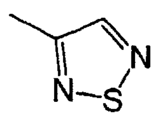 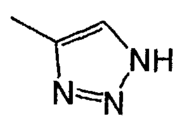 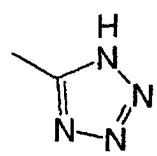

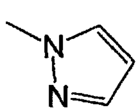 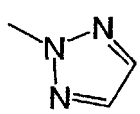 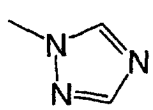 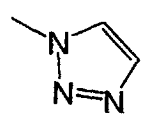 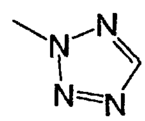

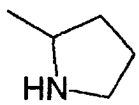 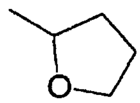 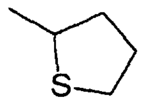 .

BRIEF DESCRIPTION OF THE DRAWINGS

Figure 1 shows a schematic view of the change in the UV spectrum of Compound C-1;
Figure 2 shows a schematic view of the change in the UV spectrum of Compound D-2;
Figure 3 shows a schematic view of the change in the UV spectrum of Compound D-3; and
Figure 4 shows a schematic view of the change in the UV spectrum of Compound D-4.

BEST MODE FOR CARRYING OUT THE INVENTION

The present invention can be carried out as described below.

First, a 3-D structure database of the compounds is searched for a compound capable of chelating both of two divalent metal ions. The 3-D structure database may not only be a crystal structure data base such as Cambridge Structural Database System but also be one made based on 2-D structures using a generator of approximate 3-D molecular structure such as CONCORD, CORINA, CONVERTER, Olive and the like. Accordingly, it is also possible to search for a compound whose 3-D structure has not been characterized experimentally or for a compound which has not been synthesized actually. The 3-D structure search may use various search programs such as UNITY, ISIS-3D, and the like.

For example, for the purpose of searching for a compound which has atom $A^1$ capable of coordinating to one divalent metal ion, atom $A^3$ capable of coordinating to the other divalent metal ion and atom $A^2$ capable of coordinating to the both divalent metal ions at the same time, a compound having three atoms $A^1$, $A^2$ and $A^3$ which fulfill the following conditions may be searched for (atoms $A^1$, $A^2$ and $A^3$ are atoms capable of coordinating to divalent metal ions).

Thus, a compound whose atoms have the structural relationship shown below is searched for.

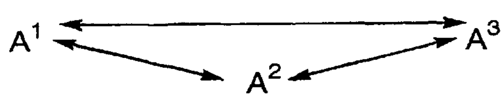


wherein the distance between atom $A^1$ and atom $A^2$, i.e., $A^1$-$A^2$ is about 2.4 to 3.6 angstrom,
the distance between atom $A^1$ and atom $A^3$, i.e., $A^1$-$A^3$ is about 3.9 to 7.2 angstrom,
the distance between atom $A^2$ and atom $A^3$ i.e., $A^2$-$A^3$ is about 2.4 to 3.6 angstrom,
the angle defined by atom $A^1$, atom $A^2$ and atom $A^3$, i.e., $A^1$-$A^2$-$A^3$ is about 110 to 180°,
the angle defined by atom $A^1$, atom $A^3$ and atom $A^2$, i.e., $A^1$-$A^3$-$A^2$ is about 0 to 35°,
the angle defined by atom $A^2$, atom $A^1$ and atom $A^3$, i.e., $A^2$-$A^1$-$A^3$ is about 0 to 35°.

For example, the search described above can be accomplished by searching for a compound in which the distance between atom $A^1$ and atom $A^2$ (distance $A^1$-$A^2$) is about 2.4 to 3.6 angstrom, the distance between atom $A^2$ and atom $A^3$ (distance $A^2$-$A^3$) is about 2.4 to 3.6 angstrom and the angle defined by atom $A^1$, atom $A^2$ and atom $A^3$ (angle $A^1$-$A^2$-$A^3$) is about 110 to 180°.

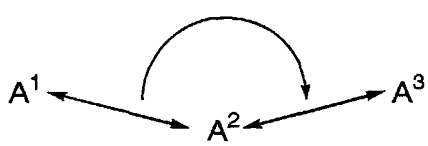


Also when searching for a compound which can form a 5- or 6-membered chelate ring by chelating one divalent metal ion and which can form a 5- or 6-membered chelate ring by chelating the other divalent metal ion, a compound capable of coordinating to two divalent metal atoms can be identified by searching for a compound fulfilling the criteria described below subsequently to or separately from the search described above.

Thus, a compound having a substructure represented by Formula (I):

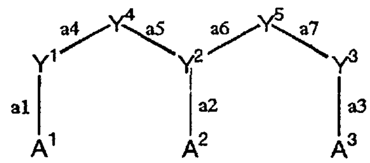

(I)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion, atom $Y^1$ and atom $Y^3$ are each independently a carbon atom, nitrogen atom or sulfur atom,
atom $Y^2$ is a carbon atom,
atom $Y^4$ and atom $Y^5$ are each independently a carbon atom or nitrogen atom,
bond a1 to bond a7 are each independently a single bond or a double bond,
one of bond a2, bond a5 and bond a6 is a double bond, and the other two are single bonds,
bond a1 and bond a3 to bond a7 may each independently constitute a part of the ring,
provided that any adjacent bonds of bond a1 to bond a7 are not double bonds at the same time,
may be searched for.

Among the compounds having the substructures described above, the following combinations of the respective atoms ($A^1$, $A^2$, $A^3$, $Y^1$, $Y^3$, $Y^4$, $Y^5$) are preferred particularly. When the combination of ($A^1$, $A^2$, $A^3$, $Y^1$, $Y^2$, $Y^3$, $Y^4$, $Y^5$) is (O, O, O, C, C, C, C, C), it means the case where atom $A^1$ is an oxygen atom, atom $A^2$ is an oxygen atom, atom $A^3$ is an oxygen atom, atom $Y^1$ is a carbon atom, atom $Y^3$ is a carbon atom, atom $Y^4$ is a carbon atom and atom $Y^5$ is a carbon atom, and the designation may be applied to other cases (in the designation here, C is a carbon atom, N is a nitrogen atom, O is an oxygen atom and S is a sulfur atom). Combination ($A^1$,$A^2$,$A^3$,$Y^1$,$Y^2$,$Y^3$,$Y^4$,$Y^5$) = (O,O,O,C,C,C,C,C), (O,O,O,C,C,C,C,N), (O,O,O,C,C,C,N,C), (O,O,O,C,C,C,N,N), (O,O,O,C,C,N,C,C), (O,O,O,C,C,N,C,N), (O,O,O,C,C,N,N,C), (O,O,O,C,C,N,N,N), (O,O,O,C,C,S,C,C), (O,O,O,C,C,S,C,N), (O,O,O,C,C,S,N,C), (O,O,O,C,C,S,N,N), (O,O,O,N,C,C,C,C), (O,O,O,N,C,C,C,N), (O,O,O,N,C,C,N,C), (O,O,O,N,C,C,N,N), (O,O,O,N,C,N,C,C), (O,O,O,N,C,N,C,N), (O,O,O,N,C,N,N,C), (O,O,O,N,C,N,N,N), (O,O,O,N,C,S,C,C), (O,O,O,N,C,S,C,N), (O,O,O,N,C,S,N,C), (O,O,O,N,C,S,N,N), (O,O,O,S,C,C,C,C), (O,O,O,S,C,C,C,N), (O,O,O,S,C,C,N,C), (O,O,O,S,C,C,N,N), (O,O,O,S,C,N,C,C), (O,O,O,S,C,N,C,N), (O,O,O,S,C,N,N,C), (O,O,O,S,C,N,N,N), (O,O,O,S,C,S,C,C), (O,O,O,S,C,S,C,N), (O,O,O,S,C,S,N,C), (O,O,O,S,C,S,N,N), (O,O,N,C,C,C,C,C), (O,O,N,C,C,C,C,N), (O,O,N,C,C,C,N,C), (O,O,N,C,C,C,N,N), (O,O,N,C,C,N,C,C), (O,O,N,C,C,N,C,N), (O,O,N,C,C,N,N,C), (O,O,N,C,C,N,N,N), (O,O,N,C,C,S,C,C), (O,O,N,C,C,S,C,N), (O,O,N,C,C,S,N,C), (O,O,N,C,C,S,N,N), (O,O,N,N,C,C,C,C), (O,O,N,N,C,C,C,N), (O,O,N,N,C,C,N,C), (O,O,N,N,C,C,N,N), (O,O,N,N,C,N,C,C), (O,O,N,N,C,N,C,N), (O,O,N,N,C,N,N,C), (O,O,N,N,C,N,N,N), (O,O,N,N,C,S,C,C), (O,O,N,N,C,S,C,N), (O,O,N,N,C,S,N,C), (O,O,N,N,C,S,N,N), (O,O,N,S,C,C,C,C), (O,O,N,S,C,C,C,N), (O,O,N,S,C,C,N,C), (O,O,N,S,C,C,N,N), (O,O,N,S,C,N,C,C), (O,O,N,S,C,N,C,N), (O,O,N,S,C,N,N,C), (O,O,N,S,C,N,N,N), (O,O,N,S,C,S,C,C), (O,O,N,S,C,S,C,N), (O,O,N,S,C,S,N,C), (O,O,N,S,C,S,N,N), (O,O,S,C,C,C,C,C), (O,O,S,C,C,C,C,N), (O,O,S,C,C,C,N,C), (O,O,S,C,C,C,N,N), (O,O,S,N,C,C,C,C), (O,O,S,N,C,C,C,N), (O,O,S,N,C,C,N,C), (O,O,S,N,C,C,N,N), (O,O,S,S,C,C,C,C), (O,O,S,S,C,C,C,N), (O,O,S,S,C,C,N,C), (O,O,S,S,C,C,N,N), (O,N,O,C,C,C,C,C), (O,N,O,C,C,C,C,N), (O,N,O,C,C,C,N,C), (O,N,O,C,C,C,N,N), (O,N,O,C,C,N,C,C), (O,N,O,C,C,N,C,N), (O,N,O,C,C,N,N,C), (O,N,O,C,C,N,N,N), (O,N,O,C,C,S,C,C), (O,N,O,C,C,S,C,N), (O,N,O,C,C,S,N,C), (O,N,O,C,C,S,N,N), (O,N,O,N,C,C,C,C), (O,N,O,N,C,C,C,N), (O,N,O,N,C,C,N,C), (O,N,O,N,C,C,N,N), (O,N,O,N,C,N,C,C), (O,N,O,N,C,N,C,N), (O,N,O,N,C,N,N,C), (O,N,O,N,C,N,N,N), (O,N,O,N,C,S,C,C), (O,N,O,N,C,S,C,N), (O,N,O,N,C,S,N,C), (O,N,O,N,C,S,N,N), (O,N,O,S,C,C,C,C), (O,N,O,S,C,C,C,N), (O,N,O,S,C,C,N,C), (O,N,O,S,C,C,N,N), (O,N,O,S,C,N,C,C), (O,N,O,S,C,N,C,N), (O,N,O,S,C,N,N,C), (O,N,O,S,C,N,N,N), (O,N,O,S,C,S,C,C), (O,N,O,S,C,S,C,N), (O,N,O,S,C,S,N,C), (O,N,O,S,C,S,N,N), (O,N,N,C,C,C,C,C), (O,N,N,C,C,C,C,N), (O,N,N,C,C,C,N,C), (O,N,N,C,C,C,N,N), (O,N,N,C,C,N,C,C), (O,N,N,C,C,N,C,N), (O,N,N,C,C,N,N,C), (O,N,N,C,C,N,N,N), (O,N,N,C,C,S,C,C), (O,N,N,C,C,S,C,N), (O,N,N,C,C,S,N,C), (O,N,N,C,C,S,N,N), (O,N,N,N,C,C,C,C), (O,N,N,N,C,C,C,N), (O,N,N,N,C,C,N,C), (O,N,N,N,C,C,N,N), (O,N,N,N,C,N,C,C), (O,N,N,N,C,N,C,N), (O,N,N,N,C,N,N,C), (O,N,N,N,C,N,N,N), (O,N,N,N,C,S,C,C), (O,N,N,N,C,S,C,N), (O,N,N,N,C,S,N,C), (O,N,N,N,C,S,N,N), (O,N,N,S,C,C,C,C), (O,N,N,S,C,C,C,N), (O,N,N,S,C,C,N,C), (O,N,N,S,C,C,N,N), (O,N,N,S,C,N,C,C), (O,N,N,S,C,N,C,N), (O,N,N,S,C,N,N,C), (O,N,N,S,C,N,N,N), (O,N,N,S,C,S,C,C), (O,N,N,S,C,S,C,N), (O,N,N,S,C,S,N,C), (O,N,N,S,C,S,N,N), (O,N,S,C,C,C,C,C), (O,N,S,C,C,C,C,N), (O,N,S,C,C,C,N,C), (O,N,S,C,C,C,N,N), (O,N,S,N,C,C,C,C), (O,N,S,N,C,C,C,N), (O,N,S,N,C,C,N,C), (O,N,S,N,C,C,N,N), (O,N,S,S,C,C,C,C), (O,N,S,S,C,C,C,N), (O,N,S,S,C,C,N,C), (O,N,S,S,C,C,N,N), (O,S,O,C,C,C,C,C), (O,S,O,C,C,C,C,N), (O,S,O,C,C,C,N,C), (O,S,O,C,C,C,N,N), (O,S,O,C,C,N,C,C), (O,S,O,C,C,N,C,N), (O,S,O,C,C,N,N,C), (O,S,O,C,C,N,N,N), (O,S,O,C,C,S,C,C), (O,S,O,C,C,S,C,N), (O,S,O,C,C,S,N,C), (O,S,O,C,C,S,N,N), (O,S,O,N,C,C,C,C), (O,S,O,N,C,C,C,N), (O,S,O,N,C,C,N,C), (O,S,O,N,C,C,N,N), (O,S,O,N,C,N,C,C), (O,S,O,N,C,N,C,N), (O,S,O,N,C,N,N,C), (O,S,O,N,C,N,N,N), (O,S,O,N,C,S,C,C), (O,S,O,N,C,S,C,N), (O,S,O,N,C,S,N,C), (O,S,O,N,C,S,N,N), (O,S,O,S,C,C,C,C), (O,S,O,S,C,C,C,N), (O,S,O,S,C,C,N,C), (O,S,O,S,C,C,N,N), (O,S,O,S,C,N,C,C), (O,S,O,S,C,N,C,N), (O,S,O,S,C,N,N,C), (O,S,O,S,C,N,N,N), (O,S,O,S,C,S,C,C), (O,S,O,S,C,S,C,N), (O,S,O,S,C,S,N,C), (O,S,O,S,C,S,N,N), (O,S,N,C,C,C,C,C), (O,S,N,C,C,C,C,N), (O,S,N,C,C,C,N,C), (O,S,N,C,C,C,N,N), (O,S,N,C,C,N,C,C), (O,S,N,C,C,N,C,N), (O,S,N,C,C,N,N,C), (O,S,N,C,C,N,N,N), (O,S,N,C,C,S,C,C), (O,S,N,C,C,S,C,N), (O,S,N,C,C,S,N,C), (O,S,N,C,C,S,N,N), (O,S,N,N,C,C,C,C), (O,S,N,N,C,C,C,N), (O,S,N,N,C,C,N,C), (O,S,N,N,C,C,N,N), (O,S,N,N,C,N,C,C), (O,S,N,N,C,N,C,N), (O,S,N,N,C,N,N,C), (O,S,N,N,C,N,N,N), (O,S,N,N,C,S,C,C), (O,S,N,N,C,S,C,N), (O,S,N,N,C,S,N,C), (O,S,N,N,C,S,N,N), (O,S,N,S,C,C,C,C), (O,S,N,S,C,C,C,N), (O,S,N,S,C,C,N,C), (O,S,N,S,C,C,N,N), (O,S,N,S,C,N,C,C), (O,S,N,S,C,N,C,N), (O,S,N,S,C,N,N,C), (O,S,N,S,C,N,N,N), (O,S,N,S,C,S,C,C), (O,S,N,S,C,S,C,N), (O,S,N,S,C,S,N,C), (O,S,N,S,C,S,N,N), (O,S,S,C,C,C,C,C), (O,S,S,C,C,C,C,N), (O,S,S,C,C,C,N,C), (O,S,S,C,C,C,N,N), (O,S,S,N,C,C,C,C), (O,S,S,N,C,C,C,N), (O,S,S,N,C,C,N,C), (O,S,S,N,C,C,N,N), (O,S,S,S,C,C,C,C), (O,S,S,S,C,C,C,N), (O,S,S,S,C,C,N,C), (O,S,S,S,C,C,N,N), (N,O,O,C,C,C,C,C), (N,O,O,C,C,C,C,N), (N,O,O,C,C,C,N,C), (N,O,O,C,C,C,N,N), (N,O,O,C,C,N,C,C), (N,O,O,C,C,N,C,N), (N,O,O,C,C,N,N,C), (N,O,O,C,C,N,N,N), (N,O,O,C,C,S,C,C), (N,O,O,C,C,S,C,N), (N,O,O,C,C,S,N,C), (N,O,O,C,C,S,N,N), (N,O,O,N,C,C,C,C), (N,O,O,N,C,C,C,N), (N,O,O,N,C,C,N,C), (N,O,O,N,C,C,N,N), (N,O,O,N,C,N,C,C), (N,O,O,N,C,N,C,N), (N,O,O,N,C,N,N,C), (N,O,O,N,C,N,N,N), (N,O,O,N,C,S,C,C), (N,O,O,N,C,S,C,N), (N,O,O,N,C,S,N,C), (N,O,O,N,C,S,N,N), (N,O,O,S,C,C,C,C), (N,O,O,S,C,C,C,N), (N,O,O,S,C,C,N,C), (N,O,O,S,C,C,N,N), (N,O,O,S,C,N,C,C), (N,O,O,S,C,N,C,N), (N,O,O,S,C,N,N,C), (N,O,O,S,C,N,N,N), (N,O,O,S,C,S,C,C), (N,O,O,S,C,S,C,N), (N,O,O,S,C,S,N,C), (N,O,O,S,C,S,N,N), (N,O,N,C,C,C,C,C), (N,O,N,C,C,C,C,N), (N,O,N,C,C,C,N,C), (N,O,N,C,C,C,N,N), (N,O,N,C,C,N,C,C), (N,O,N,C,C,N,C,N), (N,O,N,C,C,N,N,C), (N,O,N,C,C,N,N,N), (N,O,N,C,C,S,C,C), (N,O,N,C,C,S,C,N), (N,O,N,C,C,S,N,C), (N,O,N,C,C,S,N,N), (N,O,N,N,C,C,C,C), (N,O,N,N,C,C,C,N), (N,O,N,N,C,C,N,C), (N,O,N,N,C,C,N,N), (N,O,N,N,C,N,C,C), (N,O,N,N,C,N,C,N), (N,O,N,N,C,N,N,C), (N,O,N,N,C,N,N,N), (N,O,N,N,C,S,C,C), (N,O,N,N,C,S,C,N), (N,O,N,N,C,S,N,C), (N,O,N,N,C,S,N,N), (N,O,N,S,C,C,C,C), (N,O,N,S,C,C,C,N), (N,O,N,S,C,C,N,C), (N,O,N,S,C,C,N,N), (N,O,N,S,C,N,C,C), (N,O,N,S,C,N,C,N), (N,O,N,S,C,N,N,C), (N,O,N,S,C,N,N,N), (N,O,N,S,C,S,C,C), (N,O,N,S,C,S,C,N), (N,O,N,S,C,S,N,C), (N,O,N,S,C,S,N,N), (N,O,S,C,C,C,C,C), (N,O,S,C,C,C,C,N), (N,O,S,C,C,C,N,C), (N,O,S,C,C,C,N,N), (N,O,S,N,C,C,C,C), (N,O,S,N,C,C,C,N), (N,O,S,N,C,C,N,C), (N,O,S,N,C,C,N,N), (N,O,S,S,C,C,C,C), (N,O,S,S,C,C,C,N), (N,O,S,S,C,C,N,C), (N,O,S,S,C,C,N,N), (N,N,O,C,C,C,C,C), (N,N,O,C,C,C,C,N), (N,N,O,C,C,C,N,C), (N,N,O,C,C,C,N,N), (N,N,O,C,C,N,C,C), (N,N,O,C,C,N,C,N), (N,N,O,C,C,N,N,C), (N,N,O,C,C,N,N,N), (N,N,O,C,C,S,C,C), (N,N,O,C,C,S,C,N), (N,N,O,C,C,S,N,C), (N,N,O,C,C,S,N,N), (N,N,O,N,C,C,C,C), (N,N,O,N,C,C,C,N), (N,N,O,N,C,C,N,C), (N,N,O,N,C,C,N,N), (N,N,O,N,C,N,C,C), (N,N,O,N,C,N,C,N), (N,N,O,N,C,N,N,C), (N,N,O,N,C,N,N,N), (N,N,O,N,C,S,C,C), (N,N,O,N,C,S,C,N), (N,N,O,N,C,S,N,C), (N,N,O,N,C,S,N,N), (N,N,O,S,C,C,C,C), (N,N,O,S,C,C,C,N), (N,N,O,S,C,C,N,C), (N,N,O,S,C,C,N,N), (N,N,O,S,C,N,C,C), (N,N,O,S,C,N,C,N), (N,N,O,S,C,N,N,C), (N,N,O,S,C,N,N,N), (N,N,O,S,C,S,C,C), (N,N,O,S,C,S,C,N), (N,N,O,S,C,S,N,C), (N,N,O,S,C,S,N,N), (N,N,N,C,C,C,C,C), (N,N,N,C,C,C,C,N), (N,N,N,C,C,C,N,C), (N,N,N,C,C,C,N,N), (N,N,N,C,C,N,C,C), (N,N,N,C,C,N,C,N), (N,N,N,C,C,N,N,C), (N,N,N,C,C,N,N,N), (N,N,N,C,C,S,C,C), (N,N,N,C,C,S,C,N), (N,N,N,C,C,S,N,C), (N,N,N,C,C,S,N,N), (N,N,N,N,C,C,C,C), (N,N,N,N,C,C,C,N), (N,N,N,N,C,C,N,C), (N,N,N,N,C,C,N,N), (N,N,N,N,C,N,C,C), (N,N,N,N,C,N,C,N), (N,N,N,N,C,N,N,C), (N,N,N,N,C,N,N,N), (N,N,N,N,C,S,C,C), (N,N,N,N,C,S,C,N), (N,N,N,N,C,S,N,C), (N,N,N,N,C,S,N,N), (N,N,N,S,C,C,C,C), (N,N,N,S,C,C,C,N), (N,N,N,S,C,C,N,C), (N,N,N,S,C,C,N,N), (N,N,N,S,C,N,C,C), (N,N,N,S,C,N,C,N), (N,N,N,S,C,N,N,C), (N,N,N,S,C,N,N,N), (N,N,N,S,C,S,C,C), (N,N,N,S,C,S,C,N), (N,N,N,S,C,S,N,C), (N,N,N,S,C,S,N,N), (N,N,S,C,C,C,C,C), (N,N,S,C,C,C,C,N), (N,N,S,C,C,C,N,C), (N,N,S,C,C,C,N,N), (N,N,S,N,C,C,C,C), (N,N,S,N,C,C,C,N), (N,N,S,N,C,C,N,C), (N,N,S,N,C,C,N,N), (N,N,S,S,C,C,C,C), (N,N,S,S,C,C,C,N), (N,N,S,S,C,C,N,C), (N,N,S,S,C,C,N,N), (N,S,O,C,C,C,C,C), (N,S,O,C,C,C,C,N), (N,S,O,C,C,C,N,C), (N,S,O,C,C,C,N,N), (N,S,O,C,C,N,C,C), (N,S,O,C,C,N,C,N), (N,S,O,C,C,N,N,C), (N,S,O,C,C,N,N,N), (N,S,O,C,C,S,C,C), (N,S,O,C,C,S,C,N), (N,S,O,C,C,S,N,C), (N,S,O,C,C,S,N,N), (N,S,O,N,C,C,C,C), (N,S,O,N,C,C,C,N), (N,S,O,N,C,C,N,C), (N,S,O,N,C,C,N,N), (N,S,O,N,C,N,C,C), (N,S,O,N,C,N,C,N), (N,S,O,N,C,N,N,C), (N,S,O,N,C,N,N,N), (N,S,O,N,C,S,C,C), (N,S,O,N,C,S,C,N), (N,S,O,N,C,S,N,C), (N,S,O,N,C,S,N,N), (N,S,O,S,C,C,C,C), (N,S,O,S,C,C,C,N), (N,S,O,S,C,C,N,C), (N,S,O,S,C,C,N,N), (N,S,O,S,C,N,C,C), (N,S,O,S,C,N,C,N), (N,S,O,S,C,N,N,C), (N,S,O,S,C,N,N,N), (N,S,O,S,C,S,C,C), (N,S,O,S,C,S,C,N), (N,S,O,S,C,S,N,C), (N,S,O,S,C,S,N,N), (N,S,N,C,C,C,C,C), (N,S,N,C,C,C,C,N), (N,S,N,C,C,C,N,C), (N,S,N,C,C,C,N,N), (N,S,N,C,C,N,C,C), (N,S,N,C,C,N,C,N), (N,S,N,C,C,N,N,C), (N,S,N,C,C,N,N,N), (N,S,N,C,C,S,C;C), (N,S,N,C,C,S,C,N), (N,S,N,C,C,S,N,C), (N,S,N,C,C,S,N,N), (N,S,N,N,C,C,C,C), (N,S,N,N,C,C,C,N), (N,S,N,N,C,C,N,C), (N,S,N,N,C,C,N,N), (N,S,N,N,C,N,C,C), (N,S,N,N,C,N,C,N), (N,S,N,N,C,N,N,C), (N,S,N,N,C,N,N,N), (N,S,N,N,C,S,C,C), (N,S,N,N,C,S,C,N), (N,S,N,N,C,S,N,C), (N,S,N,N,C,S,N,N), (N,S,N,S,C,C,C,C), (N,S,N,S,C,C,C,N), (N,S,N,S,C,C,N,C), (N,S,N,S,C,C,N,N), (N,S,N,S,C,N,C,C), (N,S,N,S,C,N,C,N), (N,S,N,S,C,N,N,C), (N,S,N,S,C,N,N,N), (N,S,N,S,C,S,C,C), (N,S,N,S,C,S,C,N), (N,S,N,S,C,S,N,C), (N,S,N,S,C,S,N,N), (N,S,S,C,C,C,C,C), (N,S,S,C,C,C,C,N), (N,S,S,C,C,C,N,C), (N,S,S,C,C,C,N,N), (N,S,S,N,C,C,C,C), (N,S,S,N,C,C,C,N), (N,S,S,N,C,C,N,C), (N,S,S,N,C,C,N,N), (N,S,S,S,C,C,C,C), (N,S,S,S,C,C,C,N), (N,S,S,S,C,C,N,C), (N,S,S,S,C,C,N,N), (S,O,O,C,C,C,C,C), (S,O,O,C,C,C,C,N), (S,O,O,C,C,C,N,C), (S,O,O,C,C,C,N,N), (S,O,O,C,C,N,C,C), (S,O,O,C,C,N,C,N), (S,O,O,C,C,N,N,C), (S,O,O,C,C,N,N,N), (S,O,O,C,C,S,C,C), (S,O,O,C,C,S,C,N), (S,O,O,C,C,S,N,C),

(S,O,O,C,C,S,N,N), (S,O,N,C,C,C,C,C), (S,O,N,C,C,C,C,N), (S,O,N,C,C,C,N,C), (S,O,N,C,C,C,N,N), (S,O,N,C,C,N,C,C), (S,O,N,C,C,N,C,N), (S,O,N,C,C,N,N,C), (S,O,N,C,C,N,N,N), (S,O,N,C,C,S,C,C), (S,O,N,C,C,S,C,N), (S,O,N,C,C,S,N,C), (S,O,N,C,C,S,N,N), (S,O,S,C,C,C,C,C), (S,O,S,C,C,C,C,N), (S,O,S,C,C,C,N,C), (S,O,S,C,C,C,N,N), (S,N,O,C,C,C,C,C), (S,N,O,C,C,C,C,N), (S,N,O,C,C,C,N,C), (S,N,O,C,C,C,N,N), (S,N,O,C,C,N,C,C), (S,N,O,C,C,N,C,N), (S,N,O,C,C,N,N,C), (S,N,O,C,C,N,N,N), (S,N,O,C,C,S,C,C), (S,N,O,C,C,S,C,N), (S,N,O,C,C,S,N,C), (S,N,O,C,C,S,N,N), (S,N,N,C,C,C,C,C), (S,N,N,C,C,C,C,N), (S,N,N,C,C,C,N,C), (S,N,N,C,C,C,N,N), (S,N,N,C,C,N,C,C), (S,N,N,C,C,N,C,N), (S,N,N,C,C,N,N,C), (S,N,N,C,C,N,N,N), (S,N,N,C,C,S,C,C), (S,N,N,C,C,S,C,N), (S,N,N,C,C,S,N,C), (S,N,N,C,C,S,N,N), (S,N,S,C,C,C,C,C), (S,N,S,C,C,C,C,N), (S,N,S,C,C,C,N,C), (S,N,S,C,C,C,N,N), (S,S,O,C,C,C,C,C), (S,S,O,C,C,C,C,N), (S,S,O,C,C,C,N,C), (S,S,O,C,C,C,N,N), (S,S,O,C,C,N,C,C), (S,S,O,C,C,N,C,N), (S,S,O,C,C,N,N,C), (S,S,O,C,C,N,N,N), (S,S,O,C,C,S,C,C), (S,S,O,C,C,S,C,N), (S,S,O,C,C,S,N,C), (S,S,O,C,C,S,N,N), (S,S,N,C,C,C,C,C), (S,S,N,C,C,C,C,N), (S,S,N,C,C,C,N,C), (S,S,N,C,C,C,N,N), (S,S,N,C,C,N,C,C), (S,S,N,C,C,N,C,N), (S,S,N,C,C,N,N,C), (S,S,N,C,C,N,N,N), (S,S,N,C,C,S,C,C), (S,S,N,C,C,S,C,N), (S,S,N,C,C,S,N,C), (S,S,N,C,C,S,N,N), (S,S,S,C,C,C,C,C), (S,S,S,C,C,C,C,N), (S,S,S,C,C,C,N,C), and (S,S,S,C,C,C,N,N).

Among the compounds having the substructures described above, those having the combination of the respective bonds (bond a1 to bond a7) shown below are preferred particularly (designation "-" here means a single bond, and designation "=" means a double bond). Combination (a1,a2,a3,a4,a5,a6,a7) = (-,-,-,-,-, =,-), (-,-,-,-,=,-,-), (-,-,-,-,=,-,=), (-, -,-,=,-,=,-), (-,-,=,-,-,=,-), (-,-,=,-, =,-,-), (-,-,=,=,-,=,-), (-,=,-,-,-,-,-), (-,=,-,-,-,-,=), (-,=,-,=,-,-,-), (-,=,-, =,-,-,=), (-,=, =,-,-,-,-), (-,=,=,=,-,-, -), (=,-,-,-,-,=,-) (=,-,-,-,=,-,-), (=, -,-,-,=,-,=), (=,-,=,-,-,=,-), (=,-,=,-, =,-,-), (=,=,-,-,-,-,-), (=,=,-,-,-,-,=), and (=,=,=,-,-,-,-)

In this case, a compound thus searched out can chelate both of two divalent metal ions and can form 6-membered chelate rings with both of two divalent metal ions as shown below.

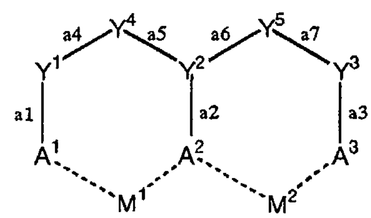

wherein $M^1$ and $M^2$ are divalent metal ions and other symbols are defined as described above.

In another case, a compound having a substructure represented by Formula (II):

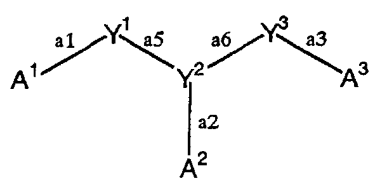

(II)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
bond a1 to bond a3, bond a5 and bond a6 are each independently a single bond or a double bond,
one of bond a2, bond a5 and bond a6 is a double bond, and the other two are single bonds,
bond a1, bond a3, bond a5 and bond a6 may each independently constitute a part of the ring,
provided that any adjacent bonds of bond a1 to bond a3, bond a5 and bond a6 are not double bonds at the same time, may be searched for.

Among the compounds having the substructures described above, the following combinations of the respective atoms (atom $A^1$ to atom $A^3$, atom $Y^1$ to $Y^3$) are preferred particularly.
Combination ($A^1$,$A^2$,$A^3$,$Y^1$,$Y^2$,$Y^3$) = (O,O,O,C,C,C), (O,O,O,C,C,N), (O,O,O,C,C,S), (O,O,O,N,C,C), (O,O,O,N,C,N), (O,O,O,N,C,S), (O,O,O,S,C,C), (O,O,O,S,C,N), (O,O,O,S,C,S), (O,O,N,C,C,C), (O,O,N,C,C,N), (O,O,N,C,C,S), (O,O,N,N,C,C), (O,O,N,N,C,N), (O,O,N,N,C,S), (O,O,N,S,C,C), (O,O,N,S,C,N), (O,O,N,S,C,S), (O,O,S,C,C,C), (O,O,S,N,C,C), (O,O,S,S,C,C), (O,N,O,C,C,C), (O,N,O,C,C,N), (O,N,O,C,C,S), (O,N,O,N,C,C), (O,N,O,N,C,N), (O,N,O,N,C,S),

(O,N,O,S,C,C), (O,N,O,S,C,N), (O,N,O,S,C,S), (O,N,N,C,C,C), (O,N,N,C,C,N), (O,N,N,C,C,S), (O,N,N,N,C,C), (O,N,N,N,C,N), (O,N,N,N,C,S), (O,N,N,S,C,C), (O,N,N,S,C,N), (O,N,N,S,C,S), (O,N,S,C,C,C), (O,N,S,N,C,C), (O,N,S,S,C,C), (O,S,O,C,C,C), (O,S,O,C,C,N), (O,S,O,C,C,S), (O,S,O,N,C,C), (O,S,O,N,C,N), (O,S,O,N,C,S), (O,S,O,S,C,C), (O,S,O,S,C,N), (O,S,O,S,C,S), (O,S,N,C,C,C), (O,S,N,C,C,N), (O,S,N,C,C,S), (O,S,N,N,C,C), (O,S,N,N,C,N), (O,S,N,N,C,S), (O,S,N,S,C,C), (O,S,N,S,C,N), (O,S,N,S,C,S), (O,S,S,C,C,C), (O,S,S,N,C,C), (O,S,S,S,C,C), (N,O,O,C,C,C), (N,O,O,C,C,N), (N,O,O,C,C,S), (N,O,O,N,C,C), (N,O,O,N,C,N), (N,O,O,N,C,S), (N,O,O,S,C,C), (N,O,O,S,C,N), (N,O,O,S,C,S), (N,O,N,C,C,C), (N,O,N,C,C,N), (N,O,N,C,C,S), (N,O,N,N,C,C), (N,O,N,N,C,N), (N,O,N,N,C,S), (N,O,N,S,C,C), (N,O,N,S,C,N), (N,O,N,S,C,S), (N,O,S,C,C,C), (N,O,S,N,C,C), (N,O,S,S,C,C), (N,N,O,C,C,C), (N,N,O,C,C,N), (N,N,O,C,C,S), (N,N,O,N,C,C), (N,N,O,N,C,N), (N,N,O,N,C,S), (N,N,O,S,C,C), (N,N,O,S,C,N), (N,N,O,S,C,S), (N,N,N,C,C,C), (N,N,N,C,C,N), (N,N,N,C,C,S), (N,N,N,N,C,C), (N,N,N,N,C,N), (N,N,N,N,C,S), (N,N,N,S,C,C), (N,N,N,S,C,N), (N,N,N,S,C,S), (N,N,S,C,C,C), (N,N,S,N,C,C), (N,N,S,S,C,C), (N,S,O,C,C,C), (N,S,O,C,C,N), (N,S,O,C,C,S), (N,S,O,N,C,C), (N,S,O,N,C,N), (N,S,O,N,C,S), (N,S,O,S,C,C), (N,S,O,S,C,N), (N,S,O,S,C,S), (N,S,N,C,C,C), (N,S,N,C,C,N), (N,S,N,C,C,S), (N,S,N,N,C,C), (N,S,N,N,C,N), (N,S,N,N,C,S), (N,S,N,S,C,C), (N,S,N,S,C,N), (N,S,N,S,C,S), (N,S,S,C,C,C), (N,S,S,N,C,C), (N,S,S,S,C,C), (S,O,O,C,C,C), (S,O,O,C,C,N), (S,O,O,C,C,S), (S,O,N,C,C,C), (S,O,N,C,C,N), (S,O,N,C,C,S), (S,O,S,C,C,C), (S,N,O,C,C,C), (S,N,O,C,C,N), (S,N,O,C,C,S), (S,N,N,C,C,C), (S,N,N,C,C,N), (S,N,N,C,C,S), (S,N,S,C,C,C), (S,S,O,C,C,C), (S,S,O,C,C,N), (S,S,O,C,C,S), (S,S,N,C,C,C), (S,S,N,C,C,N), (S,S,N,C,C,S), and (S,S,S,C,C,C).

Among the compounds having the substructures described above, those having the combination of the respective bonds (bond a1 to bond a3, bond a5, bond a6) shown below are preferred particularly.

Combination (a1,a2,a3,a5,a6) = (-,-,-,-,=), (-, -,-,=,-), (-,-,=,=,-), (-,=,-,-,-), (-,=, =,-,-), (=,-,-,-,=), (=,=,-,-,-), and (=,=, =,-,-).

In this case, a compound thus searched out can chelate both of two divalent metal ions and can form 5-membered chelate rings with both of two divalent metal ions as shown below.

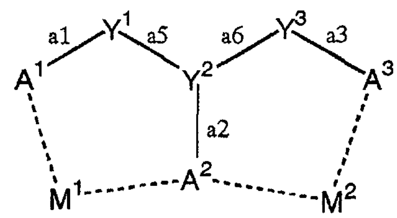

wherein $M^1$ and $M^2$ are divalent metal ions and other symbols are defined as described above.

In another case, a compound having a substructure represented by Formula (III):

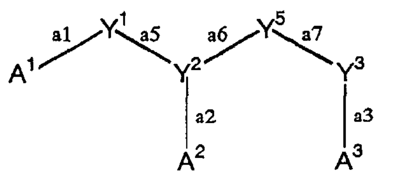

(III)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion,

atom $Y^1$ and atom $Y^3$ are each independently a carbon atom, nitrogen atom or sulfur atom,

atom $Y^2$ is a carbon atom,

atom $Y^5$ is a carbon atom or nitrogen atom,

bond a1 to bond a3 and bond a5 to bond a7 are each independently a single bond or a double bond,

one of bond a2, bond a5 and bond a6 is a double bond, and the other two are single bonds,

bond a1, bond a3 and bond a5 to bond a7 may each independently constitute a part of the ring,

provided that any adjacent bonds of bond a1 to bond a3 and bond a5 to bond a7 are not double bonds at the same time, may be searched for.

Among the compounds having the substructures described above, the following combinations of the respective atoms (atom $A^1$ to atom $A^3$, atom $Y^1$ to atom $Y^3$, atom $Y^5$) are preferred particularly. Combination ($A^1$,$A^2$,$A^3$,$Y^1$,$Y^2$,$Y^3$,$Y^5$) = (O,O,O,C,C,C,C), (O,O,O,C,C,C,N), (O,O,O,C,C,N,C), (O,O,O,C,C,N,N), (O,O,O,C,C,S,C), (O,O,O,C,C,S,N), (O,O,O,N,C,C,C), (O,O,O,N,C,C,N), (O,O,O,N,C,N,C), (O,O,O,N,C,N,N), (O,O,O,N,C,S,C), (O,O,O,N,C,S,N), (O,O,O,S,C,C,C), (O,O,O,S,C,C,N), (O,O,O,S,C,N,C), (O,O,O,S,C,N,N), (O,O,O,S,C,S,C), (O,O,O,S,C,S,N), (O,O,N,C,C,C,C), (O,O,N,C,C,C,N), (O,O,N,C,C,N,C), (O,O,N,C,C,N,N), (O,O,N,C,C,S,C), (O,O,N,C,C,S,N), (O,O,N,N,C,C,C), (O,O,N,N,C,C,N), (O,O,N,N,C,N,C), (O,O,N,N,C,N,N), (O,O,N,N,C,S,C), (O,O,N,N,C,S,N), (O,O,N,S,C,C,C), (O,O,N,S,C,C,N), (O,O,N,S,C,N,C), (O,O,N,S,C,N,N), (O,O,N,S,C,S,C), (O,O,N,S,C,S,N), (O,O,S,C,C,C,C), (O,O,S,C,C,C,N), (O,O,S,N,C,C,C), (O,O,S,N,C,C,N), (O,O,S,S,C,C,C), (O,O,S,S,C,C,N), (O,N,O,C,C,C,C), (O,N,O,C,C,C,N), (O,N,O,C,C,N,C), (O,N,O,C,C,N,N), (O,N,O,C,C,S,C), (O,N,O,C,C,S,N), (O,N,O,N,C,C,C), (O,N,O,N,C,C,N), (O,N,O,N,C,N,C), (O,N,O,N,C,N,N), (O,N,O,N,C,S,C), (O,N,O,N,C,S,N), (O,N,O,S,C,C,C), (O,N,O,S,C,C,N), (O,N,O,S,C,N,C), (O,N,O,S,C,N,N), (O,N,O,S,C,S,C), (O,N,O,S,C,S,N), (O,N,N,C,C,C,C), (O,N,N,C,C,C,N), (O,N,N,C,C,N,C), (O,N,N,C,C,N,N), (O,N,N,C,C,S,C), (O,N,N,C,C,S,N), (O,N,N,N,C,C,C), (O,N,N,N,C,C,N), (O,N,N,N,C,N,C), (O,N,N,N,C,N,N), (O,N,N,N,C,S,C), (O,N,N,N,C,S,N), (O,N,N,S,C,C,C), (O,N,N,S,C,C,N), (O,N,N,S,C,N,C), (O,N,N,S,C,N,N), (O,N,N,S,C,S,C), (O,N,N,S,C,S,N), (O,N,S,C,C,C,C), (O,N,S,C,C,C,N), (O,N,S,N,C,C,C), (O,N,S,N,C,C,N), (O,N,S,S,C,C,C), (O,N,S,S,C,C,N), (O,S,O,C,C,C,C), (O,S,O,C,C,C,N), (O,S,O,C,C,N,C), (O,S,O,C,C,N,N), (O,S,O,C,C,S,C), (O,S,O,C,C,S,N), (O,S,O,N,C,C,C), (O,S,O,N,C,C,N), (O,S,O,N,C,N,C), (O,S,O,N,C,N,N), (O,S,O,N,C,S,C), (O,S,O,N,C,S,N), (O,S,O,S,C,C,C), (O,S,O,S,C,C,N), (O,S,O,S,C,N,C), (O,S,O,S,C,N,N), (O,S,O,S,C,S,C), (O,S,O,S,C,S,N), (O,S,N,C,C,C,C), (O,S,N,C,C,C,N), (O,S,N,C,C,N,C), (O,S,N,C,C,N,N), (O,S,N,C,C,S,C), (O,S,N,C,C,S,N), (O,S,N,N,C,C,C), (O,S,N,N,C,C,N), (O,S,N,N,C,N,C), (O,S,N,N,C,N,N), (O,S,N,N,C,S,C), (O,S,N,N,C,S,N), (O,S,N,S,C,C,C), (O,S,N,S,C,C,N), (O,S,N,S,C,N,C), (O,S,N,S,C,N,N), (O,S,N,S,C,S,C), (O,S,N,S,C,S,N), (O,S,S,C,C,C,C), (O,S,S,C,C,C,N), (O,S,S,N,C,C,C), (O,S,S,N,C,C,N), (O,S,S,S,C,C,C), (O,S,S,S,C,C,N), (N,O,O,C,C,C,C), (N,O,O,C,C,C,N), (N,O,O,C,C,N,C), (N,O,O,C,C,N,N), (N,O,O,C,C,S,C), (N,O,O,C,C,S,N), (N,O,O,N,C,C,C), (N,O,O,N,C,C,N), (N,O,O,N,C,N,C), (N,O,O,N,C,N,N), (N,O,O,N,C,S,C), (N,O,O,N,C,S,N), (N,O,O,S,C,C,C), (N,O,O,S,C,C,N), (N,O,O,S,C,N,C), (N,O,O,S,C,N,N), (N,O,O,S,C,S,C), (N,O,O,S,C,S,N), (N,O,N,C,C,C,C), (N,O,N,C,C,C,N), (N,O,N,C,C,N,C), (N,O,N,C,C,N,N), (N,O,N,C,C,S,C), (N,O,N,C,C,S,N), (N,O,N,N,C,C,C), (N,O,N,N,C,C,N), (N,O,N,N,C,N,C), (N,O,N,N,C,N,N), (N,O,N,N,C,S,C), (N,O,N,N,C,S,N), (N,O,N,S,C,C,C), (N,O,N,S,C,C,N), (N,O,N,S,C,N,C), (N,O,N,S,C,N,N), (N,O,N,S,C,S,C), (N,O,N,S,C,S,N), (N,O,S,C,C,C,C), (N,O,S,C,C,C,N), (N,O,S,N,C,C,C), (N,O,S,N,C,C,N), (N,O,S,S,C,C,C), (N,O,S,S,C,C,N), (N,N,O,C,C,C,C), (N,N,O,C,C,C,N), (N,N,O,C,C,N,C), (N,N,O,C,C,N,N), (N,N,O,C,C,S,C), (N,N,O,C,C,S,N), (N,N,O,N,C,C,C), (N,N,O,N,C,C,N), (N,N,O,N,C,N,C), (N,N,O,N,C,N,N), (N,N,O,N,C,S,C), (N,N,O,N,C,S,N), (N,N,O,S,C,C,C), (N,N,O,S,C,C,N), (N,N,O,S,C,N,C), (N,N,O,S,C,N,N), (N,N,O,S,C,S,C), (N,N,O,S,C,S,N), (N,N,N,C,C,C,C), (N,N,N,C,C,C,N), (N,N,N,C,C,N,C), (N,N,N,C,C,N,N), (N,N,N,C,C,S,C), (N,N,N,C,C,S,N), (N,N,N,N,C,C,C), (N,N,N,N,C,C,N), (N,N,N,N,C,N,C), (N,N,N,N,C,N,N), (N,N,N,N,C,S,C), (N,N,N,N,C,S,N), (N,N,N,S,C,C,C), (N,N,N,S,C,C,N), (N,N,N,S,C,N,C), (N,N,N,S,C,N,N), (N,N,N,S,C,S,C), (N,N,N,S,C,S,N), (N,N,S,C,C,C,C), (N,N,S,C,C,C,N), (N,N,S,N,C,C,C), (N,N,S,N,C,C,N), (N,N,S,S,C,C,C), (N,N,S,S,C,C,N), (N,S,O,C,C,C,C), (N,S,O,C,C,C,N), (N,S,O,C,C,N,C), (N,S,O,C,C,N,N), (N,S,O,C,C,S,C), (N,S,O,C,C,S,N), (N,S,O,N,C,C,C), (N,S,O,N,C,C,N), (N,S,O,N,C,N,C), (N,S,O,N,C,N,N), (N,S,O,N,C,S,C), (N,S,O,N,C,S,N), (N,S,O,S,C,C,C), (N,S,O,S,C,C,N), (N,S,O,S,C,N,C), (N,S,O,S,C,N,N), (N,S,O,S,C,S,C), (N,S,O,S,C,S,N), (N,S,N,C,C,C,C), (N,S,N,C,C,C,N), (N,S,N,C,C,N,C), (N,S,N,C,C,N,N), (N,S,N,C,C,S,C), (N,S,N,C,C,S,N), (N,S,N,N,C,C,C), (N,S,N,N,C,C,N), (N,S,N,N,C,N,C), (N,S,N,N,C,N,N), (N,S,N,N,C,S,C), (N,S,N,N,C,S,N), (N,S,N,S,C,C,C), (N,S,N,S,C,C,N), (N,S,N,S,C,N,C), (N,S,N,S,C,N,N), (N,S,N,S,C,S,C), (N,S,N,S,C,S,N), (N,S,S,C,C,C,C), (N,S,S,C,C,C,N), (N,S,S,N,C,C,C), (N,S,S,N,C,C,N), (N,S,S,S,C,C,C), (N,S,S,S,C,C,N), (S,O,O,C,C,C,C), (S,O,O,C,C,C,N), (S,O,O,C,C,N,C), (S,O,O,C,C,N,N), (S,O,O,C,C,S,C), (S,O,O,C,C,S,N), (S,O,N,C,C,C,C), (S,O,N,C,C,C,N), (S,O,N,C,C,N,C), (S,O,N,C,C,N,N), (S,O,N,C,C,S,C), (S,O,N,C,C,S,N), (S,O,S,C,C,C,C), (S,O,S,C,C,C,N), (S,N,O,C,C,C,C), (S,N,O,C,C,C,N), (S,N,O,C,C,N,C), (S,N,O,C,C,N,N), (S,N,O,C,C,S,C), (S,N,O,C,C,S,N), (S,N,N,C,C,C,C), (S,N,N,C,C,C,N), (S,N,N,C,C,N,C), (S,N,N,C,C,N,N), (S,N,N,C,C,S,C), (S,N,N,C,C,S,N), (S,N,S,C,C,C,C), (S,N,S,C,C,C,N), (S,S,O,C,C,C,C), (S,S,O,C,C,C,N), (S,S,O,C,C,N,C), (S,S,O,C,C,N,N), (S,S,O,C,C,S,C), (S,S,O,C,C,S,N), (S,S,N,C,C,C,C), (S,S,N,C,C,C,N), (S,S,N,C,C,N,C), (S,S,N,C,C,N,N), (S,S,N,C,C,S,C), (S,S,N,C,C,S,N), (S,S,S,C,C,C,C), and (S,S,S,C,C,C,N).

Among the compounds having the substructures described above, those having the combination of the respective bonds (bond a1 to bond a3, bond a5 to bond a7) shown below are preferred particularly.
Combination (a1,a2,a3,a5,a6,a7) = (-,-,-,-,=,-), (-,-,-,=,-,-), (-,-,-,=,-,=), (-,-,=,-,=, -), (-,-,=,=,-,-), (-,=,-,-,-,-), (-,=,-, -,-,=), (-,=,=,-,-,-), (=,-,-,-,=,-), (=, -,=,-,=,-), (=,=,-,-,-,-), (=,=,-,-,-,=), and (=,=,=,-,-,-).

In this case, a compound thus searched out can chelate both of two divalent metal ions and can form one 5-membered chelate ring and one 6-membered chelate ring with both of two divalent metal ions as shown below.

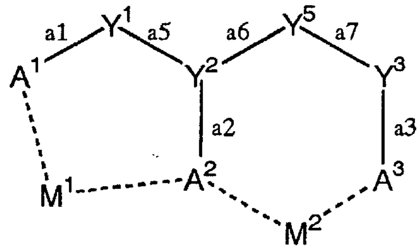

wherein $M^1$ and $M^2$ are divalent metal ions and other symbols are defined as described above.

In another case, a compound having a substructure represented by Formula (IV):

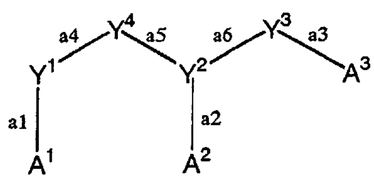

(IV)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
atom $Y^5$ is a carbon atom or nitrogen atom,
bond a1 to bond a6 are each independently a single bond or a double bond,
one of bond a2, bond a5 and bond a6 is a double bond, and the other two are single bonds,
bond a1 and bond a3 to bond a6 may each independently constitute a part of the ring,
provided that any adjacent bonds of bond a1 to bond a6 are not double bonds at the same time,
may be searched for.

Among the compounds having the substructures described above, the following combinations of the respective atoms (atom $A^1$ to atom $A^3$, atom $Y^1$ to atom $Y^4$) are preferred particularly.
Combination ($A^1$,$A^2$,$A^3$,$Y^1$,$Y^2$,$Y^3$,$Y^4$) = (O,O,O,C,C,C,C), (O,O,O,C,C,C,N), (O,O,O,C,C,N,C), (O,O,O,C,C,N,N), (O,O,O,C,C,S,C), (O,O,O,C,C,S,N), (O,O,O,N,C,C,C), (O,O,O,N,C,C,N), (O,O,O,N,C,N,C), (O,O,O,N,C,N,N), (O,O,O,N,C,S,C), (O,O,O,N,C,S,N), (O,O,O,S,C,C,C), (O,O,O,S,C,C,N), (O,O,O,S,C,N,C), (O,O,O,S,C,N,N), (O,O,O,S,C,S,C), (O,O,O,S,C,S,N), (O,O,N,C,C,C,C), (O,O,N,C,C,C,N), (O,O,N,C,C,N,C), (O,O,N,C,C,N,N), (O,O,N,C,C,S,C), (O,O,N,C,C,S,N), (O,O,N,N,C,C,C), (O,O,N,N,C,C,N), (O,O,N,N,C,N,C), (O,O,N,N,C,N,N), (O,O,N,N,C,S,C), (O,O,N,N,C,S,N), (O,O,N,S,C,C,C), (O,O,N,S,C,C,N), (O,O,N,S,C,N,C), (O,O,N,S,C,N,N), (O,O,N,S,C,S,C), (O,O,N,S,C,S,N), (O,O,S,C,C,C,C), (O,O,S,C,C,C,N), (O,O,S,N,C,C,C), (O,O,S,N,C,C,N), (O,O,S,S,C,C,C), (O,O,S,S,C,C,N), (O,N,O,C,C,C,C), (O,N,O,C,C,C,N), (O,N,O,C,C,N,C), (O,N,O,C,C,N,N), (O,N,O,C,C,S,C), (O,N,O,C,C,S,N), (O,N,O,N,C,C,C), (O,N,O,N,C,C,N), (O,N,O,N,C,N,C), (O,N,O,N,C,N,N), (O,N,O,N,C,S,C), (O,N,O,N,C,S,N), (O,N,O,S,C,C,C), (O,N,O,S,C,C,N), (O,N,O,S,C,N,C), (O,N,O,S,C,N,N), (O,N,O,S,C,S,C), (O,N,O,S,C,S,N), (O,N,N,C,C,C,C), (O,N,N,C,C,C,N), (O,N,N,C,C,N,C), (O,N,N,C,C,N,N), (O,N,N,C,C,S,C), (O,N,N,C,C,S,N), (O,N,N,N,C,C,C), (O,N,N,N,C,C,N), (O,N,N,N,C,N,C), (O,N,N,N,C,N,N), (O,N,N,N,C,S,C), (O,N,N,N,C,S,N), (O,N,N,S,C,C,C), (O,N,N,S,C,C,N), (O,N,N,S,C,N,C), (O,N,N,S,C,N,N), (O,N,N,S,C,S,C), (O,N,N,S,C,S,N), (O,N,S,C,C,C,C), (O,N,S,C,C,C,N), (O,N,S,N,C,C,C), (O,N,S,N,C,C,N), (O,N,S,S,C,C,C), (O,N,S,S,C,C,N), (O,S,O,C,C,C,C), (O,S,O,C,C,C,N), (O,S,O,C,C,N,C), (O,S,O,C,C,N,N), (O,S,O,C,C,S,C), (O,S,O,C,C,S,N), (O,S,O,N,C,C,C), (O,S,O,N,C,C,N), (O,S,O,N,C,N,C), (O,S,O,N,C,N,N), (O,S,O,N,C,S,C), (O,S,O,N,C,S,N), (O,S,O,S,C,C,C), (O,S,O,S,C,C,N), (O,S,O,S,C,N,C), (O,S,O,S,C,N,N), (O,S,O,S,C,S,C), (O,S,O,S,C,S,N), (O,S,N,C,C,C,C), (O,S,N,C,C,C,N), (O,S,N,C,C,N,C), (O,S,N,C,C,N,N), (O,S,N,C,C,S,C), (O,S,N,C,C,S,N), (O,S,N,N,C,C,C), (O,S,N,N,C,C,N), (O,S,N,N,C,N,C), (O,S,N,N,C,N,N), (O,S,N,N,C,S,C), (O,S,N,N,C,S,N), (O,S,N,S,C,C,C), (O,S,N,S,C,C,N), (O,S,N,S,C,N,C), (O,S,N,S,C,N,N), (O,S,N,S,C,S,C), (O,S,N,S,C,S,N), (O,S,S,C,C,C,C), (O,S,S,C,C,C,N), (O,S,S,N,C,C,C), (O,S,S,N,C,C,N), (O,S,S,S,C,C,C), (O,S,S,S,C,C,N), (N,O,O,C,C,C,C), (N,O,O,C,C,C,N), (N,O,O,C,C,N,C), (N,O,O,C,C,N,N), (N,O,O,C,C,S,C), (N,O,O,C,C,S,N), (N,O,O,N,C,C,C), (N,O,O,N,C,C,N), (N,O,O,N,C,N,C), (N,O,O,N,C,N,N), (N,O,O,N,C,S,C), (N,O,O,N,C,S,N), (N,O,O,S,C,C,C), (N,O,O,S,C,C,N), (N,O,O,S,C,N,C), (N,O,O,S,C,N,N), (N,O,O,S,C,S,C), (N,O,O,S,C,S,N), (N,O,N,C,C,C,C), (N,O,N,C,C,C,N), (N,O,N,C,C,N,C), (N,O,N,C,C,N,N), (N,O,N,C,C,S,C), (N,O,N,C,C,S,N), (N,O,N,N,C,C,C), (N,O,N,N,C,C,N), (N,O,N,N,C,N,C), (N,O,N,N,C,N,N), (N,O,N,N,C,S,C), (N,O,N,N,C,S,N), (N,O,N,S,C,C,C), (N,O,N,S,C,C,N), (N,O,N,S,C,N,C), (N,O,N,S,C,N,N), (N,O,N,S,C,S,C), (N,O,N,S,C,S,N), (N,O,S,C,C,C,C), (N,O,S,C,C,C,N), (N,O,S,N,C,C,C), (N,O,S,N,C,C,N), (N,O,S,S,C,C,C), (N,O,S,S,C,C,N), (N,N,O,C,C,C,C), (N,N,O,C,C,C,N), (N,N,O,C,C,N,C), (N,N,O,C,C,N,N), (N,N,O,C,C,S,C), (N,N,O,C,C,S,N), (N,N,O,N,C,C,C), (N,N,O,N,C,C,N), (N,N,O,N,C,N,C), (N,N,O,N,C,N,N), (N,N,O,N,C,S,C), (N,N,O,N,C,S,N), (N,N,O,S,C,C,C), (N,N,O,S,C,C,N), (N,N,O,S,C,N,C), (N,N,O,S,C,N,N), (N,N,O,S,C,S,C), (N,N,O,S,C,S,N), (N,N,N,C,C,C,C), (N,N,N,C,C,C,N), (N,N,N,C,C,N,C), (N,N,N,C,C,N,N), (N,N,N,C,C,S,C), (N,N,N,C,C,S,N), (N,N,N,N,C,C,C), (N,N,N,N,C,C,N), (N,N,N,N,C,N,C), (N,N,N,N,C,N,N), (N,N,N,N,C,S,C), (N,N,N,N,C,S,N), (N,N,N,S,C,C,C), (N,N,N,S,C,C,N), (N,N,N,S,C,N,C), (N,N,N,S,C,N,N), (N,N,N,S,C,S,C), (N,N,N,S,C,S,N), (N,N,S,C,C,C,C), (N,N,S,C,C,C,N), (N,N,S,N,C,C,C), (N,N,S,N,C,C,N), (N,N,S,S,C,C,C), (N,N,S,S,C,C,N), (N,S,O,C,C,C,C), (N,S,O,C,C,C,N), (N,S,O,C,C,N,C), (N,S,O,C,C,N,N), (N,S,O,C,C,S,C), (N,S,O,C,C,S,N), (N,S,O,N,C,C,C), (N,S,O,N,C,C,N) (N,S,O,N,C,N,C), (N,S,O,N,C,N,N), (N,S,O,N,C,S,C), (N,S,O,N,C,S,N), (N,S,O,S,C,C,C), (N,S,O,S,C,C,N), (N,S,O,S,C,N,C), (N,S,O,S,C,N,N), (N,S,O,S,C,S,C), (N,S,O,S,C,S,N), (N,S,N,C,C,C,C), (N,S,N,C,C,C,N), (N,S,N,C,C,N,C), (N,S,N,C,C,N,N), (N,S,N,C,C,S,C), (N,S,N,C,C,S,N), (N,S,N,N,C,C,C), (N,S,N,N,C,C,N), (N,S,N,N,C,N,C), (N,S,N,N,C,N,N), (N,S,N,N,C,S,C), (N,S,N,N,C,S,N), (N,S,N,S,C,C,C), (N,S,N,S,C,C,N), (N,S,N,S,C,N,C), (N,S,N,S,C,N,N), (N,S,N,S,C,S,C), (N,S,N,S,C,S,N), (N,S,S,C,C,C,C), (N,S,S,C,C,C,N), (N,S,S,N,C,C,C), (N,S,S,N,C,C,N), (N,S,S,S,C,C,C), (N,S,S,S,C,C,N), (S,O,O,C,C,C,C), (S,O,O,C,C,C,N), (S,O,O,C,C,N,C), (S,O,O,C,C,N,N), (S,O,O,C,C,S,C), (S,O,O,C,C,S,N), (S,O,N,C,C,C,C), (S,O,N,C,C,C,N), (S,O,N,C,C,N,C), (S,O,N,C,C,N,N), (S,O,N,C,C,S,C), (S,O,N,C,C,S,N), (S,O,S,C,C,C,C), (S,O,S,C,C,C,N), (S,N,O,C,C,C,C), (S,N,O,C,C,C,N), (S,N,O,C,C,N,C), (S,N,O,C,C,N,N), (S,N,O,C,C,S,C), (S,N,O,C,C,S,N), (S,N,N,C,C,C,C), (S,N,N,C,C,C,N), (S,N,N,C,C,N,C), (S,N,N,C,C,N,N), (S,N,N,C,C,S,C), (S,N,N,C,C,S,N), (S,N,S,C,C,C,C), (S,N,S,C,C,C,N), (S,S,O,C,C,C,C), (S,S,O,C,C,C,N), (S,S,O,C,C,N,C), (S,S,O,C,C,N,N), (S,S,O,C,C,S,C), (S,S,O,C,C,S,N), (S,S,N,C,C,C,C), (S,S,N,C,C,C,N), (S,S,N,C,C,N,C), (S,S,N,C,C,N,N), (S,S,N,C,C,S,C), (S,S,N,C,C,S,N), (S,S,S,C,C,C,C), and (S,S,S,C,C,C,N).

Among the compounds having the substructures described above, those having the combination of the respective bonds (bond a1 to bond a6) shown below are preferred particularly.
Combination (a1,a2,a3,a4,a5,a6) = (-,-,-,-,-,=), (-,-,-,-,=,-), (-,-,-,=,-,=), (-,-,=,-,-, =), (-,-,=,-,=,-), (-,-,=,=,-,=), (-,=,-, -,-,-), (-,=,-,=,-,-), (-,=,=,-,-,-), (-, =,=,=,-,-), (=,-,-,-,-,=), (=,-,-,-,=,-), (=,-,=,-,-,=), (=,-,=,-,=,-), (=,=,-,-,-, -), and (=,=,=,-,-,-).

In this case, a compound thus searched out can chelate both of two divalent metal ions and can form one 6-membered chelate ring and one 5-membered chelate ring with both of two divalent metal ions as shown below.

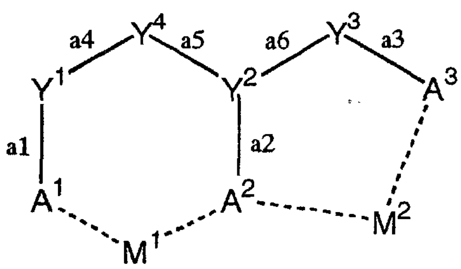

wherein $M^1$ and $M^2$ are divalent metal ions and other symbols are defined as described above.

While a compound thus searched out can be selected or synthesized and then examined for its enzyme inhibition activity, it is further possible to estimate the binding energy between the compound and the enzyme using a computer. For example, the 3-D structures of the compound searched out above and the enzyme are loaded to a molecular modeling software such as Sybyl (Tripos, Inc., (St. Lewis)), InsightII (Molecular Simulation INC., (San Diego)) or Quanta (Molecular Simulations, INC., (San Diego), docked appropriately, and then subjected to energy minimization. While compounds which cause steric repulsion to the enzyme can previously be excluded in this procedure, it is also possible to exclude them by imparting the previous search with a certain prerequisite. It is also possible that by using a simulation technology such as molecular dynamics simulation the stability of the complex consisting of an enzyme and a compound is evaluated whereby selecting an optimum compound.

A compound having the above formula to be searched for as a substructure can be an inhibitor of an enzyme having two divalent metal ions as an active center.

A substructure means here a structure which is a part of a compound. A bond in the substructure may be a single bond or a double bond, and may also be a constituent of a ring.

A part other than the substructure corresponding to the formula to be searched for may have any structure. Thus, any structure which is possible in organic chemistry may be acceptable.

An atom as a constituent of a part other than the substructure, a carbon atom, hydrogen atom, sulfur atom,

nitrogen atom, phosphorus atom, oxygen atom, boron atom, fluorine atom, chlorine atom, bromine atom, iodine atom, sodium atom, lithium atom, magnesium atom, potassium atom, calcium atom, barium atom and the like are exemplified. A carbon atom, hydrogen atom, sulfur atom, nitrogen atom, oxygen atom, fluorine atom, chlorine atom, bromine atom and iodine atom are preferred particularly.

A compound capable of coordinating to both of two divalent metal ions is preferably a compound whose molecular weight is 80 or more and 1000 or less, particularly 80 or more and 700 or less in view of the solubility and the absorption performance of the compound when the compound is employed as an inhibitor of an enzyme having two divalent metal ions as an active center, although the molecular weight is not necessarily limited.

A compound having each substructure is detailed below.

For example, a compound having a substructure represented by Formula (I):

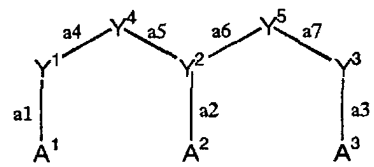

(I)

wherein each symbol is defined as described above may for be exemplified by a compound having a skeleton shown below.

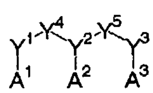
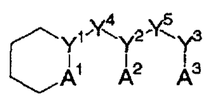
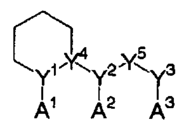
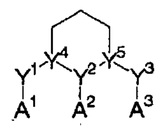

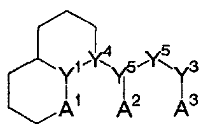
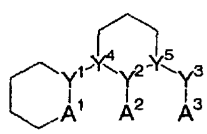
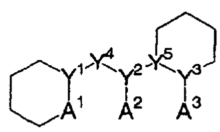
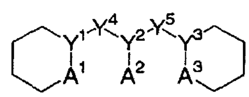

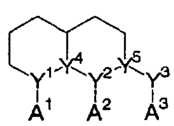
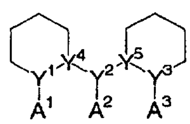
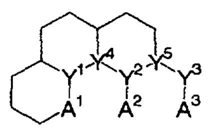
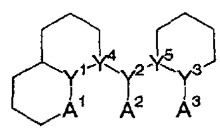

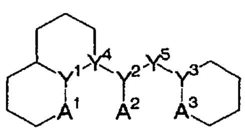
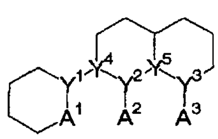
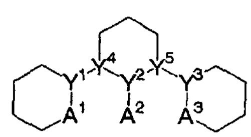
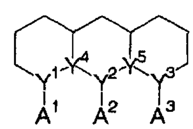

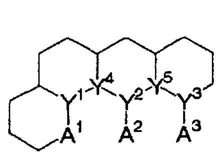
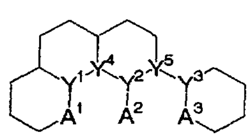
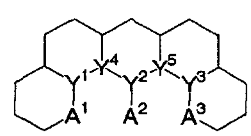

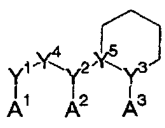
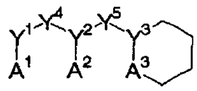
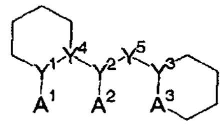
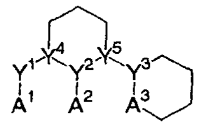
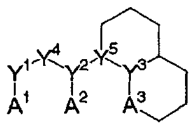
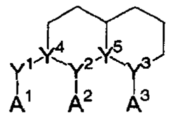
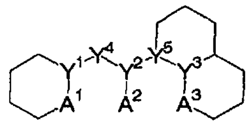
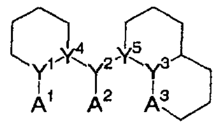
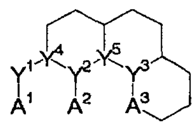
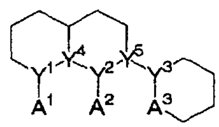
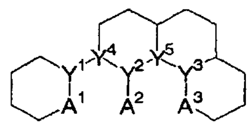
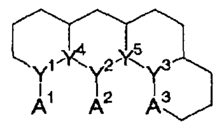

A compound having a substructure represented by Formula (II):

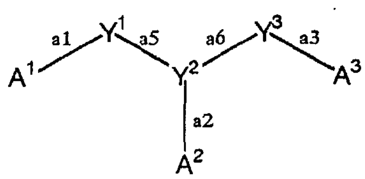

(II)

wherein each symbol is defined as described above may be exemplified by a compound having a skeleton shown below.

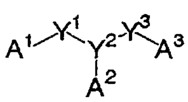
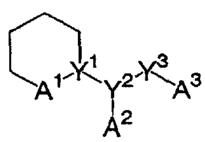
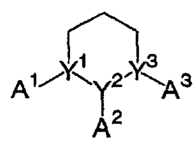
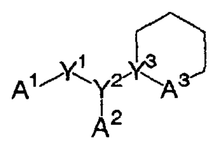
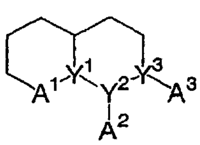
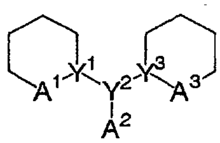
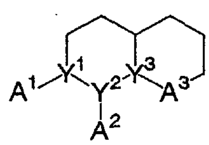
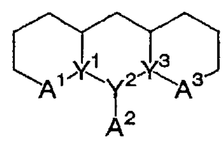

A compound having a substructure represented by Formula (III):

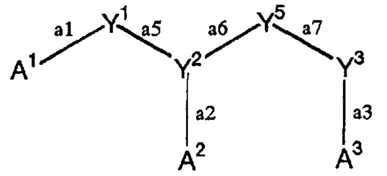

(III)

wherein each symbol is defined as described above may be exemplified by a compound having a skeleton shown below.

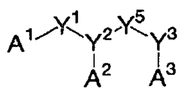

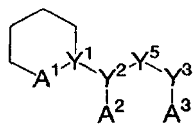

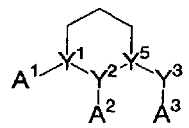

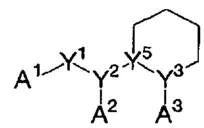

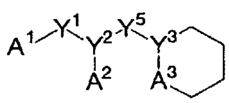

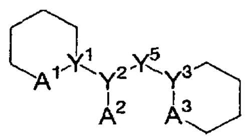

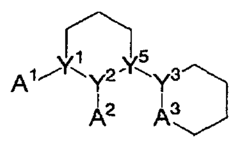

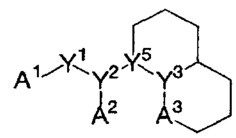

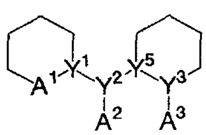

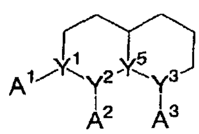

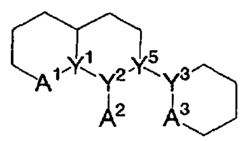

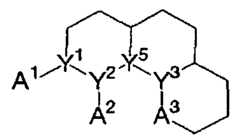

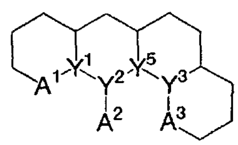

A compound having a substructure represented by Formula (IV):

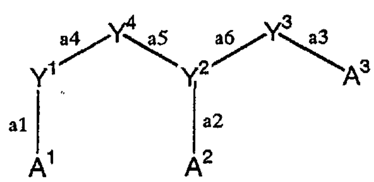

(IV)

wherein each symbol is defined as described above may be exemplified by a compound having a skeleton shown below.

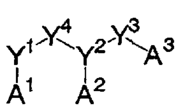

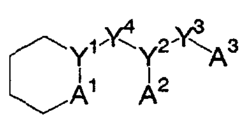

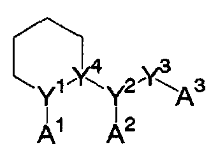

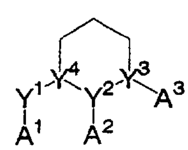

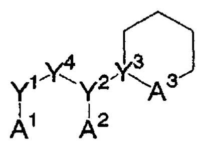 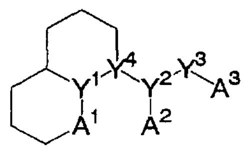 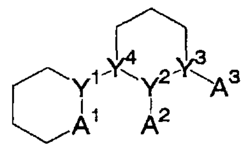 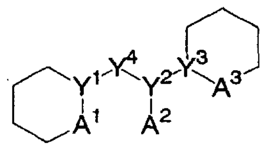

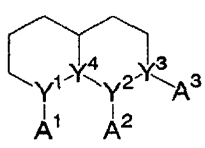 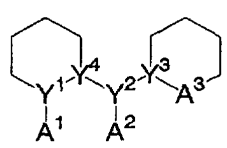 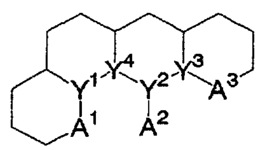 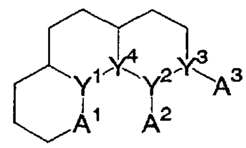

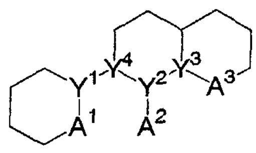

Although in the examples shown above, the ring system is indicated as a 6-membered ring for the purpose of convenience, any number of the ring members may be contemplated. One preferred especially is a 5-membered ring or 6-membered ring, particularly a 5-or 6-membered carbocyclic or heterocyclic ring. While the bond is represented as a single bond also for the purpose of convenience, it can be any possible bond (single bond or double bond) in organic chemistry. Each ring may be fused with several rings (carbocyclic rings or heterocyclic rings) having any ring members.

When bond a1 or bond a3 in a substructure shown above is a constituent of a part of a ring, thus, when atom $A^1$ or atom $A^3$ is contained in a ring, the following 5-membered or 6-membered rings may be exemplified. Any of these rings may have substituents.

For example, the rings shown below:

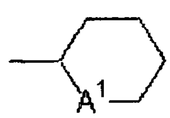 or 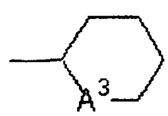

is exemplified by a 6-membered ring listed below:

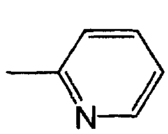 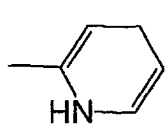 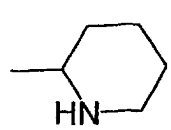 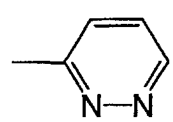 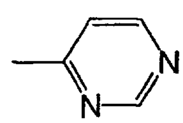

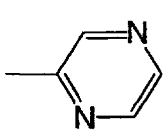 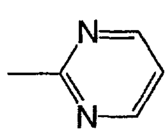 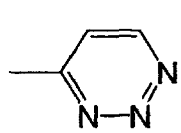 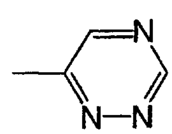 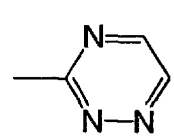

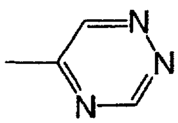 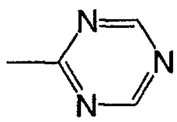 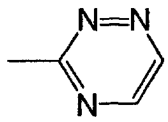 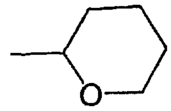 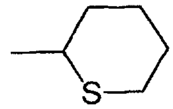

and a 5-membered ring listed below:

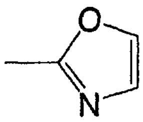 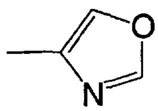 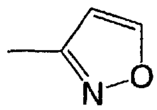 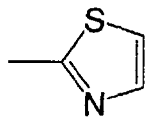 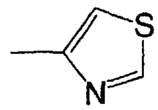

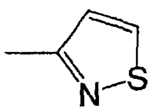 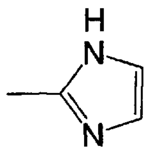 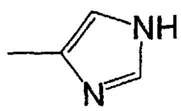 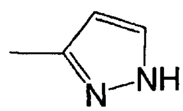 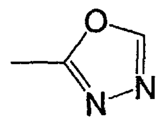

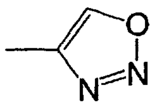 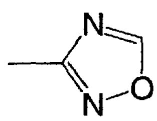 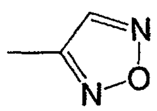 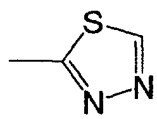 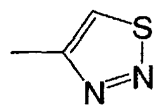

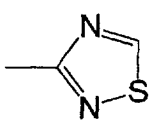 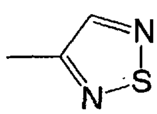 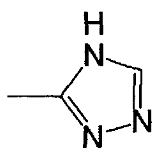 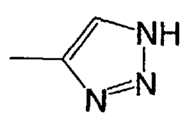 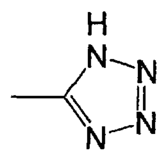

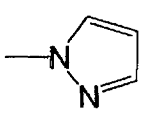 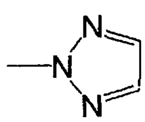 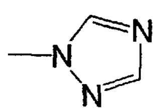 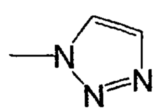 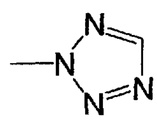

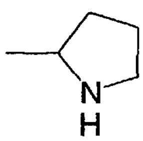 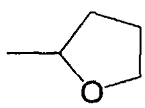 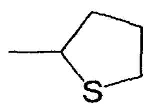

A compound having such a substructure can be found by searching the 3-D structure database of the compound using a computer or by a drug design using a computer. Further, it can be found without using a computer. Thus, a substructure data described above is employed to allow a researcher to design a compound fulfilling the respective prerequisite by himself and to synthesize the designed compound on the basis of an ordinary knowledge of organic chemistry. Thus, using a substructure shown above as a clue, a compound can be designed and synthesized. In such a procedure, the binding mode of the designed compound with an enzyme can be displayed as a computer graphics, and appropriated substituents can also be selected.

A compound capable of coordinating to both of two divalent metal ions thus selected, designed or synthesized can be examined for its activity of inhibiting an enzyme by means of a test described below.

For example, an enzyme having two divalent metal ions as an active center is a polymerase, a buffer solution containing a compound described above, the polymerase and divalent metal ions is combined sequentially with a polynucleotide as a template, an oligonucleotide as a primer and a labelled nucleotide as a substrate, and allowed to initiate the enzyme reaction, and then subjected, after a certain times, to the measurement of the labelled nucleotide incorporated. Based on the results at each concentration of the compound, the inhibitory activity of the compound on the polymerase can be calculated. As an index, the drug concentration at which the polymerase activity is inhibited by 50 % ($IC_{50}$) is employed usually. For example, when there is a compound which shows A% inhibition at using $\alpha$ μg/ml of the compound and B% inhibition at using $\beta$ μg/ml, then the $IC_{50}$ value can be obtained in accordance with the following equation provided that $A \geq 50 > B$. $IC_{50}$ (unit; μg/ml) = $10^{\{(50-B)/(A-B) \times (\log\alpha - \log\beta) + \log\beta\}}$

While an $IC_{50}$ value involves some error due to the measurement condition, an inhibitor whose IC50 value is 100 μmol/l or less, particularly 10 μmol/l or less, especially 1 μmol/l or less is preferred.

When an enzyme having two divalent metal ions as an active center is an integrase, a buffer solution containing a compound described above, the integrase, a substrate oligonucleotide and divalent metal ions is combined with a labelled oligonucleotide as a target, and allowed to initiate the enzyme reaction, and then subjected, after a certain times, to the measurement of the target labelled oligonucleotide incorporated. In this procedure, it is preferred that the substrate oligonucleotide is immobilized onto the well of an immunoplate and subjected to an assay. In such a case, it is particularly preferred that the substrate oligonucleotide in a buffer solution containing divalent metal ions is immobilized onto the well, and then the integrase is added to allow a complex of the substrate oligonucleotide and the integrase to be formed once, and then the excessive integrase forming no complex is washed out, and then the compound and the labelled oligonucleotide as a target are added to perform an assay.

Even when an enzyme having two divalent metal ions as an active center is another enzyme such as a nuclease, an appropriate assay system can be constructed to measure the inhibitory activity of a compound on the enzyme.

When a compound which can coordinate to two divalent metal ions is a compound having an inhibitory activity on several enzymes each having two divalent metal ions as an active center, the compound is investigated with regard, for example, to the substituents thereon thereby improving the selectivity of the intended enzyme. For example, when an inhibitor of a viral polymerase is searched for, then a compound is investigated with regard, for example, to the substituents thereon and a compound having a low inhibitory activity on a human polymerase.

As a means for identifying that a compound is really a compound employed in the present invention, i.e., a compound capable of coordinating to both of two divalent metal ions, the determination of the structure by X-ray diffraction may first be exemplified. A method for determining the structure of a complex of an enzyme and a compound utilizing an X-ray diffraction technology is well known (see T.L.Blunde and L.N.Johnson, Protein Crystallography, Academic Press, (1976) and Methods in Enzymology, vol.114 and 115, Ed. by H.W.Wyckoff et al., Academic Press (1985)). For example, an enzyme is purified and prepared at a high concentration, placed in a solution containing an appropriate buffer solution (HEPES, TRIS, MES and the like) together with an appropriate precipitant (ammonium sulfate, PEG, MPD and the like) to allow a crystal containing ligands to be formed in the presence of a compound according to the present invention. Alternatively, only the enzyme is crystallized first, and then the crystal is immersed in a solution containing a compound according to the present invention at an appropriate concentration, whereby obtaining the crystal containing the ligands. In this procedure, a substrate molecule, such as a nucleic acid molecule, may be allowed to coexist to obtain a crystal containing the ligand further associated therewith. Subsequently, the crystal containing the ligand thus obtained is irradiated with X-ray (obtained from a rotating anode X-ray generator or synchrotron radiation) to obtain the diffraction pattern of the crystal. The diffraction intensity is measured using an imaging plate or an X-ray detector such as a CCD detector. The diffraction intensity thus obtained is then subjected, as an intensity data set, to the analysis of the 3-D structure using a diffraction intensity processing software such as DENZO (HKL Inc.) and the like. The analysis of the 3-D structure may employ a structure analysis software such as a program package X-PLOR (Written by A.T.Brunger, X-PLOR, Version 98.0; Yale University press: New Haven, CT, 1998; Supplied from Molecular Simulation, Inc.,). A recent data measurement using a synchrotron radiation and an imaging plate and the sophistication using the X-PLOR allows a 3-D structure whose R value is about 0.25 or less and whose resolution is about 2 to 3 angstrom (quality sufficient to identify a compound employed in the present invention) to be obtained.

The visualization of the complex of the enzyme and the compound can be accomplished using a molecular

modeling program-such as Sybyl (Tripos, Inc., (St. Lewis)), InsightII (Molecular Simulation INC., (San Diego)) or Quanta (Molecular Simulations, INC., (San Diego), and the mode of the interaction between the enzyme and the compound can be elucidated using tools included in these programs.

The second means for identifying that a compound is really a compound employed in the present invention, i.e., a compound capable of coordinating to both of two divalent metal ions, is a technology of Nuclear Magnetic Resonance Spectroscopy (NMR). A method for determining the structure of a complex of an enzyme and a compound utilizing an NMR technology is well known (see J.Cavanagh, Protein NMR Spectroscopy: Principles And Practice, Academic Press (1996)).

For example, an enzyme is purified and prepared at a high concentration, and formulated into an enzyme solution containing a compound employed in the present invention, which is then subjected to the measurement of a chemical shifts reflecting the chemical environment of an atom and also subjected to nuclear overhouser enhancement (NOE) reflecting the distance between atoms, whereby obtaining the 3-D structure data of the complex of the enzyme with the compound. In this procedure, it is also possible to determine the 3-D structure of the complex of the enzyme with the compound in a solution by utilizing an enzyme labelled with a stable isotope such as 13C and 15N and using a heteronuclear multidimensional NMR technique.

Also in a method referred to as an isotope filter, an isotopically enriched enzyme and a non-labelled compound are used to collect a compound-derived signal selectively, whereby determining the 3-D structure of a compound employed in the present invention which is bound to the enzyme. In this procedure, the compound employed in the present invention can be proven to verified to divalent metal ions by observing the change in the chemical shifts at the site of the coordination.

The visualization of the structure of the complex of the enzyme and the compound can be accomplished by using the molecular modeling program described above.

The third means for identifying that a compound is really a compound employed in the present invention, i.e., a compound capable of coordinating to both of two divalent metal ions, is a molecular modeling technology. A process for making a theoretical model of a complex of an enzyme and a compound is well known (see, G.L.Seibel and P.A.Kollman, Molecular Mechanics and the Modeling of Drug Structures, Ch.18.2 in Comprehensive medicinal Chemistry, Ed. by C.Hansch, Pergammon Press, (1990), and Ed. by T.J.Perun and C.L.Propst, Computer-Aided Drug Design, Marcel Dekker, Inc (1989)). For the purpose of constructing the structure of a complex of a compound employed in the present invention and an enzyme, the molecular modeling program described above is employed. As a data of the structure of the enzyme, an existing X-ray crystal structure or a structure determined by NMR obtained from a protein structure database (Protein Data Bank) may be employed, or a homology model derived therefrom may also be employed. A candidate compound is docked into the position fulfilling the "two metal chelating model" described above, and then examined for the stability of the complex using a molecular mechanics such as energy minimization and molecular dynamics calculation. Typically, for the purpose of evaluating the inter-molecular interaction between an enzyme (including metal ions) and a compound, both of van der Waals interaction and electrostatic interaction are employed. For a molecular force field, AMBER (S.J.Weiner et al., Journal of Computational Chemistry, Vol.7, p230 to 252 (1986)) or CVFF (J.R.Maple et al., Journal of Computational Chemistry, Vol.15, p162-182)) can be utilized.

When conducting such an analysis, water molecules are provided around a complex whereby simulating the environment in an aqueous solution more accurately. The accuracy of each complex model is often comparable with the accuracy of the structure determined by X-ray crystal analysis or NMR analysis, when the 3-D structure of the enzyme having analogous amino acid sequence is known.

The visualization of the structure of the complex of an enzyme and a compound can be accomplished by using the molecular modeling program described above.

Otherwise, a compound can be identified as a compound employed in the present invention, i.e., a compound capable of coordinating to both of two divalent metal ions, also by an experimental method for examining the affinity between the compound and divalent metal ions.

First, a crystal containing the compound and the divalent metal ions is prepared, and analyzed for the structure of the chelate molecule, whereby accomplishing the identification. In this procedure, a co-crystal may be obtained from a solution containing the compound and the divalent metal ions, or a monocrystal of the compound is immersed in a solution of the divalent metal ions to obtain a crystal containing ligand.

The identification can be accomplished also by observing the change in various spectra appearing upon a stepwise addition of a divalent metal ion solution to a solution of the respective compound, for example, the change in the wavelength of the maximum absorption in the UV spectrum.

Alternatively, a solution containing the compound and the divalent metal ions is prepared and the molecular ions of the complex are detected by electrospray ionization mass spectroscopy (ESI) or atmospheric pressure chemical ionization mass spectroscopy (APCI), whereby accomplishing the identification.

Also for the purpose of identifying a compound capable of coordinating to two divalent metal ions, the compound is brought into contact with the divalent metal ions and then verified to form a complex whose two divalent metal

ions are chelated. The complex whose two divalent metal ions are chelated mentioned here means a complex in which one molecule of the compound is chelating the two divalent metal ions. A 1:1 complex, 2:1 complex, 1:2 complex and 4:4 complex are exemplified as such a complex.

A complex described above may be formed also by bringing a base and a divalent metal salt, preferably 2 equivalents or more of a base and 1 equivalent or more of a divalent metal salt, into contact with each other.

The formation of a complex described above can be identified by means of elemental analysis, mass spectroscopy, X-ray crystal analysis and/or change in UV spectral curve.

For example, the identification on the basis of the change in the UV spectral curve may be accomplished by changing the concentration of the divalent metal ions gradually while observing the accompanying change in the UV spectral curve. In the case of a compound chelating two divalent metals, the change in the UV spectral curve as a result of the formation of the first chelate is observed first, and the subsequent increase in the concentration of the divalent metal ions then causes the change in the UV spectral curve as a result of the formation of the second chelate.

It is also possible to accomplish the identification by forming a complex in accordance with the method described in Polyhedron 16, 1279, 1997 followed by elemental analysis, mass spectroscopy and/or X-ray crystal analysis.

A compound employed in the present invention, capable of coordinating to both of two divalent metal ions may be used after preparing a prodrug thereof. Thus, A prodrug is a derivative of the compound of the present invention having a group which can be decomposed chemically or metabolically, and such prodrug is converted to a pharmaceutically active compound of the present invention by means of solvolysis or by placing the compound in vivo under a physiological condition. Therefore, a prodrug itself may not possess an anti-integrase activity, so long as it can be converted to the active compound of the present invention. A method for the selection and process of an appropriate prodrug derivative are described in the literature such as Design of Prodrugs, Elsevier, Amsterdam 1985.

When a compound employed in the present invention, capable of coordinating to both of two divalent metal ions has a carboxyl group, an ester derivative prepared by reacting a basal acid compound with a suitable alcohol or an amide derivative prepared by reacting a basal acid compound with a suitable amine is exemplified as a prodrug. A particularly preferred ester derivative as an prodrug is methyl ester, ethyl ester, n-propyl ester, isopropyl ester, n-butyl ester, isobutyl ester, tert-butyl ester, morpholinoethyl ester, N,N-diethylglycolamido ester or the like. A particularly preferred amide derivative as a prodrug is amide, N-methyl amide, N-ethyl amide, N-benzyl amide or the like.

When a compound employed in the present invention, capable of coordinating to both of two divalent metal ions has a hydroxyl group, an acyloxy derivative prepared by reacting with a suitable acyl halide (e.g., acid chloride, halogenated acid) or a suitable acid anhydride (e.g., mixed acid anhydride) is exemplified as a prodrug. A particularly preferred acyloxy derivative as a prodrug is $-OCOC_2H_5$, -OCO(tert-Bu), $-OCOC_{15}H_{31}$, -OCO(m-COONa-Ph), $-OCOCH_2CH_2COONa$, $-OCOCH(NH_2)CH_3$, and $-OCOCH_2N(CH_3)_2$ or the like.

When a compound employed in the present invention, capable of coordinating to both of two divalent metal ions has a amino group, an amide derivative prepared by reacting with a suitable acid halide or a suitable acid anhydride is exemplified as a prodrug. A particularly preferred amide derivative as a prodrug is $-NHCO(CH_2)_{20}CH_3$, $-NHCOCH(NH_2)CH_3$ or the like.

A pharmaceutically acceptable salt of a compound capable of coordinating to both of two divalent metal ions, when exemplified as a basic salt, includes alkaline metal salts such as sodium salts and potassium salts; alkaline earth metal salts such as calcium salts and magnesium salts; ammonium salts; aliphatic amine salts such as trimethylamine salts, triethylamine salts, dicyclohexylamine salts, ethanolamine salts, diethanolamine salts, triethanolamine salts and procaine salts; aryl lower alkylamine salts such as N,N-dibenzyl ethylenediamine; heteroaromatic amine salts such as pyridine salts, picoline salts, quinoline salts and isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts and tetrabutylammonium salts; basic amino acid salts such as arginine salts and lysine salts. Acid addition salts may for example be inorganic acid addition salts such as hydrochlorides, sulfates, nitrates, phosphates, carbonates, bicarbonates and perchlorates; organic acid addition salts such as acetates, propionates, lactates, maleates, fumarates, tartarates, malates, citrates and ascorbates; sulfonates such as methanesulfonates, isethionates, benzenesulfonates and p-toluenesulfonates; acidic amino acid addition salts such as aspartates and glutamates.

Hydrates and various solvates of compounds each capable of coordinating to both of two divalent metal ions employed in the present invention are encompassed in the present invention, including monohydrates and dihydrates. Those containing residual water may also be contemplated.

The term "inhibition" means a suppression of the activity of an enzyme by a compound which can coordinate to both of two divalent metal ions employed in the present invention. The term "pharmaceutically acceptable" means that there is no hazard prophylactically or therapeutically.

The term "inhibitor of an enzyme having two divalent metal ions as an active center" means "pharmaceutical composition having an inhibitory activity on an enzyme having two divalent metal ions as an active center".

The compounds of the present invention can be administered orally or parenterally. For oral administration,

the compounds of the present invention can be used in any form of usual formulations, for example, solid formulations such as tablets, powders, granules, capsules; aqueous formulations; oleaginous suspensions; solutions such as syrup or elixir. For parenteral administration, the compounds of the present invention can be used as an aqueous or oleaginous suspension injection, or nose drops. In the preparation of such formulations, conventional excipients, binding agents, lubricants, aqueous solvents, oleaginous solvents, emulsifying agents, suspending agents, preservatives, stabilizers, and the like can be optionally used.

A formulation according to the present invention may be manufactured by combining (for example, admixing) a curatively effective amount of a compound of the present invention with a pharmaceutically acceptable carrier or diluent. The formulation of the present invention may be manufactured with the use of well-known and easily available ingredients in accordance with a known method.

In the case of manufacturing a pharmaceutical composition according to the present invention, an active ingredient is admixed or diluted with a carrier, or they are contained in a carrier in the form of capsule, sacheier, paper, or another container. In the case of functioning a carrier as a diluent, the carrier is a solid, semi-solid, or liquid material which functions as a medium. Accordingly, a formulation according to the present invention may be produced in the form of tablet, pill, powder medicine, intraoral medicine, elixir agent, suspending agent, emulsifier, dissolving agent, syrup agent, aerosol agent (solid in liquid medium), and ointment. Such a formulation may contain up to 10% of an active compound. It is preferred to formulate a compound of the present invention prior to administration.

Any suitable carrier which has been well known by those skilled in the art may be used for the formulation. In such formulation, a carrier is in the form of solid, liquid, or a mixture of solid and liquid. For instance, a compound of the present invention is dissolved into 4% dextrose / 0.5% sodium citrate aqueous solution so as to be 2 mg/ml concentration for intravenous injection. Solid formulation includes powder, tablet, and capsule. Solid carrier consists of one or more of material(s) for serving also as fragrant, lubricant, dissolving agent, suspension, binder, tablet disintegrator, capsule. A tablet for oral administration contains a suitable excipient such as calcium carbonate, sodium carbonate, lactose, calcium phosphate and the like together with a disintegrator such as corn starch, alginic acid and the like and/or a binder such as gelatin, acacia and the like, and a lubricant such as magnesium stearate, stearic acid, talc and the like.

In a powder medicine, a carrier is a finely pulverized solid which is blended with finely pulverized active ingredients. In a tablet, active ingredients are admixed with a carrier having required binding power in a suitable ratio, and it is solidified in a desired shape and size. Powder medicine and tablet contain about 1 to about 99% by weight of the active ingredients being novel compounds according to the present invention. Example of suitable solid carriers include magnesium carbonate, magnesium stearate, talc, sugar, lactose, pectin, dextrin, starch, gelatin, tragacanth gum, methyl cellulose, sodium carboxymethylcellulose, low-melting wax, and cocoa butter.

An axenic liquid formulation contains suspending agent, emulsifier, syrup agent, and elixir agent. Active ingredients may be dissolved or suspended into a pharmaceutically acceptable carrier such as sterile water, a sterile organic solvent, a mixture thereof and the like. Active ingredients may be dissolved frequently into a suitable organic solvent such as propylene glycol aqueous solution. When finely pulverized active ingredients are dispersed into aqueous starch, sodium carboxylmethylcellulose solution, or suitable oil, the other compositions can be prepared.

Although an appropriate dosage of the compound of the present invention varies depending on the administration route, age, body weight, conditions of the patient, and kind of disease, in the case of oral administration, the daily dosage can be between approximately 0.05-3000 mg, preferably approximately 0.1-1000 mg, for an adult. The daily dosage can be administered in divisions. In the case of parenteral administration, the daily dosage for an adult can be between approximately 0.01-1000 mg, preferably approximately 0.05-500 mg.

## EXAMPLES

As an enzyme having two divalent metal ions as an active center, an HIV integrase is exemplified below for describing the present invention in more detail. The HIV integrase is an enzyme which catalyzes nucleic acid related reactions which acts upon introduction of a DNA which has been reverse-transcribed from an RNA genome of a human immunodeficiency virus into a host cell genome.

First, the 3-D structure in geometrical coordinates of the HIV integrase disclosed in a reference Proc. Natl. Acad. Sci. USA 96, 13040-13043 was obtained. When the structure in geometrical coordinates of an intended enzyme has already been disclosed in Protein Data Bank, it can be obtained via internet. When the 3-D structure of an intended enzyme can not be obtained and the structure of the active center is not characterized, then the 3-D structure data of an enzyme having a high amino acid sequence homology with the intended enzyme is obtained, and the structure of the unknown part is constructed for example by a homology modeling technology.

Subsequently, since this crystal structure had only one identified metal ion (only $M^1$), another metal ion ($M^2$) was aligned based on the 3-D structure (1VSH) of the integrase of ASV classified as a retrovirus similarly to HIV. The 3-D structure around the active center of the ASV integrase has an extremely high homology with the HIV integrase,

especially with regard to the position of the catalytic triad described above and the metal ion $M^1$, which are in almost complete agreement with each other. Since the side chain of the 152nd glutamic acid was not directed correctly due to the defect of $M^2$ in this HIV integrase structure, the correction to the original metal-coordinating position was made here. The followings are the active centers of the ASV integrase and the HIV integrase.

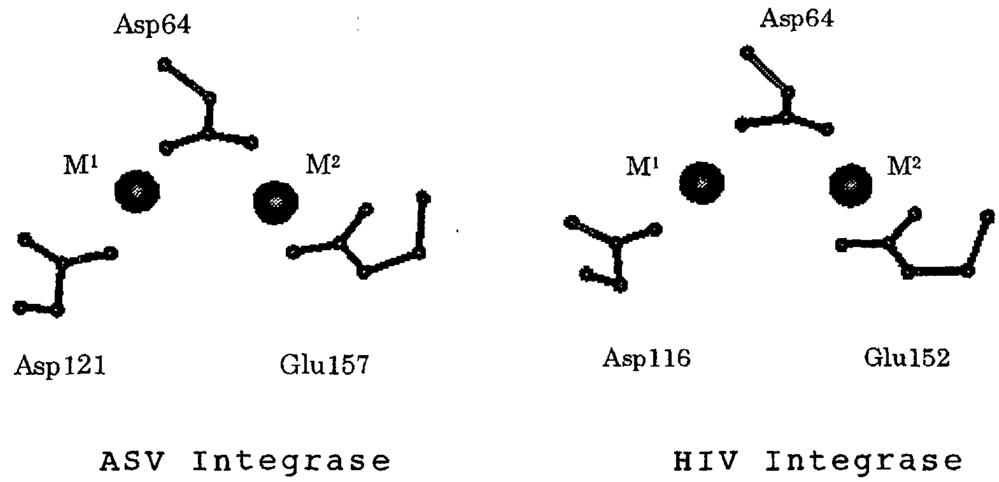


It is appropriate to align these two divalent metal ions as described above in view of the functions of the HIV integrase. Thus, in the active center of the HIV integrase, the acidic amino acid residue Asp116, Asp64 and Glu152 are present together with 2 divalent metal ions chelated thereby, and a nucleic acid molecule as a substrate interacts with the metal ions as shown below to exert the catalytic function.

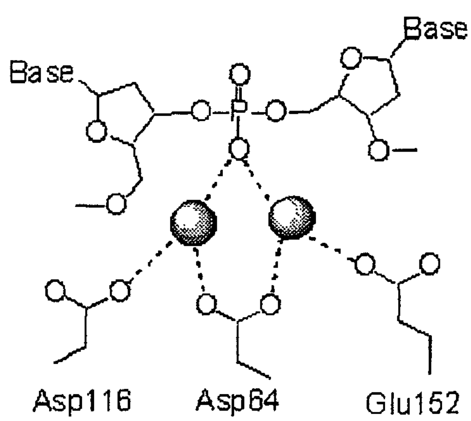


is a divalent metal ion.

For designing a compound capable of coordinating to both of two divalent metal ions possessed by the HIV integrase, the scaffold may be selected from the compounds having the substructures described above such as compounds having substructures represented by Formula (I) to Formula (IV). Since the active center of the HIV integrase is present on the surface of the enzyme rather than in a deep pocket, the structural restriction due to the steric repulsion observed in a small active site like a deep pocket is minimal, whereby allowing a diversity of the scaffolds to be selected.

On the other hand, a substituent should be discussed in view of both of the van der Waals interaction and the static interaction with the enzyme. When searching for a compound having an inhibitory activity on an enzyme having two divalent metal ions while intending a compound having a high inhibitory activity and a high enzyme specificity, the substituent is handled separately on enzyme-by-enzyme basis. The introduction of an appropriate substituent serves

to further increase the binding energy between the compound and the enzyme. Thus, the compound is bound to the enzyme by chelating two divalent metal ions in the enzyme, and a further interaction between the appropriate substituent and the enzyme results in a further increase in the binding energy between the compound and the enzyme. Moreover, the introduction of a appropriate substituent serves not only to increase the energy of binding to a target enzyme but also to reduce the energy of binding to a non-target enzyme, whereby exerting the selectivity of the inhibitory activity on the target enzyme.

When investigating an appropriate substituent, an enzyme and a compound described above are displayed in the form of the two-metal chelation on a computer graphics, and the gap between the enzyme and the compound is investigated to select an appropriate substituent. The substituent can be selected on the basis of the van der Waals interaction and the electrostatic interaction between the enzyme and the compound. Such an investigation can be conducted efficiently by utilizing the structure-activity relationship (SAR) of the compounds which have already been synthesized and examined for their activities.

In the HIV integrase, the region comprising Thr66, His67, Leu68, Asn155, Lys156, Lys159 and Ile162 has a shallow pocket structure (a large sphere shown below each denotes an amino acid).

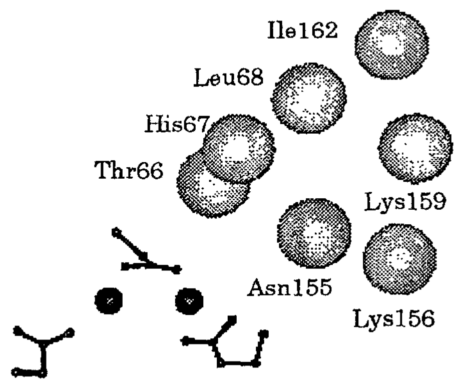


Accordingly, for the purpose of exerting the integrase inhibiting activity, a compound should be a compound capable of coordinating to both of two divalent metal ions, and also for the purpose of exerting a further potent HIV integrase inhibiting activity, a compound having a substituent which interacts with the site comprising Thr66, His67, Leu68, Asn55, Lys156, Lys159 and Ile162 of the HIV integrase is preferred.

Thus, as an HIV integrase inhibitor, it is preferable to use a compound capable of coordinating to both of two divalent metal ions and also having moiety T in the position shown below.

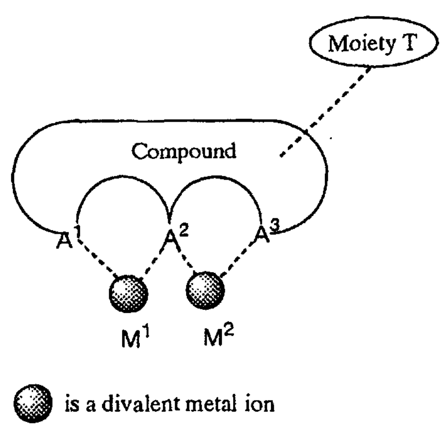


First, the position of moiety T is specified using the geometrical coordinates of atom $A^1$ to atom $A^3$.

1) The distance between the center of moiety T and the position of atom $A^2$ (distance T-$A^2$) is about 6.0 to 11.0

angstrom, preferably about 7.0 to 10.0 angstrom, more preferably about 7.5 to 9.5 angstrom;
2) the angle defined by the center of moiety T, the position of atom $A^2$ and the position of atom $A^3$ (angle T-$A^2$-$A^3$) is about 5.0 to 30.0°, preferably about 10.0 to 30.0°, more preferably about 12.0 to 230.0°; and,
3) the torsional angle defined by the center of moiety T, position of atom $A^2$, position of atom $A^3$ and position of atom $A^1$ (torsional angle T-$A^2$-$A^3$-$A^1$) is about 30.0 to 100.0°, preferably about 30.0 to 90.0°, more preferably about 40.0 to 90.0°.

The position of moiety T may be specified also as follows.

1) The distance between the center of moiety T and the position of atom $A^3$ (distance T-$A^3$) is about 3.0 to 8.0 angstrom, preferably about 4.5 to 6.5 angstrom;
2) the angle defined by the center of moiety T, the position of atom $A^3$ and the position of atom $A^2$ (angle T-$A^3$-$A^2$) is about 130.0 to 165.0°, preferably about 140.0 to 160.0°; and,
3) the torsional angle defined by the center of moiety T, position of atom $A^3$, position of atom $A^2$ and position of atom $A^1$ (torsional angle T-$A^3$-$A^2$-$A^1$) is about 240.0 to 320.0°, preferably about 250.0 to 315.0.

The position of moiety T is specified as follows using the geometrical coordinates of the divalent metal atoms. The divalent metal ion to which atom $A^1$ and atom $A^2$ coordinate is designated as $M^1$, while the divalent metal ion to which atom $A^2$ and atom $A^3$ coordinate is designated as $M^2$.

1) The distance between the center of moiety T and the position of divalent metal ion $M^2$ (distance T-$M^2$) is about 3.5 to 8.5 angstrom, preferably about 4.5 to 7.0 angstrom;
2) the angle defined by the center of moiety T, the position of divalent metal ion $M^2$ and the position of atom $A^2$ (angle T-$M^2$-$A^2$) is about 110.0 to 140.0°, preferably about 115.0 to 135.0°; and,
3) the torsional angle defined by the center of moiety T, center of divalent metal ion $M^2$, position of atom $A^2$ and center of divalent metal ion $M^1$ (torsional angle T-$M^1$-$A^2$-$M^1$) is about 130.0 to 165.0°, preferably about 135.0 to 155.0°.

The distance T-$A^2$ mentioned here means the distance between the center of moiety T and the position of atom $A^2$.
The angle T-$A^2$-$A^3$ means the angle defined by the line passing through the center of moiety T and the position of atom $A^2$ and the line passing through the position of atom $A^2$ and the position of atom $A^3$.
The torsional angle T-$A^2$-$A^3$-$A^1$ is the angle defined by the plane passing through the center of moiety T, the position of atom $A^2$ and the position of atom $A^3$ and the plane passing through the position of atom $A^2$, the position of atom $A^3$ and the position of atom $A^1$.
Other distances, angles and torsional angles may be defined similarly.
The geometrical coordinates of moiety T, atoms A1 to A3, divalent metal ions ($M^1$, $M^2$) are those of the respective centers. The geometrical coordinates of the center of moiety T are the arithmetic means of the constituent heavy atom's geometrical coordinates (geometrical coordinates of the atoms as constituents of moiety T except for hydrogen atoms). For example, in the cases of an isopropyl group and a cyclopropyl group, the center is the center of the triangle formed by the three carbon atoms as constituents of the isopropyl group and the cyclopropyl group. In the case of a tert-butyl group, the center is the center of the triangular pyramid formed by the four carbon atoms as constituents of the tert-butyl group. In the cases of a phenyl group and a cyclohexyl group, the center is the center of the hexagon formed by the six carbon atoms as constituents of the phenyl group and the cyclohexyl group. In the cases of a pyridyl group, the center is the center of the hexagon formed by the five carbon atoms and one nitrogen atom as constituents of the pyridyl group. When moiety T is a fused cyclic group (for example, naphthyl group, benzofuryl group, benzothienyl group and the like), then the geometric coordinates of the center of the ring closer to atom $A^2$ is calculated as the center of the fused cyclic group. When moiety T has a ring system, then a substituent on the ring is not taken into account when calculating the center.
Moiety T and a substructure described above may be bound covalently via any organic residue. An atom as a constituent of such an organic residue may for example be a carbon atom, hydrogen atom, sulfur atom, nitrogen atom, phosphorus atom, oxygen atom, boron atom, fluorine atom, chlorine atom, bromine atom, iodine atom, sodium atom, lithium atom, magnesium atom, aluminum atom, potassium atom, calcium atom, barium atom and the like. Especially, a carbon atom, hydrogen atom, sulfur atom, nitrogen atom, oxygen atom, fluorine atom, chlorine atom, bromine atom and iodine atom are preferred. The organic residues may be chains or may have ring systems.
While the distance, angle and torsional angle mentioned above are defined on the basis of the geometrical coordinates of moiety T, atom $A^1$ to $A^3$, divalent metal ions $M^1$ and $M^2$, they can be defined on the basis of the site which interacts with the enzyme.

Thus, the case in which moiety T is a moiety which interacts with at least one amino acid selected from the group consisting of Thr66, His67, Leu68, Asn155, Lys156, Lys159 and Ile162 of an HIV integrase is mentioned. More preferably, a moiety which interacts with at least two, particularly three amino acid residues described above is mentioned.

The interaction referred to here means a van der Waals interaction and/or an electrostatic interaction with amino acids of an enzyme. For example, when the inter-atomic distance between said amino acid residue and the compound is approximately the sum of the van der Waals radii of the both, then the van der Waals interaction can be contemplated. When said amino acid residue and the compound form a hydrogen bond or an ion pair, then the static interaction can be contemplated.

As moiety T, a group whose volume is 50 cubic angstrom or more can be exemplified. Such a substituent interacts with the site comprising Thr-66, His-67, Leu-68, Asn-155, Lys-156, Lys-159 and Ile-162 of an HIV integrase.

For example, a group represented by Formula:

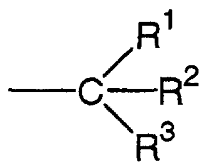

wherein 1) $R^1$, $R^2$ and $R^3$ are hydrogen, optionally substituted alkyl, optionally substituted alkenyl or optionally substituted alkynyl, and two of $R^1$, $R^2$ and $R^3$ are optionally substituted alkyl or optionally substituted alkynyl, 2) $R^1$ is hydrogen, optionally substituted alkyl, optionally substituted alkenyl or optionally substituted alkynyl, and $R^2$ and $R^3$ are taken together to form an optionally substituted 3- to 8-membered ring comprising of hydrogen atoms, carbon atoms, nitrogen atoms, sulfur atoms and oxygen atoms, or 3) $R^1$ and $R^2$ are taken together with $R^3$ to form an optionally substituted 3- to 8-membered ring comprising of hydrogen atoms, carbon atoms, nitrogen atoms, sulfur atoms and oxygen atoms, or a group represented by Formula:

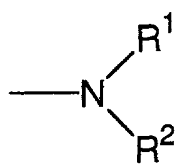

wherein 1) $R^1$ and $R^2$ are optionally substituted alkyl, optionally substituted alkenyl or optionally substituted alkynyl, or 2) $R^2$ and $R^3$ are taken together to form an optionally substituted 3- to 8-membered ring comprising of hydrogen atoms, carbon atoms, nitrogen atoms, sulfur atoms and oxygen atoms may be exemplified.

It is preferred especially that moiety T is an optionally substituted carbocyclic group, optionally substituted heterocyclic group, optionally substituted branched alkyl or optionally substituted branched alkenyl.

The term "alkyl" means a C1-C6 straight or branched alkyl group, for example, methyl, ethyl, n-propyl, isopropyl, n-butyl, isobutyl, sec-butyl, tert-butyl, n-pentyl, isopentyl, neopentyl, tert-pentyl, n-hexyl, isohexyl or the like. Preferred is a C1-4 straight or branched alkyl group such as methyl, ethyl, n-propyl, isopropyl, n-butyl, isobutyl, sec-butyl, tert-butyl or the like.

The term "alkenyl" means a C2-C6 straight or branched alkenyl group which is the above "alkyl" having one or more double bond, for example, vinyl, 1-propenyl, 2-propenyl, 1-butenyl, 2-butenyl, 3-butenyl, 1,3-butadienyl or the like. Preferred is a C2-3 straight alkenyl group such as vinyl, 1-propenyl or 2-propenyl.

The term "alkynyl" means a straight or branched alkynyl having 2 to 8 carbon atoms and at least one triple bond. For example, ethynyl, 1-propynyl, 2-propynyl, 1-butynyl, 2-butynyl and 3-butynyl can be exemplified.

The term "3- to 8-membered cyclic group comprising of hydrogen atoms, carbon atoms, nitrogen atoms, sulfur atoms and oxygen atoms" means a 3- to 8-membered carbocyclic group comprising of hydrogen atoms and carbon atoms as well as a 3- to 8-membered heterocyclic group comprising of hydrogen atoms, carbon atoms, nitrogen atoms, sulfur atoms and oxygen atoms. For example, phenyl, cyclopropyl, cyclobutyl, cyclopentyl, cyclohexyl, cycloheptyl, cyclooctyl, furyl, thienyl, pyrrolyl, imidazolyl, pyrazolyl, triazolyl, tetrazolyl, oxazolyl, isoxazolyl, thiazolyl, isothiazolyl, pyridyl, pyridazinyl, pyrimidinyl, furazanyl, pyrazinyl, thiadiazolyl, oxadiazolyl, aziridinyl, piperidino, piperidyl, morpholino, morpholinyl, pyrrolinyl, pyrrolidinyl, imidazolinyl, piperazino, piperazinyl, thiolanyl, tetrahydrofuranyl, dioxanyl,

oxathianyl and tetrahydropyranyl can be exemplified.

The term "carbocyclic group" means "aromatic carbocyclic group" and "non-aromatic carbocyclic group".

The term "aromatic carbocyclic group" means an aromatic carbocyclic group having 6 or more ring members such as aryl, naphthyl (for example, 1-naphthyl, 2-naphthyl) and anthryl.

The term "non-aromatic carbocyclic group" means "cycloalkyl" and "cycloalkenyl".

The term "cycloalkyl" means a C3-C8 cyclic alkyl group, for example, cyclopropyl, cyclobutyl, cyclopentyl, cyclohexyl, cycloheptyl, cyclooctyl or the like. Preferred is a C3-6 cyclic alkyl group such as cyclopropyl, cyclobutyl, cyclopentyl or cyclohexyl.

The term "cycloalkenyl" means a C3-C8 cyclic alkenyl group which is the above "cycloalkyl" having one or more double bond, for example, 1-cyclopropen-1-yl, 2-cyclopropen-1-yl, 1-cyclobuten-1-yl, 2-cyclobuten-1-yl, 1-cyclopenten-1-yl, 2-cyclopenten-1-yl, 3-cyclopenten-1-yl, 1-cyclohexen-1-yl, 2-cyclohexen-1-yl, 3-cyclohexen-1-yl, 1-cyclohepten-1-yl, 2-cyclohepten-1-yl, 3-cyclohepten-1-yl, 4-cyclohepten-1-yl or the like. Preferred is a C3-C6 cyclic alkenyl group, for example, 1-cyclopropen-1-yl, 2-cyclopropen-1-yl, 1-cyclobuten-1-yl, 2-cyclobuten-1-yl, 1-cyclopenten-1-yl, 2-cyclopenten-1-yl, 3-cyclopenten-1-yl, 1-cyclohexen-1-yl, 2-cyclohexen-1-yl or 3-cyclohexen-1-yl.

The term "heterocyclic group" means "aromatic heterocyclic group" and "non-aromatic heterocyclic group".

The term "aromatic heterocyclic group" means a 5- to 8-membered aromatic heterocyclic group having 1 to 4 oxygen, sulfur and/or nitrogen atoms in the rings, to which other 1 to 4 5- to 8-membered aromatic carbocyclic rings or other 5- to 8-membered aromatic heterocyclic rings may further be fused, and which has a bond in any substitutable position. The aromatic heterocyclic group may have the bond in any position on a carbon atom or nitrogen atom as a constituent of the ring, and may have the bond in any of the aromatic heterocyclic rings and aromatic carbocyclic rings. The nitrogen atom contained in the ring may be quaternized.

The term "aromatic heterocyclic group" includes furyl (for example, furan-2-yl, furan-3-yl), thienyl (for example, thiophen-2-yl, thiophen-3-yl), pyrrolyl (for example, pyrrol-1-yl, pyrrol-2-yl, pyrrol-3-yl), imidazolyl (for example, imidazol-1-yl, imidazol-2-yl, imidazol-4-yl), pyrazolyl (for example, pyrazol-1-yl, pyrazol-3-yl, pyrazol-4-yl), triazolyl (for example, 1H-[1,2,4]triazol-1-yl, 4H-[1,2,4]triazol-4-yl, 1H-[1,2,4]triazol-3-yl), tetrazolyl(for example, 1H-tetrazol-1-yl, 2H-tetrazol-2-yl, 1H-tetrazol-5-yl, 2H-tetrazol-5-yl), oxazolyl (for example, oxazol-2-yl, oxazol-4-yl, oxazol-5-yl), isoxazolyl (for example, isoxazol-3-yl, isoxazol-4-yl, isoxazol-5-yl), thiazolyl (for example, thiazol-2-yl, thiazol-4-yl, thiazol-5-yl), isothiazolyl (for example, isothiazol-3-yl, isothiazol-4-yl, isothiazol-5-yl), pyridyl (for example, pyridin-2-yl, pyridin-3-yl, pyridin-4-yl), pyridazinyl (for example, pyridazin-3-yl, pyridazin-4-yl), pyrimidinyl (for example, pyrimidin-2-yl, pyrimidin-4-yl, pyrimidin-5-yl), furazanyl (for example, furazan-3-yl), pyrazinyl (for example, pyrazin-2-yl), thiadiazolyl (for example, [1,3,4]thiaziazol-2-yl), oxadiazolyl (for example, [1,3,4]-oxadiazol-2-yl), benzofuryl (for example, benzo[b]furan-2-yl, benzo[b]furan-3-yl, benzo[b]furan-4-yl, benzo[b]furan-5-yl, benzo[b]furan-6-yl, benzo[b]furan-7-yl), benzothienyl (for example, benzo[b]thiophen-2-yl, benzo[b]thiophen-3-yl, benzo[b]thiophen-4-yl, benzo[b]thiophen-5-yl, benzo[b]thiophen-6-yl, benzo[b]thiophen-7-yl), benzimidazolyl (for example, benzimidazol-1-yl, benzimidazol-2-yl, benzimidazol-4-yl, benzimidazol-5-yl), benzothiazolyl (for example, benzothiazol-2-yl, benzothiazol-3-yl, benzothiazol-4-yl, benzothiazol-5-yl, benzothiazol-6-yl, benzothiazol-7-yl), indolyl (for example, indol-1-yl, indol-2-yl, indol-4-yl, indol-5-yl, indol-6-yl, indol-7-yl), dibenzofuryl, quinolyl (for example, quinolin-2-yl, quinolin-3-yl, quinolin-4-yl, quinolin-5-yl, quinolin-6-yl, quinolin-7-yl, quinolin-8-yl), isoquinolyl (for example, isoquinolin-1-yl, isoquinolin-3-yl, isoquinolin-4-yl, isoquinolin-5-yl, isoquinolin-6-yl, isoquinolin-7-yl, isoquinolin-8-yl), cinnolyl (for example, cinnolin-3-yl, cinnolin-4-yl, cinnolin-5-yl, cinnolin-6-yl, cinnolin-7-yl, cinnolin-8-yl), quinazolyl (for example, quinazolin-2-yl, quinazolin-4-yl, quinazolin-5-yl, quinazolin-6-yl, quinazolin-7-yl, quinazolin-8-yl), quinoxanyl (for example, quinoxalin-2-yl, quinoxalin-5-yl, quinoxalin-6-yl), phthalazinyl (for example, phthalazin-1-yl, phthalazin-5-yl, phthalazin-6-yl), puryl (for example, purin-2-yl, purin-6-yl, purin-7-yl, purin-8-yl, purin-9-yl), puteridinyl, carbazolyl, phenanthridinyl, acridinyl, phanazinyl, 1,10-phenanthronyl, isoindolyl, 1H-indazolyl or indolidinyl (for example, indolidin-1-yl) and the like.

The term "non-aromatic heterocyclic group" a non-aromatic heterocyclic group containing 1 to 3 oxygen, sulfur and/or nitrogen atoms in the rings of the above-mentioned "cycloalkyl" or "cycloalkenyl". For example, aziridinyl (for example, aziridin-1-yl, aziridin-2-yl and the like), piperidino, piperidyl (for example, 2-piperidyl, 3-piperidyl, 4-piperidyl and the like), morpholino, morpholinyl (for example, 2-morpholinyl, 3-morpholinyl and the like), pyrrolinyl (for example, 1-pyrrolinyl, 2-pyrrolinyl, 3-pyrrolinyl, 4-pyrrolinyl, 5-pyrrolinyl and the like), pyrrolidinyl (for example, 1-pyrrolidinyl, 2-pyrrolidinyl, 3-pyrrolidinyl and the like), imidazolinyl (for example, 1-imidazolinyl, 2-imidazolinyl, 4-imidazolinyl and the like), piperadino, piperazinyl (for example, 2-piperazinyl and the like), thiolanyl (for example, thiolan-2-yl, thiolan-3-yl and the like), tetrahydrofuranyl (for example, tetrahydrofuran-2-yl, tetrahydrofuran-3-yl), dioxanyl (for example, 1,4-dioxan-2-yl and the like), oxathianyl (for example, 1,4-oxathian-2-yl, 1,4-oxathian-3-yl and the like), tetrahydropyranyl (for example, tetrahydropyran-2-yl, tetrahydropyran-3-yl, tetrahydropyran-4-yl and the like). Preferably, a 5- or 6-membered nitrogen-containing heterocyclic group is exemplified, including piperidino, piperidyl (for example, 2-piperidyl, 3-piperidyl, 4-piperidyl and the like), morpholino, morpholinyl (for example, 2-morpholinyl, 3-morpholinyl and the like), piperidino, piperidyl (for example, 2-piperidyl, 3-piperidyl, 4-piperidyl and the like), morpholino, morpholinyl (for example, 2-morpholinyl, 3-morpholinyl and the like), pyrolinyl (for example, 1-pyrrolinyl, 2-pyrrolinyl, 3-pyrrolinyl, 4-pyrrolinyl, 5-pyrrolinyl and the like), pyrrolidinyl (for example, 1-pyrrolidinyl, 2-pyrrolidinyl, 3-pyrrolidinyl and the like), imidazolinyl (for example, 1-imidazolinyl, 2-imidazolinyl, 4-imidazolinyl and the like), piperazino, piperazinyl (for example, 2-piperazinyl and the like) are exemplified. It is also possible that any "non-aromatic heterocyclic group" can have a bond on any of carbon atoms and nitrogen atoms, similarly to the "aromatic heterocyclic group" described above. The nitrogen atom contained in the ring may be quaternized.

The term "branched alkyl" means a branched alkyl having 3 to 8 carbon atoms, for example, isopropyl, isobutyl, sec-butyl, tert-butyl, isopentyl, neopentyl, tert-pentyl, isohexyl and the like.

The term "branched alkenyl" means a branched alkenyl having 3 to 8 carbon atoms which has one or more double bonds in the "branched alkyl" described above, for example isopropenyl, 3-methyl-2-butenyl and the like.

As a substituent on "optionally substituted alkyl", "optionally substituted alkenyl", "optionally substituted alkynyl", "optionally substituted 3- to 8-membered ring comprising of hydrogen atoms, carbon atoms, nitrogen atoms, sulfur atoms and oxygen atoms", "optionally substituted carbocyclic group", "optionally substituted heterocyclic group", "optionally substituted branched alkyl" and "optionally substituted branched alkenyl" may for example be hydroxy, carboxy, halogen (F, Cl, Br, I), lower haloalkyl (for example, $CF_3$, $CH_2CF_3$ and the like), lower alkyl (for example, methyl, ethyl, isopropyl, tert-butyl and the like), lower alkenyl (for example, vinyl, allyl and the like), lower alkynyl (for example, ethynyl and the like), cycloalkyl (for example, cyclopropyl, cyclobutyl, cyclohexyl and the like), cycloalkynyl (for example, 1-cyclohexenyl and the like), lower alkyloxy (for example, methoxy, ethoxy, propoxy, butoxy and the like), lower alkyloxycarbonyl (for example, methoxycarbonyl, ethoxycarbonyl, tert-butoxycarbonyl and the like), nitro, nitroso, amino, lower alkyl-substituted amino (for example, methylamino, ethylamino, dimethylamino and the like), azide, amidino, guanidino, optionally substituted aryl (for example, phenyl, p-tolyl and the like), heteroaryl (for example, pyridyl, furyl and the like), heteroaryl lower alkyl (for example, picolyl and the like), optionally substituted aryl lower alkyl (for example, benzyl, 4-methylbenzyl, 4-fluorobenzyl and the like), aryl lower alkyloxy (for example, benzyloxy and the like), aryl lower alkylthio (for example, benzylthio and the like), cyano, isocyano, hydroxylamino, mercapto, lower alkylthio (for example, methylthio and the like), carbamoyl, lower alkyl substituted carbamoyl (for example, N-methylcarbamoyl and the like), lower alkylsulfonyl (for example, mesyl, ethanesulfonyl and the like), optionally substituted arylsulfonyl (for example, benzenesulfonyl, 2-toluenesulfonyl, 4-toluenesulfonyl and the like), sulfamoyl, sulfoamino, formyl, lower alkyl carbonyl (for example, acetyl, propionyl, benzoyl, p-toluoyl, cyclohexylcarbonyl and the like), lower alkylcarbonyloxy (for example, acetyloxy, benzoyloxy and the like), hydrazino, arylmino (for example, anilino, toluidino, xylidino and the like), lower alkylcarbonylamino(for example, acetamide and the like), arylcarbonylamino (for example, benzamide and the like), morpholino and the like.

The term "organic residue" used in $R^6$ and $R^8$ includes all monovalent organic residues and hydrogen atom. While not limited specifically, "optionally substituted alkyl", "optionally substituted alkenyl", "optionally substituted alkynyl", "optionally substituted carbocyclic group", "optionally substituted heterocyclic group", "optionally substituted branched alkyl", "optionally substituted branched alkenyl", and the above substituents can be exemplified.

The term "organic residue" used in $R^4$, $R^5$ and $R^7$ includes all divalent organic residues and single bond, and may be any organic residue allowing the center of moiety T to be in the position specified above. While not limited specifically, a divalent group derivatized from the "organic residue" used in $R^3$ and $R^5$ described above by removing one hydrogen atom is exemplified.

Based on the findings described above, the following HIV integrase inhibitors were designed and examined for their HIV integrase inhibiting activities. The following HIV integrase inhibitors are only examples, to whose structures the present invention is not limited.

First, as a compound having a substructure represented by Formula (III):

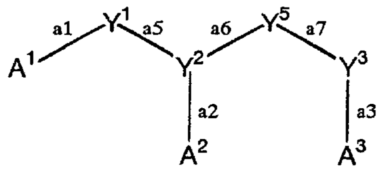

(III)

wherein each symbol is defined as described above), a compound (hereinafter referred to as Group A compound) represented by Formula:

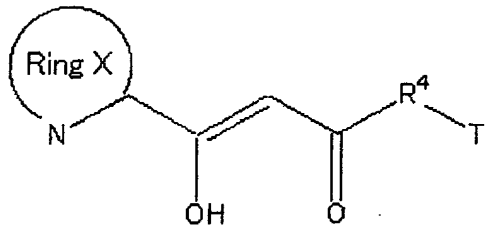

wherein $R^4$ is an organic residue, moiety T is an optionally substituted carbocyclic group, optionally substituted heterocyclic group, optionally substituted branched alkyl or optionally substituted branched alkenyl, ring X is an optionally substituted nitrogen-containing heterocyclic group, and the nitrogen atom (N) containing in ring X is an atom capable of coordinating to a divalent metal ion was synthesized.

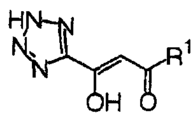
A-1

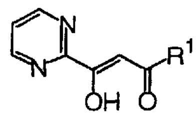
A-2

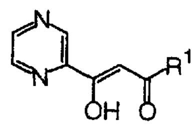
A-3

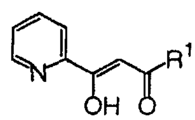
A-4

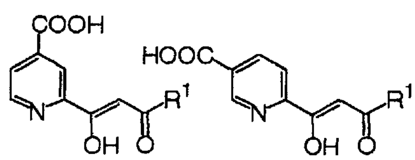
A-5 A-6

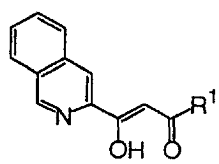
A-7

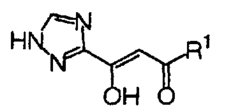
A-8

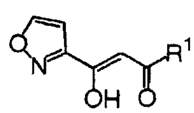
A-9

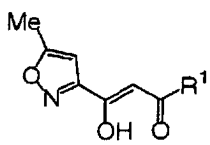
A - 10

wherein $R^1$ is 5-(4-fluorobenzyl)furan-2-yl.

Any of Group A compounds can chelate two divalent metal ions as shown below to form a 5- or 6-membered ring, whereby exhibiting a potent integrase inhibiting activity.

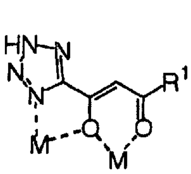
A-1

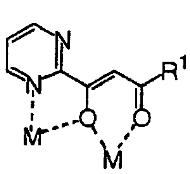
A-2

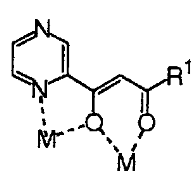
A-3

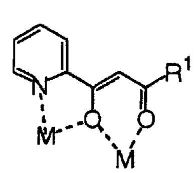
A-4

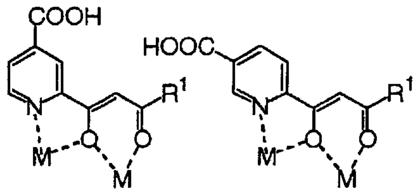

A-5 A-6

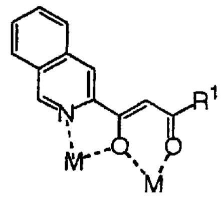

A-7

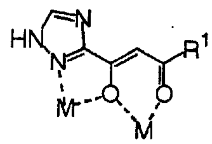

A-8

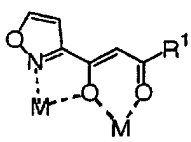

A-9

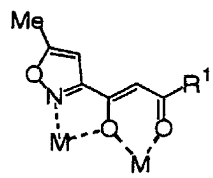

A - 10

wherein M is a divalent metal ion and $R^1$ is 5-(4-fluorobenzyl)furan-2-yl.

As a comparison, each of the following compounds (hereinafter referred to as Group B compounds) was synthesized and investigated.

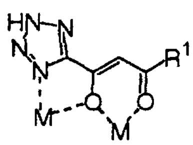

A-1

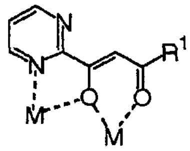

A-2

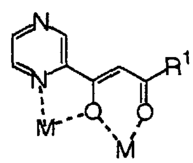

A-3

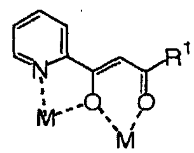

A-4

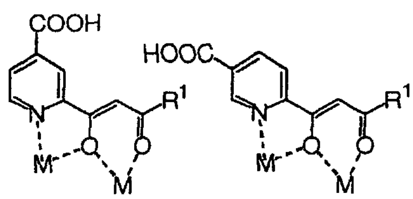

A-5 A-6

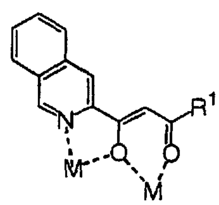

A-7

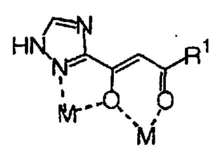

A-8

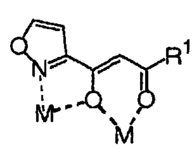

A-9

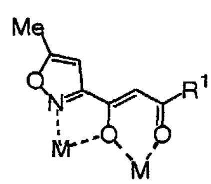

A - 10

wherein $R^1$ is 5-(4-fluorobenzyl)furan-2-yl.

Each Group B compound chelates one divalent metal ions, but can not chelate the other divalent ion as shown below. Thus, it cannot chelate both of the two divalent metal ions at the same time. Accordingly, it cannot bind strongly to the active center of an integrase, resulting in an extremely reduced integrase inhibiting activity of each Group B compound when compared with Group A compounds.

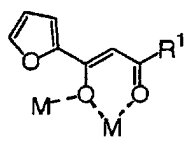
B-1

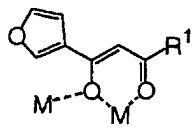
B-2

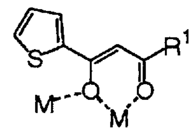
B-3

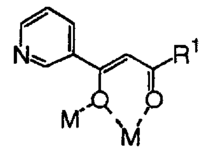
B-4

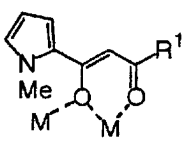
B-5

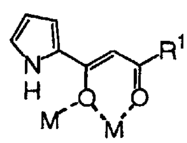
B-6

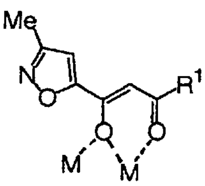
B-7

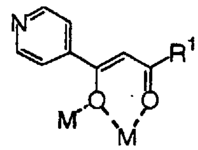
B-8

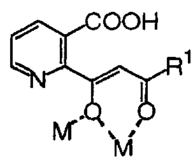
B-9

wherein M is a divalent metal ion and $R^1$ is 5-(4-fluorobenzyl)furan-2-yl.

The reasons why each Group B compound cannot show a sufficient inhibiting activity are discussed below. In any of Compounds B-2, B-4 and B-8, the coordinating atom $A^1$ is a carbon atom, and the left chelate ring in the figure cannot be formed. In any of Compound B-1, B-3 and B-7, the chelating atom $A^1$ is an oxygen or sulfur atom as a constituent of the aromatic ring, and the left chelate ring in the figure cannot be formed. In Compound B-5, a steric hindrance by a methyl group makes it impossible to form the left chelate ring in the figure. In Compound B-6, a low acidicity of the pyrrole ring leads to a difficulty in replacing a hydrogen atom with a metal under a physiological condition, which makes it impossible to form the left chelate ring in the figure. In Compound B-9, the pyridine ring can not be in a plane conformation due to the steric hindrance by a carboxyl group and hydrogen atom, and can not allow coordinating atom $A^1$ to coordinate to the metal, resulting in the difficulty in forming the left chelate ring in the figure.

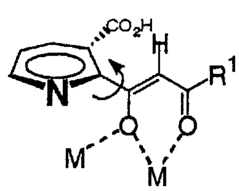

wherein $R^1$ is 5-(4-fluorobenzyl)furan-2-yl.

Based on the understandings discussed above, a compound represented by Formula:

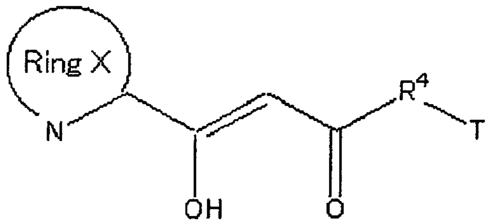

wherein each symbol is defined as described above is proven to be a compound capable of coordinating to both of two divalent metal ions and can inhibit an enzyme having the two divalent metal ions as an active center.

Subsequently, as a compound capable of coordinating to both of two divalent metal ions other than those shown above, each of a (hereinafter referred to as Group C compounds) compound represented by Formula:

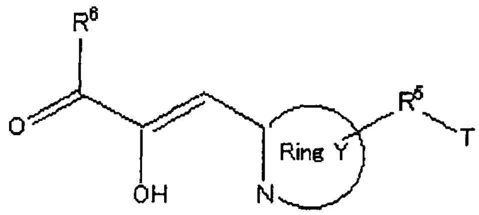

wherein $R^5$ and $R^6$ are each independently an organic residue, moiety T is an optionally substituted carbocyclic group, optionally substituted heterocyclic group, optionally substituted branched alkyl or optionally substituted branched alkenyl, ring Y is an optionally substituted nitrogen-containing heterocyclic group, and the nitrogen atom (N) containing in ring Y is an atom capable of coordinating to a divalent metal ion and a compound represented by Formula:

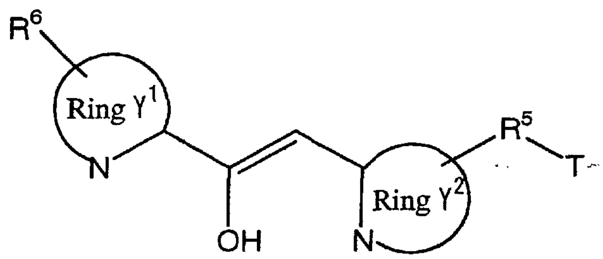

wherein $R^5$ and $R^6$ are each independently an organic residue, moiety T is an optionally substituted carbocyclic group, optionally substituted heterocyclic group, optionally substituted branched alkyl or optionally substituted branched alkenyl, each of ring $Y^1$ and ring $Y^2$ is an optionally substituted nitrogen-containing heterocyclic group, and the nitrogen atom (N) containing in ring $Y^1$ and ring $Y^2$ is an atom capable of coordinating to a divalent metal ion was synthesized.

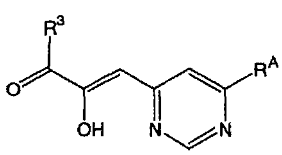
C-1

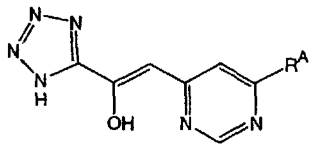
C-2

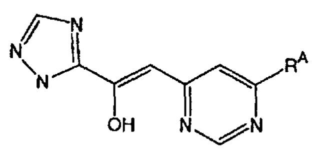
C-3

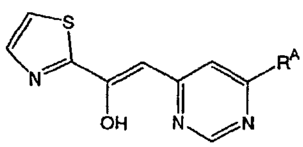
C-4

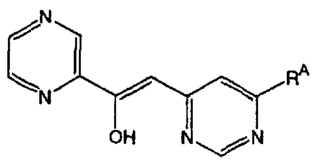
C-5

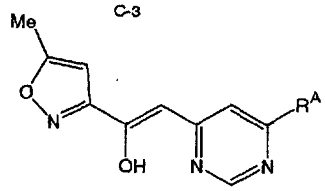
C-6

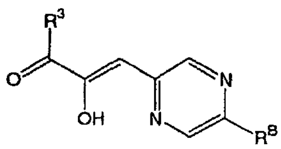
C-7

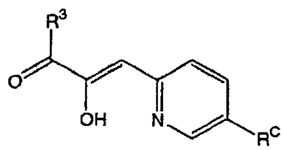
C-8

wherein $R^2$ is hydroxy, each of $R^A$ and $R^B$ is phenethyl, and $R^C$ is benzyloxy.

Any of Group C compounds can chelate two divalent metal ions as shown below to form a 5- or 6-membered ring, whereby exhibiting a potent integrase inhibiting activity.

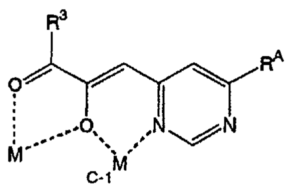
C-1

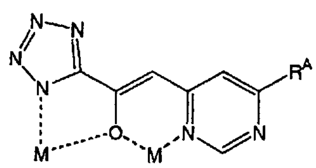
C-2

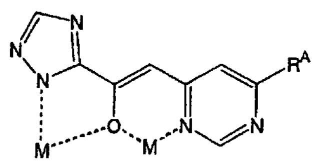
C-3

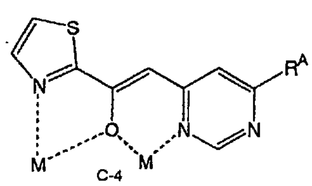
C-4

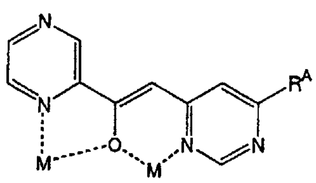
C-5

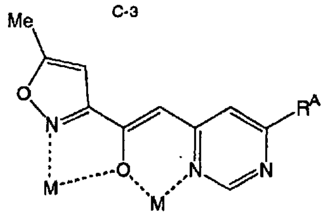
C-6

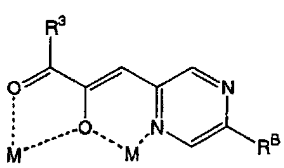
C-7

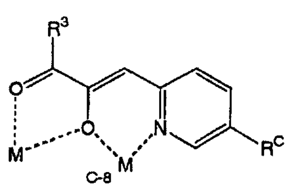
C-8

wherein M is a divalent metal ion, $R^2$ is hydroxy, each of $R^A$ and $R^B$ is phenethyl, and $R^C$ is benzyloxy.

As a comparison, each of the following compounds (hereinafter referred to as Group D compounds) was synthesized and investigated.

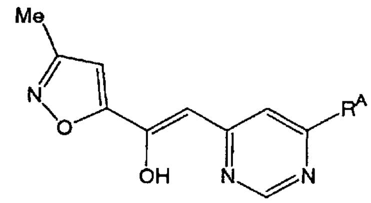

D-1

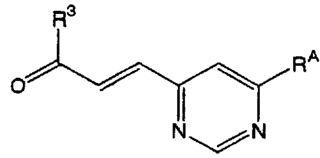

D-2

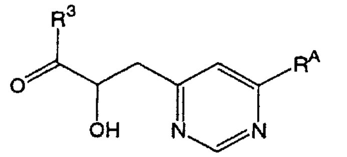

D-3

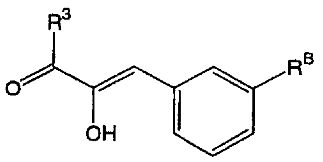

D-4

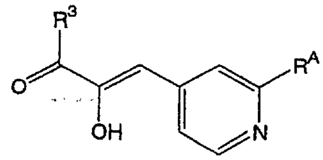

D-5

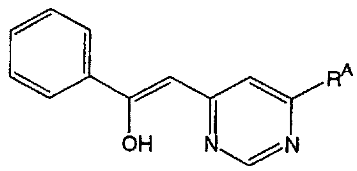

D-6

wherein $R^A$ is phenethyl, $R^B$ is benzyloxy and $R^C$ is hydroxy.

Each Group D compound cannot chelate both of the two divalent metal ions as shown below. Accordingly, it cannot bind strongly to the active center of an integrase, resulting in an extremely reduced integrase inhibiting activity of each Group D compound when compared with Group C compounds.

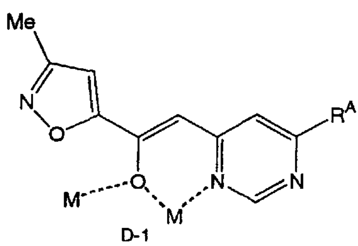

D-1

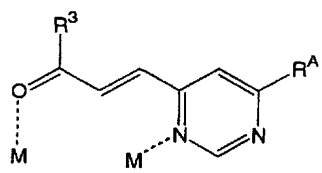

D-2

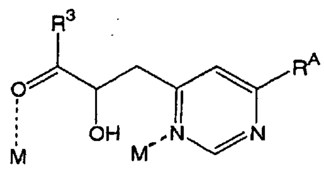

D-3

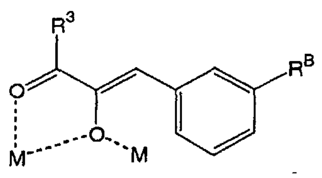

D-4

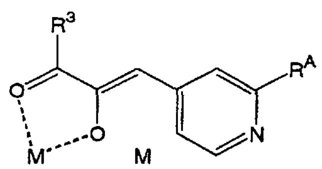

D-5

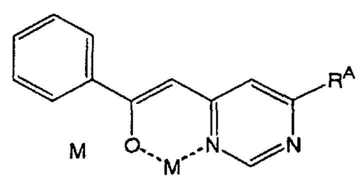

D-6

wherein $R^A$ is phenethyl, $R^B$ is benzyloxy and $R^C$ is hydroxy.

The reasons why each Group D compound cannot exert a sufficient inhibiting activity are discussed below. In Compound D-1, the coordinating atom $A^1$ is an oxygen atom as a constituent of the aromatic ring, and the left chelate ring in the figure cannot be formed. In Compound D-2, the coordinating atom $A^1$ is absent, and the both chelate rings cannot be formed. In Compound D-3, the coordinating atom $A^2$ is out of the plane since all of bonds a2, a5 and a6 are single bonds, and a low acidicity of the hydroxyl group leads to a difficulty in replacing a hydrogen atom with a metal atom under a physiological condition, resulting in the difficulty in forming both chelate rings. In Compound D-4, the coordinating atom $A^3$ is a carbon atom, and the right chelate ring in the figure cannot be formed. Similar condition can be applied to D-5 and D-6.

Based on the understandings discussed above, each of a compound represented by Formula:

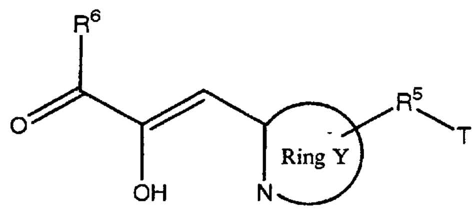

wherein each symbol is defined as described above and a compound represented by Formula:

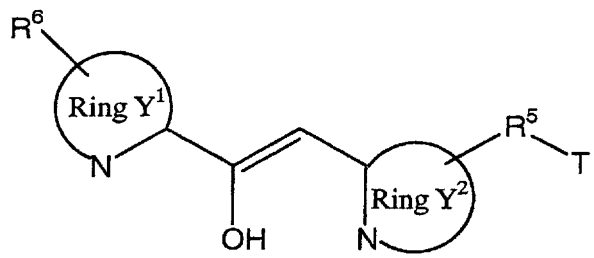

wherein each symbol is defined as described above is proven to be a compound which can coordinate to both of two divalent metal ions and can inhibit an enzyme having the two divalent metal ions as an active center.

Moreover, as a compound capable of coordinating to both of two divalent metal ions other than those shown above, each of a compound (hereinafter referred to as Group E compounds) represented by Formula:

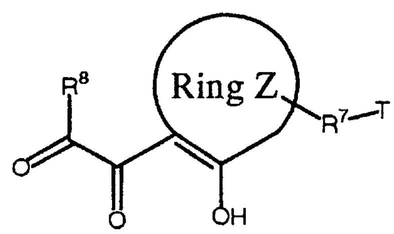

wherein $R^7$ and $R^8$ are each independently an organic residue, moiety T is an optionally substituted carbocyclic group, optionally substituted heterocyclic group, optionally substituted branched alkyl or optionally substituted branched alkenyl, ring Z is an optionally substituted carbocyclic ring or optionally substituted heterocyclic ring) and a compound represented by Formula:

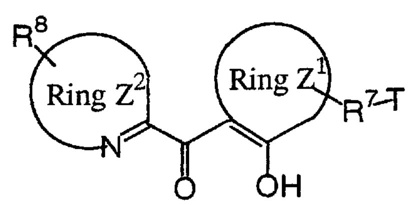

wherein $R^7$ and $R^8$ are each independently an organic residue, moiety T is an optionally substituted carbocyclic group, optionally substituted heterocyclic group, optionally substituted branched alkyl or optionally substituted branched alkenyl, ring $Z^1$ is an optionally substituted carbocyclic ring or optionally substituted heterocyclic ring, ring $Z^2$ is an optionally substituted nitrogen-containing heterocyclic ring, and the nitrogen atom (N) containing in ring $Z^2$ is an atom capable of coordinating to a divalent metal ion were synthesized.

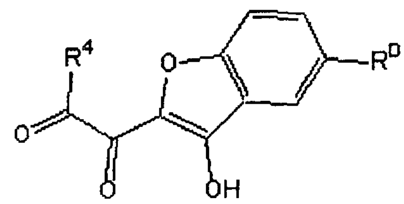

E-1 $R^4$ = OMe, $R^D$ = p-fluorobenzyloxy
E-2 $R^4$ = OH, $R^D$ = p-fluorobenzyloxy
E-5 $R^4$ = OMe, $R^D$ = p-fluorobenzyl
E-6 $R^4$ = OMe, $R^D$ = p-fluorobenzyl

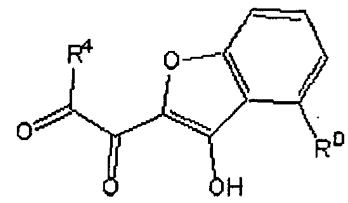

E-3 $R^4$ = OMe, $R^D$ = p-fluorobenzyloxy
E-4 $R^4$ = OH, $R^D$ = p-fluorobenzyloxy

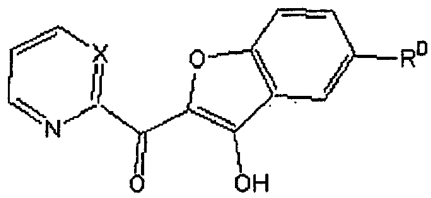

E-7 X = N, $R^D$ = p-fluorobenzyl
E-8 X = CH, $R^D$ = p-fluorobenzyl

wherein $R^4$ is hydroxy, X is N or CH, $R^D$ is p-fluorobenzyloxy or p-fluorobenzyl.

Any of Group E compounds can chelate two divalent metal ions as shown below to form a 5- or 6-membered ring, thereby exhibiting a potent integrase inhibiting activity.

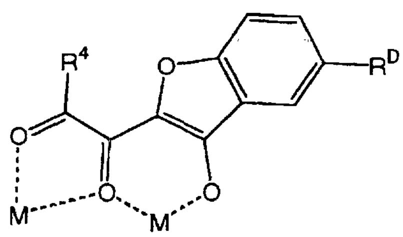

E-1

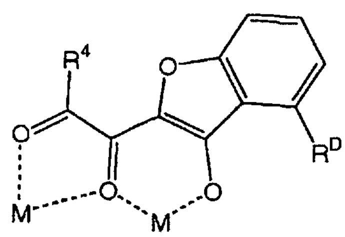

E-2

wherein $R^4$ is hydroxy and $R^D$ is benzyloxy.

Based on the understandings discussed above, each of a compound represented by Formula:

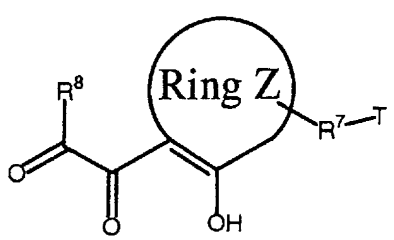

wherein each symbol is defined as described above and a compound represented by Formula:

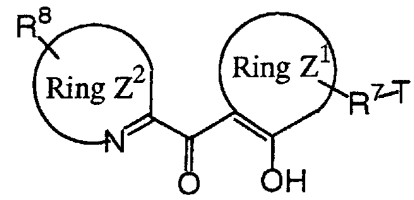

wherein each symbol is defined as described above are proven to be a compound which can coordinate to both of two divalent metal ions and can inhibit an enzyme having the two divalent metal ions as an active center.

The followings are the methods for producing the compounds thus synthesized as well as the physical data thereof. Generally, each reaction was conducted under nitrogen, and each solvent was used after drying over molecular sieves and the like. Each extract was dried over sodium sulfate, magnesium sulfate, and the like.

Group A compounds

Compound A-8

1-[5-(4-Fluorobenzyl)furan-2-yl]-3-hydroxy-3-(1H-[1,2,4]triazol-3-yl)-propenone

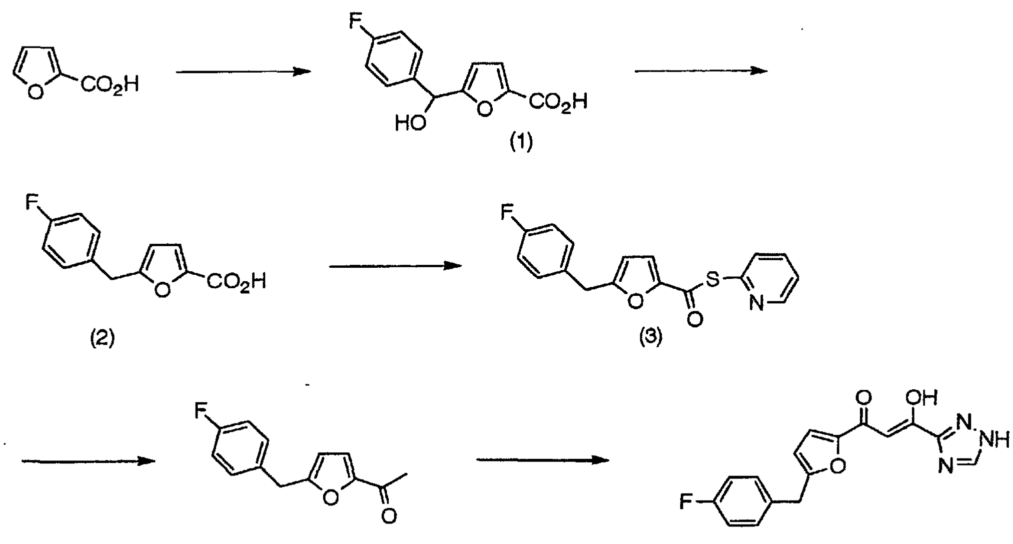

(1) (2) (3) (4) (5) Compound A-8

(1) 2-Furancarboxylic acid (5.6 g, 50 mmol) was reacted with 4-fluorobenzaldehyde (6.8 g, 55 mmol) in accordance with the method described in Tetrahedron Letters, 1979, 5, p469. A crude crystal was washed with isopropyl ether and 5-[[1-(4-fluorophenyl)-1-hydroxy]mehyl]-furane-2-carboxylic acid (8.1 g, yield: 69% ) was obtained. Melting point: 139-140°C (decomposition)

NMR ($CDCl_3$) δ: 5.88(1H, s), 6.28(1H, d, J=3.6Hz), 7.07(2H, t, J=8.7Hz), 7.25(1H, d, J=3.6Hz), 7.39-7.44(2H, m).

(2) The compound obtained above (4.72 g, 20 mmol) was reduced with trimethylchlorosilane (10.8 g, 100 mmol) and sodium iodide (15 g, 100 mmol) in accordance with the method described in Tetrahedron, 1995, 51, p11043 to obtain 5-(4-fluorobenzyl)-furan-2-carboxylic acid (3.52 g, yield: 80%) as a crystal. NMR ($d_6$-DMSO) δ: 4.05(2H, s), 6.31(1H, d, J=3.3Hz), 7.12-7.18(3H, m), 7.27-7.32(2H, m), 12.9(1H, brs).

(3) The compound obtained above (3.52 g, 16 mmol) was reacted with dipyridyl sulfide (4.2 g, 19.2 mmol) and triphenylphosphine (5.04 g, 19.2 mmol) in accordance with the method described in Bull. Chem. Soc. Japan., 1974, 47, p1777 to obtain 5-(4-flluorobenzyl)-furan-2-carboxylic acid 2-pyridylthioester (3.7 g, yield: 77%).

Melting point: 88-89°C

NMR($CDCl_3$) δ: 4.04(2H, s), 6.15(1H, d, J=3.3Hz), 7.03(2H, t, J=8.7Hz), 7.22(1H, d, J=3.3Hz), 7.22-7.26(2H, m), 7.29-7.34(1H, m), 7.70-7.79(2H, m), 8.63-8.66(1H, m).

(4) The compound obtained above (3.7 g, 12.4 mmol) was reacted with THF solution of methylmagnesium bromide (1M, 14 ml) in accordance with the method described in Bull.Chem.Soc.Japan., 1974, 47, p1777 to obtain 2-acetyl-5-(4-fluorobenzyl)-furan (2.7 g) quantitatively as an oil.
NMR($CDCl_3$) δ: 2.43(3H, s), 4.01(2H, s), 6.10(1H, d, J=3.6Hz), 7.01(2H, t, J=9.0Hz), 7.10(1H, d, J=3.6Hz), 7.18-7.23(2H, m).
(5) To a solution of the compound obtained above (1.31 g, 6 mmol) in THF (18 ml) was added dropwise a solution of lithium bistrimethylsilylamide in THF (1 M)(7.8 ml, 7.8 mmol) while keeping the temperature at -70 to 65°C. Subsequently, the reaction mixture was warmed gradually to -10°C, cooled again to -70°C. To the mixture was added dropwise a solution of 1-trityl-1H-[1,2,4-triazole]-3-carboxylic acid ethyl ester (2.99 g, 7.8 mmol) in THF(30 ml). The reaction mixture was allowed to warm gradually to room temperature, and stirred further for 1.5 hour. The reaction mixture was added to an excessive amount of an aqueous ammonium chloride, extracted with ethyl acetate, washed with brine, and dried. The solvent was evaporated, and the residue was combined with dioxane (75 ml) and 1N HCl (20 ml). The resulting mixture was heated at 80°C for 0.5 hours with stirring. Then dioxane was removed under reduced pressure, and the residue was partitioned between ethyl acetate and water. The ethyl acetate layer was washed with water and dried. The solvent was evaporated, and the residue was dissolved in ether, and extracted three times with 1N NaOH (6 ml). An alkaline extract was washed twice with ether, neutralized with 1N HCl, and extracted with ethyl acetate. The extract was washed with water and brine, and then dried. The solvent was evaporated, and the resultant crude crystal was washed with a small amount of ethyl acetate, recrystallized from ethyl acetate to obtain the title compound (1.15 g, yield: 61%). Melting point: 183-185°C

| Elemental analysis: $C_{16}H_{12}FN_3O_3$: | | | | |
|---|---|---|---|---|
| Calcd (%) | C, 61.34; | H, 3.86; | N, 13.41; | F, 6.06. |
| Found (%) | C, 61.22; | H, 3.72; | N, 13.41; | F, 6.03. |

NMR($d_6$-DMSO) δ: 4.15(2H, s), 6.47(1H, d, J=3.3Hz), 6.93(1H, s), 7.17(2H, t, J=9.0Hz), 7.31-7.37(2H, m), 7.50 (1H, d, J=3.3Hz), 8.70(1H, brs).

In accordance with the synthesis method described above, the following compounds (A-1 to A-7, A-8 to A-10) were synthesized.

Compound A-1

1-[5-(4-Fluorobenzyl)furan-2-yl]-3-hydroxy-3-(2H-tetrazol-5-yl)-propenone

Melting point: 121-123°C
Recrystallization solvent: Ether

| Elemental analysis: $C_{15}H_{11}FN_4O_3$: | | | | |
|---|---|---|---|---|
| Calcd (%) | C, 57.33; | H, 3.53; | N, 17.83; | F, 6.04. |
| Found (%) | C, 57.25; | H, 3.58; | N, 17.53; | F, 5.81. |

NMR($d_6$-DMSO) δ: 4.16(2H, s), 6.51(1H, d, J=3.6Hz), 7.05(1H, s), 7.18(2H, t, J=8.7Hz), 7.32-7.38(2H, m), 7.65(1H, d, J=3.6Hz).

Compound A-2

1-[5-(4-Fluorobenzyl)furan-2-yl]-3-hydroxy-3-(pyrimidin-2-yl)-propenone

Melting point: 77-80°C
Recrystallization solvent: Ethyl acetate-chloroform

| Elemental analysis: $C_{18}H_{13}FNO_3$ $0.2H_2O$ $0.2C_4H_8O_2$ $0.03CHCl_3$ | | | | |
|---|---|---|---|---|
| Calcd (%) | C, 64.78; | H, 4.34; | N, 8.02; | F, 5.44. |
| Found (%) | C, 65.04; | H, 4.04; | N, 7.77; | F, 5.56. |

NMR($CDCl_3$) δ: 4.07(2H, s), 6.18(1H, d, J=3.2Hz), 7.03(2H, t, J=8.8Hz), 7.18-7.22(3H, m), 7.39(1H, s), 7.39(1H, t, J=4.8Hz), 8.92(2H, d, J=4.8Hz).

Compound A-3

1-[5-(4-Fluorobenzyl)furan-2-yl]-3-hydroxy-3-(pyrazin-2-yl)-propenone

Melting point: 127-129°C
Recrystallization solvent: Ethyl acetate

| Elemental analysis: $C_{18}H_{13}FN_2O_3$ | | | | |
|---|---|---|---|---|
| Calcd (%) | C, 66.66; | H, 4.04; | N, 8.64; | F,5.86. |
| Found (%) | C, 66.73; | H, 4.05; | N, 8.63; | F,5.61. |

NMR($CDCl_3$) δ: 4.07(2H, s), 6.18(1H, d, J=3.4Hz), 7.03(2H, t, J=8.8Hz), 7.20-7.30(4H, m), 8.65-8.75(2H, m), 9.25(1H, s).

Compound A-4

1-[5-(4-Fluorobenzyl)furan-2-yl]-3-hydroxy-3-(pyridin-2-yl)-propenone

Melting point: 84-85°C
Recrystallization solvent: Ether-hexane

| Elemental analysis: $C_{19}H_{14}FNO_3$ $0.2H_2O$: | | | | |
|---|---|---|---|---|
| Calcd (%) | C, 69.80; | H, 4.44; | N, 4.28; | F, 5.81. |
| Found (%) | C, 69.76; | H, 4.34; | N, 4.34; | F, 5.73. |

NMR($CDCl_3$) δ: 4.06(2H, s), 6.16(1H, d, J=3.3Hz), 7.03(2H, t, J=8.4Hz), 7.20-7.30(3H, m), 7.32(1H, s), 7.40-7.48(1H, m), 7.87(1H, dt, J=1.5,7.5Hz), 8.11(1H, d, J=7.5Hz), 8.68-8.74(1H, m).

Compound A-5

3-(4-Carboxypyridin-2-yl)-1-[5-(4-fluorobenzyl)furan-2-yl]-3-hydroxy-propenone

Melting point: 208-210°C
Recrystallization solvent: Isopropyl ether

| Elemental analysis: $C_{20}H_{14}FNO_5$: | | | | |
|---|---|---|---|---|
| Calcd (%) | C, 65.40; | H, 3.84; | N, 3.81; | F, 5.17. |
| Found (%) | C, 65.14; | H, 3.79; | N, 3.90; | F, 4.95. |

NMR($CDCl_3$) δ: 4.09(2H, s), 6.25(1H, d, J=3.6Hz), 7.03(2H, t, J=8.4Hz), 7.21-7.32(3H, m), 7.65(1H, s), 7.96-8.02(1H, m), 8.56(1H, brs), 8.85(1H, d, J=5.1Hz).

Compound A-6

3-(5-Carboxypyridin-2-yl)-1-[5-(4-fluorobenzyl)furan-2-yl]-3-hydroxy-propenone

Melting point: 196-198°C
Recrystallization solvent: Isopropyl ether

| Elemental analysis: $C_{20}H_{14}FNO_5$ $0.2H_2O$ | | | | |
|---|---|---|---|---|
| Calcd (%) | C, 64.76; | H, 3.91; | N, 3.78; | F, 5.12. |

(continued)

| Elemental analysis: $C_{20}H_{14}FNO_5$ 0.2$H_2O$ | | | | |
|---|---|---|---|---|
| Found (%) | C, 64.95; | H, 3.73; | N, 3.93; | F, 4.99. |

NMR($CDCl_3$) δ: 4.08(2H, s), 6.18(1H, d, J=3.6Hz), 7.03(2H, t, J=9.0Hz), 7.20-7.32(3H, m), 7.37(1H, s), 8.20(1H, d, J=8.4Hz), 8.51(1H, dd, J=8.4, 1.8Hz), 9.34(1H, brs).

Compound A-7

1-[5-(4-Fluorobenzyl)furan-2-yl]-3-hydroxy-3-(isoquinolin-3-yl)-propenone

Melting point: 154-156°C
Recrystallization solvent: Ethyl acetate

| Elemental analysis: $C_{23}H_{16}FNO_3$ 0.1$H_2O$: | | | | |
|---|---|---|---|---|
| Calcd (%) | C, 73.63; | H, 4.35; | N, 3.73; | F,5.06. |
| Found (%) | C, 73.38; | H, 4.32; | N, 3.80; | F,5.11. |

NMR($CDCl_3$) δ: 4.08(2H, s), 6.16(1H, d, J=3.6Hz), 7.03(2H, t, J=9.0Hz), 7.20-7.30(3H, m), 7.44(1H, s), 7.70-7.82(2H, m), 7.95-8.15(2H, m), 8.52(1H, s), 9.29(1H, s).

Compound A-9

1-[5-(4-Fluorobenzyl)furan-2-yl]-3-hydroxy-3-(isoxazol-3-yl)-propenone

Melting point: 50-52°C
Recrystallization solvent: Hexane

| Elemental analysis: $C_{17}H_{12}FNO_4$: | | | | |
|---|---|---|---|---|
| Calcd (%) | C, 65.18; | H, 3.86; | N, 4.47; | F, 6.06. |
| Found (%) | C, 65.04; | H, 3.76; | N, 4.40; | F, 5.95. |

NMR($CDCl_3$) δ: 4.04(2H, s), 6.18(1H, d, J=3.3Hz), 6.82(1H, d, J=1.8Hz), 6.92(1H, s), 7.03(2H, t, J=8.7Hz), 7.15-7.30 (3H, m), 8.52(1H, d, J=1.8Hz).

Compound A-10

1-[5-(4-Fluorobenzyl)furan-2-yl]-3-hydroxy-3-(5-methylisoxazol-3-yl)-propenone

Melting point: 95-97°C
Recrystallization solvent: Isopropanol

| Elemental analysis: $C_{18}H_{14}FNO_4$: | | | | |
|---|---|---|---|---|
| Calcd (%) | C, 66.05; | H, 4.31; | N, 4.28; | F,5.80. |
| Found (%) | C, 66.12; | H, 4.29; | N, 4.48; | F,5.65. |

NMR($CDCl_3$) δ: 2.51(3H, s), 4.04(2H, s), 6.16(1H, d, J=3.6Hz), 6.43(1H, s), 6.86(1H, s), 7.02(2H, t, J=8.4Hz), 7.18-7.24 (3H, m).

Group B compound

Compound B-1

1-[5-(4-Fluorobenzyl)furan-2-yl]-3-(furan-2-yl)-3-hydroxy-propenone

Melting point: 44-45°C
Recrystallization solvent: Isopropyl ether-hexane

| Elemental analysis: $C_{18}H_{13}FO_4$: | | | |
|---|---|---|---|
| Calcd (%) | C, 69.23; | H, 4.20; | F, 6.08. |
| Found (%) | C, 69.16; | H, 4.11; | F, 6.18. |

NMR($CDCl_3$) δ: 4.04(2H, s), 6.15(1H, d, J=3.6Hz), 6.56(1H, s), 6.58(1H, d, J=1.8Hz), 7.03(2H, t, J=8.7Hz), 7.13(1H, d, J=3.6Hz), 7.19-7.28(3H, m), 7.58-7.62(1H, m).

Compound B-2

1-[5-(4-Fluorobenzyl)furan-2-yl]-3-(furan-3-yl)-3-hydroxy-propenone

Melting point: 53-55°C
Recrystallization solvent: Isopropyl ether-hexane

| Elemental analysis: $C_{18}H_{13}FO_4$: | | | |
|---|---|---|---|
| Calcd (%) | C, 69.23; | H, 4.20; | F, 6.08. |
| Found (%) | C, 69.24; | H, 4.06; | F, 5.96. |

NMR($CDCl_3$) δ: 4.04(2H, s), 6.12-6.16(1H, m), 6.31(1H, s), 6.72-6.76(1H, m), 7.03(2H, t, J=8.7Hz), 7.13(1H, d, J=3.6Hz), 7.16-7.28(2H, m), 7.46-7.50(1H, m), 8.04-8.07(1H, m).

Compound B-3

1-[5-(4-Fluorobenzyl)furan-2-yl]-3-(thiophen-2-yl)-3-hydroxy-propenone

Melting point: 50-52°C
Recrystallization solvent: Hexane-ethyl acetate

| Elemental analysis: $C_{18}H_{13}FO_3S$: | | | | |
|---|---|---|---|---|
| Calcd (%) | C, 65.84; | H, 3.99; | F, 5.79; | S, 9.76. |
| Found (%) | C, 65.61; | H, 3.93; | F, 5.63; | S, 9.72. |

NMR($CDCl_3$) δ: 4.05(2H, s), 6.15(1H, d, J=3.3Hz), 6.52(1H, s), 7.03(2H, t, J=8.4Hz), 7.11(1H, d, J=3.3Hz), 7.12-7.19 (1H, m), 7.20-7.30(2H, m), 7.61(1H, dd, J=5.1, 0.8Hz), 7.77(1H, dd, J=5.1, 0.8Hz).

Compound B-4

1-[5-(4-Fluorobenzyl)furan-2-yl]-3-hydroxy-3-(pyridin-3-yl)-propenone

Melting point: 53-54°C
Recrystallization solvent: Isopropyl ether

| Elemental analysis: $C_{19}H_{14}FNO_3$ $0.1H_2O$ | | | | |
|---|---|---|---|---|
| Calcd (%) | C, 70.19; | H, 4.40; | N, 4.31; | F,5.84. |
| Found (%) | C, 70.25; | H, 4.30; | N, 4.44; | F,5.72. |

NMR($CDCl_3$) δ: 4.07(2H, s), 6.19(1H, d, J=3.6Hz), 6.68(1H, s), 7.04(2H, t, J=8.4Hz), 7.20-7.30(3H, m), 7.38-7.50(1H, m), 8.22(1H, d, J=8.4Hz), 8.65-8.82(1H, brs), 9.05-9.20(2H, brs).

Compound B-5

1-[5-(4-Fluorobenzyl)furan-2-yl]-3-hydroxy-3-(1-methylpyrrol-2-yl)-propenone

Melting point: 75-76°C
Recrystallization solvent: Isopropyl ether

| Elemental analysis: $C_{19}H_{16}FNO_3$ | | | | |
|---|---|---|---|---|
| Calcd (%) | C, 70.14; | H, 4.96; | N, 4.31; | F,5.84. |
| Found (%) | C, 69.94; | H, 4.95; | N, 4.25; | F,5.67. |

NMR($CDCl_3$) δ: 4.00(3H, s), 4.03(2H, s), 4.21(2H, s), 6.08-6.20(2H, m), 6.80-6.85(1H, m), 6.95-7.05(4H, m), 7.05-7.35 (2H, m). Keto form

Compound B-6

1-[5-(4-Fluorobenzyl)furan-2-yl]-3-hydroxy-3-(pyrrol-2-yl)-propenone

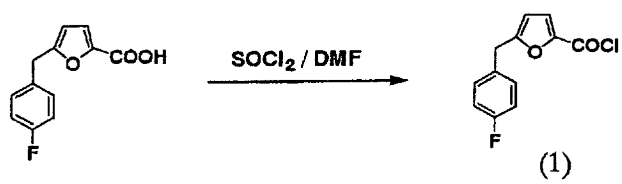

(1)

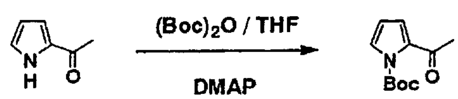

(2) (3)

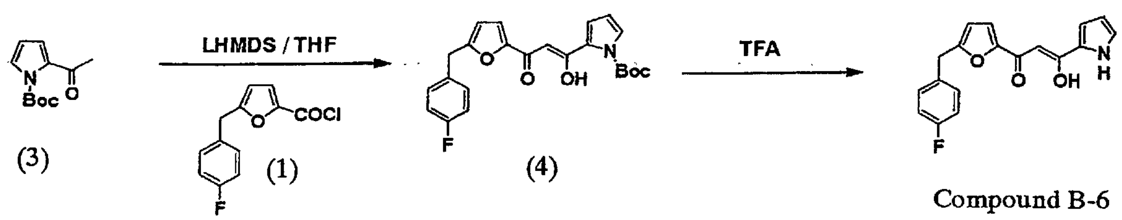

(3) (1) (4)

Compound B-6

(1) 5-(4-fluorobenzyl)-2-furancarboxylic acid (450 mg, 2 mmol) was combined with thionyl chloride (1 ml, 13.7 mmol) and DMF (0.025 ml) and stirred at room temperature for 30 minutes. An excessive thionyl chloride was removed, and the precipitated residue was washed with n-hexane to obtain a crude 5-(4-fluorobenzyl)-2-furancarboxyl chloride (480 mg).
NMR($CDCl_3$) δ:4.03(2H, s), 6.20(1H, d, J=3.6Hz), 7.03(2H, t, J=8.7Hz), 7.19-7.24(2H, m), 7.42(1H, d, J=3.6Hz).
(2) A solution of 2-acetylpyrrole (1.09 g, 10 mmol) in THF (15 ml) was treated under ice-cooling with a solution of di-tert-butyl dicarbonate (2.58 g, 12 mmol) in THF (5 ml). Subsequently, 4-dimethylaminopyridine (122 mg, 1 mmol)

was added as crystal. The mixture was stirred at room temperature for 30 minutes, and the solvent was concentrated in vacuo and then ice-water was added. The mixture was extracted with ethyl acetate, washed with water, dried, and the solvent was evaporated to obtain 2-acetyl-1-tert-butoxycarbonylpyrrole (2.1 g) as a pale yellow oil. NMR($CDCl_3$) δ:1.58(9H, s), 2.45(3H, s), 6.17(1H, t, J=3.0Hz), 6.85-6.89(1H, m), 7.30-7.34(1H, m).
(3) To a solution of 2-Acetyl-1-tert-butoxycarbonylpyrrole (313.7 mg, 1.5 mmol) in THF (10 ml) was added dropwise a solution of lithium bistrimethylsilylamide in THF (1 M) (2 ml, 2 mmol) while keeping the temperature at -65°C or lower. Then the reaction mixture was warmed gradually to 0°C, cooled again to -70°C. To the resulting mixture was added dropwise a solution of 5-(4-fluorobenzyl)-furancarboxyl chloride (358 mg, 1.5 mmol) in THF (5ml). The reaction mixture was allowed to warm gradually to room temperature, and then stirred further for 30 minutes. The reaction mixture was added to an excessive amount of an aqueous ammonium chloride, extracted with ethyl acetate, washed with brine, and dried. The solvent was removed, and the resultant yellow oil was combined with trifluoroacetic acid (2 ml), and the mixture was stirred at 30 minutes. Trifluoroacetic acid was evaporated, and the residue was extracted with ethyl acetate, washed with an aqueous sodium hydrogen carbonate followed by brine, and then dried. The solvent was removed to obtain a residue, which was recrystallized from n-hexane-isopropyl ether to obtain the title compound (200 mg, yield 43%) as a yellow crystal.
Melting point: 96-98°C
Recrystallization solvent: Hexane-isopropyl ether

| Elemental analysis: $C_{18}H_{14}FNO_3$ 0.1$H_2O$ | | | | |
|---|---|---|---|---|
| Calcd (%) | C, 69.05; | H, 4.57; | N, 4.47; | F,6.07. |
| Found (%) | C, 68.91; | H, 4.51; | N, 4.53; | F,5.71. |

NMR($CDCl_3$) δ: 4.04(2H, s), 6.12(1H, m), 6.25-6.35(1H, m), 6.39(1H, s), 6.95-7.10(4H, m), 7.15-7.30(3H, m), 9.10-9.25(1H, brs).

Compound B-7

1-[5-(4-Fluorobenzyl)furan-2-yl]-3-hydroxy-3-(3-methylisoxazol-5-yl)-propenone

Melting point: 106-107°C
Recrystallization solvent: Isopropanol

| Elemental analysis: $C_{18}H_{14}FNO_4$: | | | | |
|---|---|---|---|---|
| Calcd (%) | C, 66.05; | H, 4.31; | N, 4.28; | F,5.80. |
| Found (%) | C, 66.09; | H, 4.18; | N, 4.53; | F,5.57. |

NMR($CDCl_3$) δ: 2.39(3H, s), 4.06(2H, s), 6.19(1H, d, J=3.3Hz), 6.72(1H, d, J=3.3Hz), 6.73(1H, s), 7.03(2H, t, J=8.7Hz), 7.21-7.26(2H, m).

Compound B-8

1-[5-(4-Fluorobenzyl)furan-2-yl]-3-hydroxy-3-(pyridin-4-yl)-propenone

Melting point: 90-92°C
Recrystallization solvent: Isopropyl ether

| Elemental analysis: $C_{19}H_{14}FNO_3$ 0.1$H_2O$ | | | | |
|---|---|---|---|---|
| Calcd (%) | C, 70.19; | H, 4.40; | N, 4.31; | F,5.84. |
| Found (%) | C, 70.07; | H, 4.33; | N, 4.47; | F,5.74. |

NMR($CDCl_3$) δ: 4.07(2H, s), 6.20(1H, d, J=3.6Hz), 6.70(1H, s), 7.04(2H, t, J=8.4Hz), 7.20-7.28(3H, m), 7.75(2H, d, J=5.7Hz), 8.70-8.90(2H, brs).

Compound B-9

3-(3-Carboxypyridin-2-yl)-1-[5-(4-fluorobenzyl)furan-2-yl]-3-hydroxy-propenone

Melting point: 127-130°C
Recrystallization solvent: Ethyl acetate

| Elemental analysis: $C_{20}H_{14}FNO_5$ 0.2$H_2O$ | | | | |
|---|---|---|---|---|
| Calcd (%) | C, 64.76; | H, 3.91; | N, 3.78; | F, 5.12. |
| Found (%) | C, 65.56; | H, 3.71; | N, 3.88; | F, 5.02. |

NMR($CDCl_3$) δ: 3.99(2H, s), 6.15(1H, d, J=3.3Hz), 6.24(1H, s), 6.92-7.06(2H, m), 7.10-7.26(3H, m), 7.60-7.70(1H, m), 7.90-8.04(1H, m), 8.64-8.70(1H, m).

Group C compound

Compound c-8

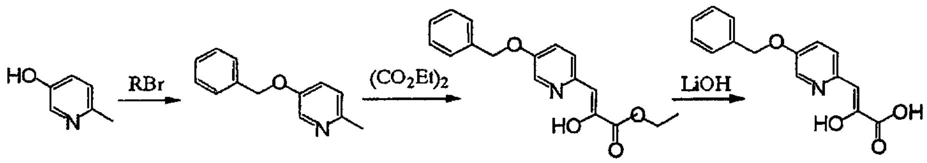


(C-8)

A solution of 5-hydroxy-2-methylpyridine (2.18 g, 20 mmol) in DMF (15 ml) was combined with benzylbromide (4.00 g, 24 mmol) and calcium carbonate (3.30 g, 24 mmol) under ice-cooling and the resulting mixture was stirred at room temperature for 2 hours. The reaction mixture was combined with an aqueous ammonium chloride, extracted with diethyl ether, washed with water, and dried. The solvent was evaporated, and the residue was subjected to a column chromatography on a silica gel, and the fraction eluted with n-hexane-ethyl acetate was concentrated to obtain a 5-benzyloxy-2-methylpyridine (2.04 g).

To a solution of 5-benzyloxy-2-methylpyridine (598 mg, 3 mmol) in THF (20 ml) was added dropwise n-butyl lithium (3mmol) at -78°C. Then to the mixture was added diethyl oxalate (4.5 g, 30 mmol) and the resulting mixture was stirred for 30 minutes. The reaction mixture was combined with an aqueous ammonium chloride, extracted with ethyl acetate, washed with water, and dried. The solvent was removed, and the residue was subjected to a column chromatography on a silica gel, and the fraction eluted with n-hexane-ethyl acetate was concentrated. The precipitated crystal was washed with diethyl ether, and dried under reduced pressure to obtain an ester form (101 mg).
1H-NMR($CDCl_3$): 1.39(3H, t, J=7.0Hz), 4.35(2H, q, J=7.0Hz), 5.14(2H, s), 6.58(1H, s), 7.18-7.43(7H, m), 8.24(1H, d, J=2.4Hz).

A solution of the ester (71 mg, 0.24 mmol) in methanol was combined with an aqueous lithium hydroxide (1N, 0.29 ml) and stirred at room temperature for 6 hours. The methanol was distilled off under reduced pressure, and water was added. A citric acid was added until the aqueous solution became acidic, and the mixture was extracted with ethyl acetate, washed with water, and dried. The solvent was distilled off under reduced pressure, and the precipitated crystal was washed with diethyl ether and dried under reduced pressure to obtain Compound C-8 (30 mg).

1H-NMR($d_6$-DMSO) δ: 5.23(2H, s), 6.66(1H, s), 7.30-7.65(7H, m), 8.38(1H, d, J=3.3Hz).

Compound c-1

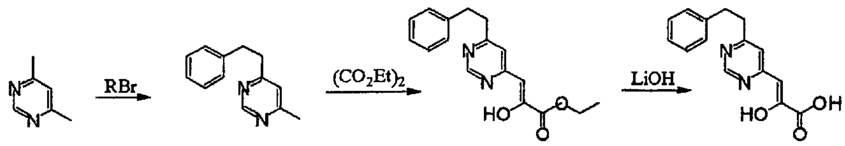


(C-1)

To a solution of 4,6-dimethylpyrimidine (1.08 g, 10 mmol) in THF (40 ml) was added dropwise n-butyl lithium at -78°C. Then the mixture was combined with benzylbromide (1.71 g, 10 mmol), warmed to 0°C, and stirred for 30 minutes. The reaction mixture was combined with an aqueous ammonium chloride, extracted with ethyl acetate, washed with water, and dried. The solvent was removed, and the residue was subjected to a column chromatography on a silica gel, and the fraction eluted with n-hexane-ethyl acetate was concentrated to obtain 4-methyl-6-phenethylpyrimidine (1.7 g).

4-Methyl-6-phenethylpyrimidine obtained above was subjected to the method similar to the synthesis of Compound C-8 described above to obtain an ester and Compound C-1.

Ester

1H-NMR($CDCl_3$): 1.40(3H, t, J=7.1Hz), 3.06(4H, s), 4.35(2H, q, J=7.1Hz), 6.39(1H, s), 6.86(1H, s), 7.12-7.35(6H, m), 8.95(1H, s).

Compound C-1

1H-NMR($d_6$-DMSO) δ: 3.00(4H, s), 6.29(1H, s), 7.15-7.40(6H, m), 8.92(1H, s).

Compound C-2

1H-NMR($d_6$-DMSO) δ:2.85-3.05(4H,m), 6.27(1H,s), 7.07(1H,s), 7.16-7.35(5H,m), 8.70(1H,s).

Compound C-3

1H-NMR($d_6$-DMSO) δ:2.90-3.05(4H,m), 6.29(1H,s), 7.08(1H,s), 7.18-7.35(5H,m), 8.35(1H,bs), 8.75(1H,s).

Compound C-4

1H-NMR($CDCl_3$) δ:3.00-3.07(4H,m), 6.44(1H,s), 6.70(1H.s), 7.18-7.29(5H,m), 7.53(1H,d,J=3.0Hz), 7.92(1H,d, J=3.0Hz), 8.67(1H,s).

Compound C-5

1H-NMR($CDCl_3$) δ:3.00-3.12(4H,m), 6.67(1H,s), 6.84(1H,s), 7.16-7.32(5H,m), 8.59(1H,s), 8.62(1H,s), 8.90(1H,s), 9.21(1H,s).

Compound C-6

1H-NMR($CDCl_3$) δ:2.48(3H,s), 2.95-3.08(4H,m), 6.16(1H,s), 6.34(1H,s), 6.71(1H,s), 7.18-7.35(5H,m), 8.77(1H,s).

Compound C-7

1H-NMR($d_6$-DMSO) δ: 3.00-3.17(4H,m), 6.62(1H,s), 7.10-7.30(5H,m), 8.44(1H,s), 8.85(1H,s).

Group D compound

The data of the compound synthesized as group D compound are described below.

Compound D-1

1H-NMR($CDCl_3$) δ:2.36(3H, s), 3.00-3.08(4H, m), 6.11(1H, s), 6.56(1H, s), 6.79(1H, s), 7.18-7.36(5H, m), 8.82(1H, s).

Compound D-2

1H-NMR($d_6$-DMSO) δ:3.06(4H, m), 7.00(1H, d, J=15.7Hz), 7.15-7.30(5H, m),7.44 (1H, d, J=15.7Hz), 7.71 (1H, s), 9.11(1H, s).

Compound D-3

1H-NMR($d_6$-DMSO) δ:2.87(1H, dd, J=14.2, 8.7Hz), 2.99(4H, s), 3.06(1H, dd, J=14.2, 4.3Hz), 4.02(1H, s), 4.42 (1H, dd, J=8.7, 4.3Hz), 7.17-7.33(6H, m), 8.98(1H, s).

Compound D-4

1H-NMR($d_6$-DMSO) δ:5.13 (2H, s), 6.38 (1H, s), 7.00 (2H, d, J=10.0Hz), 7.30-7.50 (5H, m), 7.70 (2H, d, J=10.0Hz).
Compound D-5
1H-NMR(DMSO-$d_6$) δ: 2.98(4H,s), 6.17(1H,bs), 7.17-7.30(5H,m), 7.52(1H,bs), 7.56(1H,d,J=6Hz), 8.30(1H,bs).
Compound D-6
1H-NMR($CDCl_3$) δ: 2.97(4H,m), 4.40(2/3H,s), 5.95(2/3H,s), 6.73(2/3H,d,J=1.1Hz), 7.13-7.32(16/3H,m), 7.40-7.52 (8/3H,m), 7.56-7.64(1/3H,m), 7.80-7.88(4/3H,m), 8.05-8.07(2/3H,m), 8.82(2/3H,s), 9.10(1/3H,d,J=1.4Hz).

Group E compound

Compound E-1

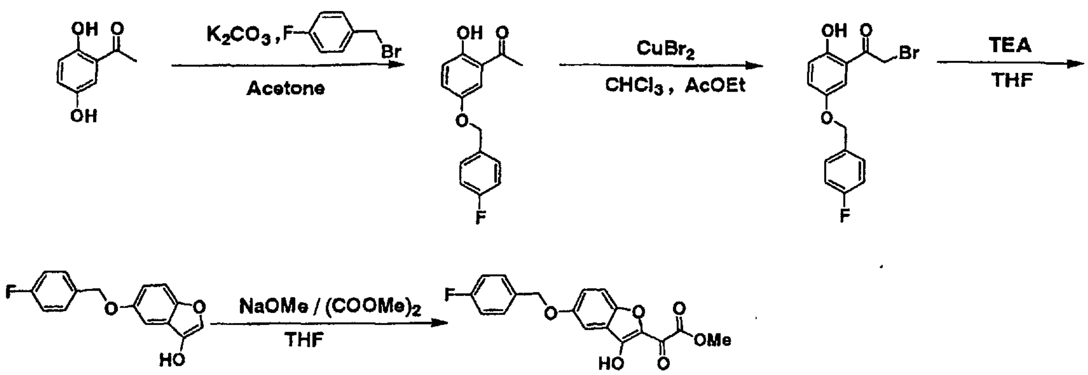


E-1

[5-(4-Fluoro-benzyloxy)-3-hydroxy-benzofuran-2-yl]-2-oxo-acetic acid methyl ester

(1) Potassium carbonate (2.0 g, 14.2 mmol) was added to a solution of 2,5-dihydroxyacetophenone (2.0 g, 13 mmol) in MeCN (30 ml) under ice-cooling and resulting mixture was stirred for 5 minutes. To the mixture was added dropwise a solution of benzyl bromide (2.55 g, 13.5 mmol) in MeCN (5 ml) over 5 minutes. The mixture was warmed to room temperature, stirred for 30 minutes, and then heated and stirred at 50°C for 60 minutes, then at 80°C for 120 minutes. The solvent was distilled off, and the residue was washed with ether to obtain 1-[5-(4-fluorobenzyloxy)-2-hydroxyphenyl]-ethanone (2.0 g, 7.7 mmol) as a crystal at the yield of 59.1%.
(2) The compound obtained above (2.0 g, 7.7 mmol) and cupric bromide (3.43 g, 15.4 mmol) was refluxed in chloroform (10 ml)-ethyl acetate (10 ml) for 3 hours. After cooling to room temperature, the mixture was filtered under reduced pressure, and the residue was washed with isopropyl ether. The residue obtained by concentrating the filtrate was subjected to a chromatography on a silica gel eluted with hexane-ethyl acetate (3:1 v/v). The fraction containing the intended substance was concentrated to 2-bromo-1-[5-(4-fluorobenzyloxy)-2-hydroxyphenyl]-ethanone (1.25 g, 3.7 mmol) as a crystal at the yield of 48.0%.
(3) To a solution of the compound obtained above (720 mg, 2.1 mmol) in THF (10 ml) was added triethylamine (430 mg, 4.2 mmol) at room temperature, and the resulting mixture was stirred for 120 minutes. The mixture was extracted with ethyl acetate, washed with water, and dried, and the solvent was distilled off to obtain an oil, which was subjected to a chromatography on a silica gel eluted with hexane-ethyl acetate (3:1 v/v). The fraction containing the intended substance was concentrated to 5-(4-fluorobenzyloxy)-benzofuran-3-ol (200 mg, 0.78 mmol) at the yield of 37.0%.
(4) A solution of the compound obtained above (129 mg, 0.5 mmol) in THF (10 ml) was combined with dimethyl oxalate (83 mg, 0.7 mmol). To the mixture was added dropwise a 1M solution of sodium methoxide (0.6 ml, 0.6 mmol) under ice-cooling and resulting mixture was stirred for 15 minutes, and then the reaction mixture was added to a cooled solution of hydrochloric acid. The mixture was extracted with ethyl acetate, washed with water, and dried, and then the solvent was distilled off to obtain the yellow residue, which was washed with isopropyl ether to obtain [5-(4-fluorobenzyloxy)-3-hydroxybenzofuran-2-yl]-2-oxo-acetic acid methyl ester (108 mg, 0.31 mmol) as a yellow crystal at the yield of 63%.

Melting point: 183-185°C
Recrystallization solvent: Isopropyl ether

| Elemental analysis:$C_{18}H_{13}FO_6$: | | | |
|---|---|---|---|
| Calcd (%) | C, 62.79; | H, 3.81; | F, 5.52. |
| Found (%) | C, 62.97; | H, 3.73; | F, 5.40. |

NMR($CDCl_3$) δ: 4.10(3H, s), 5.08(2H, s), 7.10(2H, t, J=8.4Hz), 7.20-7.30(2H, m), 7.38-7.46(3H, m).

Compound E-2

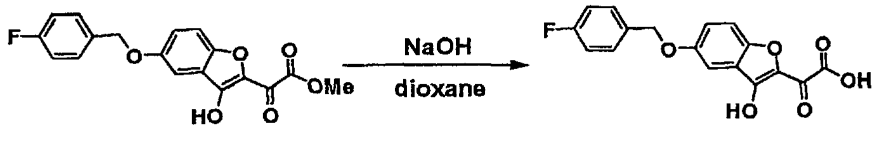

E-1 E-2

[5-(4-Fluoro-benzyloxy)-3-hydroxy-benzofuran-2-yl]-2-oxo-acetic acid

To a solution of Compound E-1 (84.0 mg, 0.24 mmol) in dioxane (5 ml) was added dropwise a 4N aqueous lithium hydroxide (122 μl, 0.49 mmol) under ice-cooling. After warming to room temperature, the mixture was combined further with an aqueous sodium hydroxide (240 μl, 0.24 mmol) and stirred for 60 minutes, and then the reaction mixture was added to a cooled solution of hydrochloric acid. The mixture was extracted with ethyl acetate, washed with water, and dried, and then the solvent was distilled off to obtain the orange residue, which was washed with ether-chloroform to obtain the title compound (30 mg, 0.09 mmol) as an orange crystal at the yield of 37.5%. Melting point: 210°C dec. Recrystallization solvent: Chloroform-ether

| Elemental analysis: $C_{17}H_{11}FO_6$ 0.3 $H_2O$, 0.2 $C_4H_{10}O$ 0.1 | | | |
|---|---|---|---|
| Calcd (%) | C, 59.32; | H, 3.81; | F, 5.04. |
| Found (%) | C, 59.10; | H, 3.69; | F, 4.94. |

NMR($d_6$-$CDCl_3$) δ: 5.08(2H, s), 7.10(2H, t, J=8.7Hz), 7.26(2H, s), 7.36-7.48(3H, m).

Compound E-3

[4-(4-Fluoro-benzyloxy)-3-hydroxy-benzofuran-2-yl]-2-oxo-acetic acid methyl ester

(1) A solution of 2,6-dihydroxyacetophenone (4.0 g, 26 mmol) in acetone (15 ml) - MeCN (20 ml) was combined with potassium carbonate (4.0 g, 28.4 mmol) under ice-cooling and the resulting mixture was stirred for 5 minutes. To the resulting mixture was added dropwise a solution of benzylbromide (5.1 g, 27.0 mmol) in MeCN (5 ml) over 5 minutes. The mixture was warmed to room temperature, stirred for 30 minutes, and then heated and stirred at 80°C for 120 minutes. After cooling to room temperature, the precipitated pale yellow crystal was filtered off. The filtrate was concentrated under reduced pressure to obtain the residue, which was washed with ether to obtain a pale yellow crystal. The crystal obtained above was combined to obtain 1-[4-(4-fluorobenzyloxy)-2-hydroxyphenyl]-ethanone (2.1 g, 8.1 mmol) as a crystal at the yield of 31.1%.

(2) The compound obtained above (2.1 g, 8.1 mmol) and cupric bromide (3.60 g, 16.1 mmol) was refluxed in chloroform (10 ml)-ethyl acetate (10 ml) for 3 hours. After cooling to room temperature, the mixture was filtered under reduced pressure, and the residue was washed with isopropyl ether. The residue obtained by concentrating the filtrate was dissolved in THF (10 ml), combined with triethylamine (430 mg, 4.2 mmol) and stirred for 120 minutes. The mixture was extracted with ethyl acetate, washed with water, and dried, and then the solvent was

distilled off to obtain an oil, which was subjected to a chromatography on a silica gel eluted with hexane-ethyl acetate (3:1 v/v). The fraction containing the intended substance was concentrated to obtain 4-(4-fluorobenzyloxy)-benzofuran-3-ol (55.0 mg, 0.21 mmol).

(3) A solution of the compound obtained above (55.0 mg, 0.21 mmol) in THF (10 ml) was combined with dimethyl oxalate (35 mg, 0.3 mmol). To the mixture was added dropwise a 1M solution of sodium methoxide (420 µl, 0.42 mmol) under ice-cooling and stirred for 15 minutes. The resulting mixture was added to a cooled solution of hydrochloric acid. The mixture was extracted with ethyl acetate, washed with water, and dried, and then the solvent was distilled off to obtain a yellow residue, which was washed with isopropyl ether to obtain [4-(4-fluorobenzyloxy)-3-hydroxybenzofuran-2-yl]-2-oxo-acetic acid-methyl ester (47 mg, 0.14 mmol) as a yellow crystal at the yield of 65%.
Melting point: 138-141°C
Recrystallization solvent: Ether

| Elemental analysis: $C_{18}H_{13}FO_6$ 0.1 $H_2O$ 0.1 $C_4H_{10}O$ | | | |
|---|---|---|---|
| Calcd (%) | C, 62.20; | H, 4.09; | F, 5.35. |
| Found (%) | C, 61.96; | H, 3.84; | F, 5.07. |

NMR($d_6$-$CDCl_3$) δ: 4.09(3H, s), 5.26(2H, s), 6.68(1H, d, J=8.1Hz), 7.02-7.16(3H, m), 7.42-7.54(3H, m).

Compound E-4

[4-(4-Fluoro-benzyloxy)-3-hydroxy-benzofuran-2-yl]-2-oxo-acetic acid

To a solution of Compound E-3 (30.0 mg, 0.09 mmol) in dioxane (5 ml) was added dropwise a 1N aqueous sodium hydroxide (170 µl, 0.17 mmol) under ice-cooling. After warming to room temperature, the precipitated yellow crystal obtained by stirring for 30 minutes was filtered off and washed with ether-chloroform to obtain the title compound (14 mg, 0.04 mmol) as a yellow crystal at the yield of 47.1%.
Melting point: 195-200°C
Recrystallization solvent: Chloroform-ether

| Elemental analysis:$C_{17}H_{10}FO_6$ 0.8 $H_2O$ 0.06 $CHCl_3$ 1.0 Na | | | |
|---|---|---|---|
| Calcd (%) | C, 54.81; | H, 3.14; | F, 5.08. |
| Found (%) | C, 54.84; | H, 3.30; | F, 4.79. |

NMR($d_6$-DMSO) δ: 5.26(2H, s), 6.81(1H, d, J=8.1Hz), 7.0(1H, d, J=8.1Hz), 7.26(2H, t, J=8.7Hz),7.45(1H, t, J=8.1Hz), 7.55-7.62(2H, m).

Compound E-5

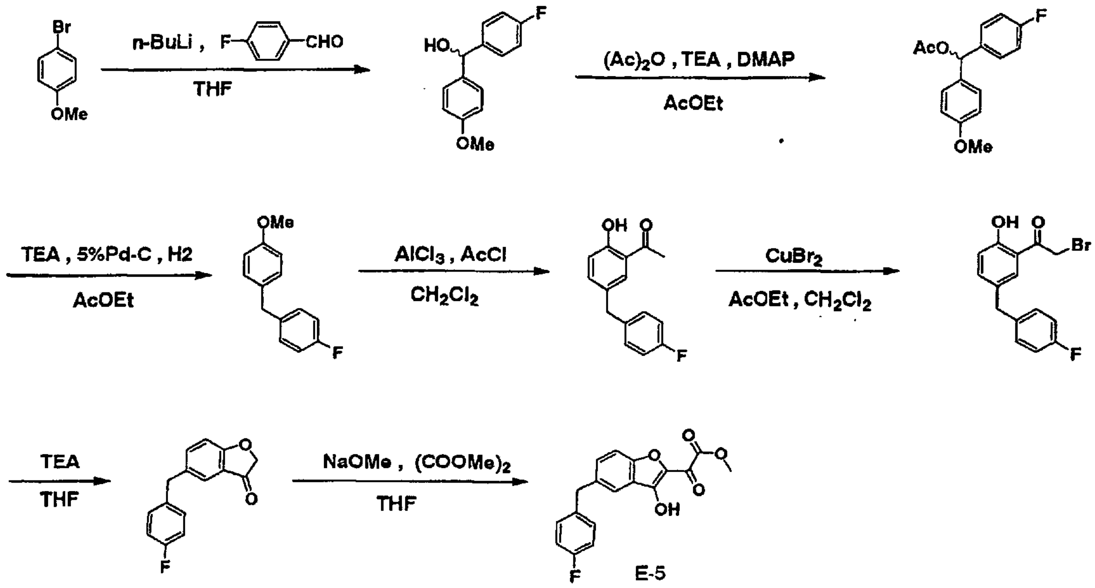


[5-(4-Fluoro-benzyl)-3-hydroxy-benzofuran-2-yl]-2-oxo-acetic acid methyl ester

Melting point: 128-130°C
Recrystallization solvent: Chloroform

| Elemental analysis: $C_{18}H_{13}FO_5$ 0.3 $CHCl_3$ | | | |
|---|---|---|---|
| Calcd (%) | C, 61.37; | H, 3.68; | F, 5.22. |
| Found (%) | C, 61.63; | H, 3.63; | F, 5.04. |

NMR($CDCl_3$) δ: 4.05(2H, s), 4.10(3H, s), 6.99(2H, t, J=8.77Hz), 7.12-7.18(2H, m), 7.40(2H, s), 7.61(1H, s).

Compund E-6

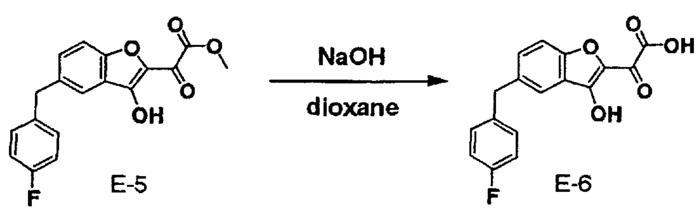


[5-(4-Fluoro-benzyl)-3-hydroxy-benzofuran-2-yl]-2-oxo-acetic acid

Melting point: 207-210°C
Recrystallization solvent: Chloroform-ethyl acetate

| Elemental analysis: $C_{17}H_{11}FO_5$ 0.1 HCl | | | |
|---|---|---|---|
| Calcd (%) | C, 64.23; | H, 3.52; | F, 5.98. |
| Found (%) | C, 64.03; | H, 3.64; | F, 5.72. |

NMR($d_6$-DMSO) δ: 4.05(2H, s), 7.12(2H, t, J=9.0Hz), 7.26-7.34(2H, m), 7.44(2H, s), 7.64(1H, s).

Compound E-7

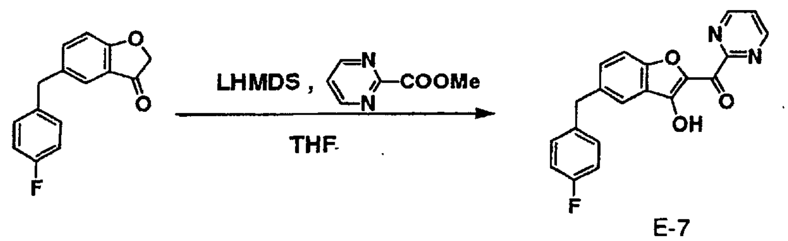

E-7

1-[5-(4-Fluoro-benzyl)-3-hydroxy-benzofuran-2-yl]-1-pyrimidin-2-yl-methanone

A solution of 5-(4-fluorobenzyl)-benzofuran-3-ol (242 mg, 1 mmol) in THF (10 ml) was cooled and treated dropwise with a solution of lithium bistrimethylsilyl amide in THF (1M) (1.3 ml, 1.3 mmol) with keeping the temperature -65°C or below. Then the reaction mixture was warmed gradually to 0°C, again cooled to -70°C, and treated dropwise with a solution of 2-methoxycarbonylpyrimidine (166 mg, 1.3 mmol) in THF (5 ml). The reaction mixture was warmed gradually and stirred at room temperature for 30 minutes. The reaction mixture was added to an aqueous ammonium chloride, extracted with ethyl acetate, washed, and dried. The solvent was distilled off, and the residue was washed with ether-chloroform to obtain the title compound (30 mg, 0.09 mmol) as a yellow crystal at the yield of 8.6%.
Melting point: 258°C dec.
Recrystallization solvent: Chloroform-ether

| Elemental analysis:$C_{20}H_{13}FN_2O_3$ 0.1 $CHCl_3$ 0.1 $C_2H_{10}O$ | | | | |
|---|---|---|---|---|
| Calcd (%) | C, 66.78; | H, 3.86; | N, 7.59; | F,5.15. |
| Found (%) | C, 66.59; | H, 3.64; | N, 7.34; | F,5.17. |

NMR($d_6$-DMSO) δ: 4.08(2H, s), 7.12(2H, t, J=8.7Hz), 7.26-7.36(2H, m), 7.44-7.56(2H, m), 7.68(1H, brs), 7.86-7.92 (1H, s),9.20(2H, d, J=5.1Hz).

Compound E-8

1-[5-(4-Fluoro-benzyl)-3-hydroxy-benzofuran-2-yl]-1-pyridine-2-yl-methanone

Melting point: 258°C dec.
Recrystallization solvent: Isopropyl ether

| Elemental analysis: $C_{21}H_{14}FNO_3$ 0.3 $C_6H_5NO_2$ | | | | |
|---|---|---|---|---|
| Calcd (%) | C, 71.26; | H, 4.07; | N, 4.74; | F, 4.94. |
| Found (%) | C, 71.12; | H, 4.11; | N, 4.96; | F, 4.96. |

NMR($CDCl_3$) δ: 4.07(2H, s), 6.99(2H, t, J=8.7Hz), 7.14-7.22(2H, m), 7.32-7.46(2H, m), 7.62-7.64(1H, m),7.68-7.76(1H, m),8.12-8.20(1H, m),8.50-8.58(1H, m),8.64-8.72(1H, m).

Experimental example 1

The inhibitory effects of the compounds of the present invention for HIV-1 integrase have been determined by the assay described below.

(1) Preparation of DNA solutions.

Substrate DNA and target DNA, which sequences were indicated below, were synthesized by Amersham Pharmacia Biotech and dissolved in KTE buffer (composition: 100 mM KCl, 1 mM EDTA, 10 mM Tris-HCl (pH 7.6)) at concentration of 2 pmol/μl and 5 pmol/μl, respectively. The DNA solutions were annealed with each complement by slowly cooling after heating.
(Substrate DNA)

```
5'- Biotin-ACC CTT TTA GTC AGT GTG GAA AAT CTC TAG CAG T-3'

3'-           GAA AAT CAG TCA CAC CTT TTA GAG ATC GTC A-5'
```

(Target DNA)

```
5'-    TGA CCA AGG GCT AAT TCA CT-Dig-3'

3'-Dig-ACT GGT TCC CGA TTA AGT GA    -5'
```

(2) Calculations of the percent inhibitions (the $IC_{50}$ values of test compounds)

Streptavidin, obtained from Vector Laboratories, was dissolved in 0.1 M carbonate buffer (composition: 90 mM $Na_2CO_3$, 10 mM $NaHCO_3$) at concentration of 40 μg/ml. After coating each well of microtiter plates (obtained from NUNC) with 50 μl of the above solution at 4 °C over night, each well was washed twice with PBS (composition: 13.7 mM NaCl, 0.27 mM KCl, 0.43 mM $Na_2HPO_4$, 0.14 mM $KH_2PO_4$) and blocked with 300 μl of 1% skim milk in PBS for 30 min. Additionally, each well was washed twice with PBS and added 50 μl of substrate DNA solution (2 pmol/μl). The microtiter plates were kept at room temperature for 30 min. Then, each well was washed twice with PBS and once with $H_2O$.

Subsequently, in the each well prepared above were added 45 μl of the reaction buffer prepared from 12 μl of the buffer (composition: 150 mM MOPS (pH 7.2), 75 mM $MnCl_2$, 50 mM 2-mercaptoethanol, 25% glycerol, 500 μg/ml bovine serum albumin-fraction V), 1 μl of target DNA (5 pmol/μl), and 32 μl of the distilled water. Additionally, 6 μl of either a test compound in DMSO or DMSO for positive control(PC) was mixed with the above reaction buffer, then 9 μl of an integrase solution (30 pmol) was added and mixed well. In the well of negative control (NC) was added 9 μl of the integrase dilution buffer (composition: 20 mM MOPS (pH7.2), 400 mM potassium glutamate, 1 mM EDTA, 0.1% NP-40, 20% glycerol, 1 mM DTT, 4M urea).

The microtiter plates were incubated at 30 °C for 1 hour. The reaction solution was removed and each well was washed twice with PBS. Subsequently, each well of the microtiter plates was filled with 100 μl of anti-digoxigenin antibody labeled with alkaline phosphatase (Sheep Fab fragment: obtained from Boehringer) and incubated at 30 °C for 1 hour. Then, each well was washed twice with 0.05% Tween20 in PBS and once with PBS. Next, 150 μl of the Alkaline phosphatase reaction buffer (composition: 10mM *p*-Nitrophenylphosphate (obtained from Vector Laboratories), 5 mM $MgCl_2$, 100 mM NaCl, 100 mM Tris-HCl (pH 9.5)) was added in each well. The microtiter plates were incubated at 30 °C for 2 hours and the reaction was terminated by the addition of 50 μl of 1 N NaOH solution. The optical density (OD) at 405 nm of each well was measured and the percent inhibition was determined by the following expression.

The percent inhibition (%) =

$$100[1-\{(C\ abs.- NC\ abs.) / (PC\ abs.- NC\ abs.)\}]$$

C abs. ; the OD of the well of the compounds
NC abs. : the OD of the negative control (NC)
PC abs. : the OD of the positive control (PC)

When the percent inhibition (%) is X% at the concentration of x μg/ml and the percent inhibition (%) is Y% at the concentration of y μg/ml, one of which is more than 50% and the other is less than 50%, $IC_{50}$ can be determined by the following expression.

$$IC_{50}(\mu g/ml) = x-\{(X-50)(x-y)/(X-Y)\}$$

The IC $_{50}$ values, the concentration of the compounds at percent inhibition 50%, are shown in the following Table 1. Compound No. in the Table 1 is the same as compound No. of the above example.

Table 1

| Compound No. | $IC_{50}$ (μg/ml) | Compound No. | $IC_{50}$ (μg/ml) |
|---|---|---|---|
| A-1 | 0.4 | C-1 | 1.5 |
| A-2 | 0.08 | C-2 | 1.4 |
| A-3 | 0.6 | C-3 | 0.50 |
| A-4 | 0.2 | C-4 | 0.60 |
| A-5 | 0.07 | C-5 | 1.6 |
| A-6 | 0.1 | C-6 | 0.84 |
| A-7 | 1.4 | C-7 | 0.61 |
| A-8 | 0.55 | C-8 | 0.46 |
| A-9 | 3.3 | D-1 | >100 |
| A-10 | 1.8 | D-2 | >100 |
| B-1 | >100 | D-3 | >100 |
| B-2 | >100 | D-4 | >100 |
| B-3 | >100 | D-5 | >100 |
| B-4 | >100 | D-6 | >100 |
| B-5 | >100 | E-1 | 2.6 |
| B-6 | >100 | E-2 | 2.6 |
| B-7 | >100 | | |
| B-8 | >100 | | |
| B-9 | >100 | | |

The following compounds were examined for their position of moiety T. Example 1 corresponds to Group A compounds, Example 2 to Group C compounds, and Examples 3 and 4 to Group E compounds. Examples 5 and 6 are inventive inhibitors which were not classified to the Groups described above, and only their data are indicated.

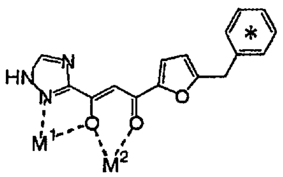

Example 1

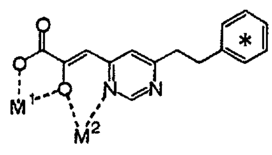

Example 2

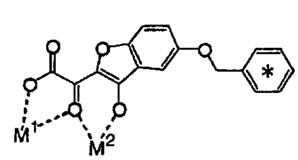

Example 3

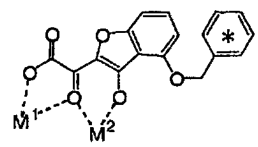

Example 4

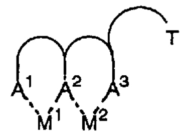

wherein * denotes the center of moiety T.

| | Distance (T-$M^3$) | Angle (T-$M^2$-O) | Torsional angle (T-$M^2$-O-$M^1$) |
|---|---|---|---|
| Example 1 | 7.22 Å | 122.1° | 151.3° |
| Example 2 | 6.18 Å | 127.3° | 143.9° |
| Example 3 | 6.14 Å | 119.8° | 156.4° |
| Example 4 | 6.25 Å | 116.8° | 153.0° |
| Example 5 | 6.62 Å | 132.1° | 152.2° |
| Example 6 | 4.53 Å | 129.5° | 139.2° |

Without using the geometrical coordinates of the divalent metal ions, the positions of moiety T was characterized as follows.

Table 3

| | Distance (T-$A^3$) | Angle (T-$A^3A^2$) | Torsional angle (T-$A^3$-$A^2$-$A^1$) |
|---|---|---|---|
| Example 1 | 5.91 Å | 159.4° | 314.4° |
| Example 2 | 6.18 Å | 145.0° | 300.0° |
| Example 3 | 6.14 Å | 154.4° | 305.3° |
| Example 4 | 6.25 Å | 149.2° | 305.6° |
| Example 5 | 6.62 Å | 154.8° | 253.8° |
| Example 6 | 4.53 Å | 141.1° | 251.0° |

Table 4

| | Distance (T-$A^2$) | Angle (T-$A^2$-$A^3$) | Torsional angle (T-$A^2$-$A^3$-$A^1$) |
|---|---|---|---|
| Example 1 | 8.82 Å | 16.6° | 48.1° |
| Example 2 | 9.51 Å | 18.4° | 59.8° |
| Example 3 | 9.04 Å | 21.0° | 50.8° |
| Example 4 | 9.18 Å | 17.6° | 38.9° |
| Example 5 | 9.61 Å | 14.9° | 87.1° |
| Example 6 | 7.74 Å | 12.4° | 76.7° |

Experiment 2

In accordance with the report by V.I.Ovcharenko et al. (Polyhedron, 16, 1279, 1997), Compound C-1 was used to synthesize various metal complexes.

A solution of Compound C-1 (270 mg, 1 mmol) in methanol (8 ml)-dioxane (8 ml) was combined with an aqueous sodium hydroxide (1N, 2 ml) at room temperature, followed by magnesium chloride (1 mmol), and stirred. Methanol and dioxane were distilled off under reduced pressure, and an insoluble complex was recovered by filtration, washed with water, dried to obtain a complex (280 mg). The results of the elemental analysis are as follows.
Anal. Calcd for $C_{30}H_{24}Mg_2N_4O_6$: C, 61.58; H, 4.13; Mg, 8.31; N, 9.57; O, 16.41
Found: C, 55.91; H, 4.72; Mg, 7.15; N, 8.82; % Water: 9.80%.

Furthermore, manganese, zinc, nickel and copper complexes were prepared similarly using chlorides.
Mn Complex
Anal. Calcd for $C_{30}H_{24}Mn_2N_4O_6$: C, 55.74; H, 3.74; Mn, 17.00; N, 8.67; O, 14.85

Found: C, 51.91; H, 4.09; N, 8.16; % Water: 6.49%.
Zn Complex
Anal. Calcd for $C_{30}H_{24}N_4O_6Zn_2$: C, 54.00; H, 3.63; Zn, 19.60; N, 8.40; O, 14.39
Found: C, 49.75; H, 3.9; N, 7.88; % Water: 6.67%.
Ni Complex
Anal. Calcd for $C_{30}H_{24}N_4Ni_2O_6$: C, 55.10; H, 3.70; Ni, 17.95; N, 8.57; O, 14.68
Found: C, 46.63; H, 4.54; N, 7.46; % Water: 14.53%.
Cu Complex

The Cu complex was subjected to mass spectroscopy. The analysis was conducted by electron spray ionization (a sample was dissolved in MeOH, and pumped at 5 µl/min). The peak [M+H]+ was measured at m/z 663.

From these results, a compound of the present invention was proven to form 2:2 complexes with divalent metal ions when combined with the divalent metal ions. Such a complex is produced as a result of the simultaneous chelating of a compound of the present invention with two metal ions. Thus, any compound capable of forming such a complex (for example, 1:2 complex, 2:4 complex, 2:2 complex) when combined with divalent metal ions may be employed in the present invention, and an enzyme inhibitor containing such a compound and a method for inhibiting an enzyme using such a compound are encompassed in the present invention.

Experiment 3

Compounds C-1, D-2, D-5 and D-6 were employed to determine UV spectra with treating dropwise with metal solutions.

The UV spectrum measurement was conducted with the cell length of 1 cm at a wavelength of 240 nm to 500 nm. Each test solution was obtained by preparing Solutions A1, A2, B and C as shown below, adding an indicated volume of Solution A1 or A2 to 1 ml of Solution B, making the total volume 10 ml with Solution C. The concentrations of Compounds C-1, D-2, D-5 and D-6 in test solutions here were all adjusted at 0.05 mmol/l, and the concentrations of $Mg^{2+}$ were as indicated in Table 5.

Solution A1 : $MgCl_2$ 4 mol/l, MOPS 50 mmol/l, NaOH 23.6 mmol/l, Solvent: Water
Solution A2 : $MgCl_2$ 1 mol/l, MOPS 50 mmol/l, NaOH 23.6 mmol/l, Solvent: Water
Solution B Compound 1 0.5 mmol/l, Solvent: DMSO

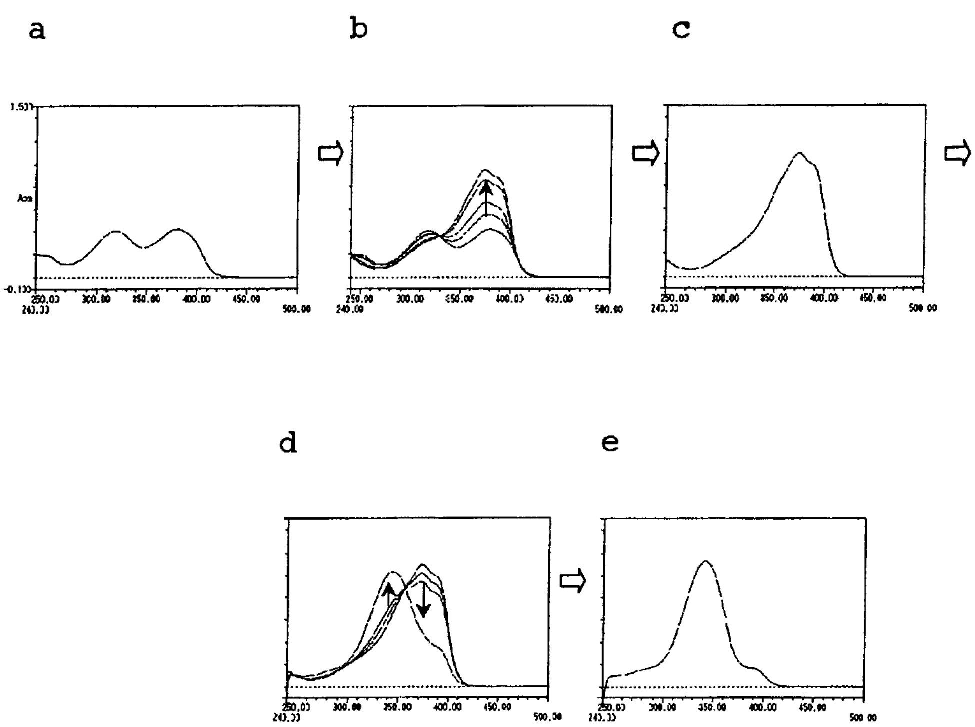

("a" is the UV spectrum curve of Sample 1, "b" shows the change in the UV spectrum curve from Sample 1 to 6, "c" is the UV spectrum curve of Sample 6, "d" shows the change in the UV spectrum curve from Sample 6 to 10, "e" is the UV spectrum curve of Sample 10, and the abscissa represents the wavelength (nm), and the ordinate represents the absorption (abs).)

The results described above indicate that Compound C-1 forms the chelate as shown below.

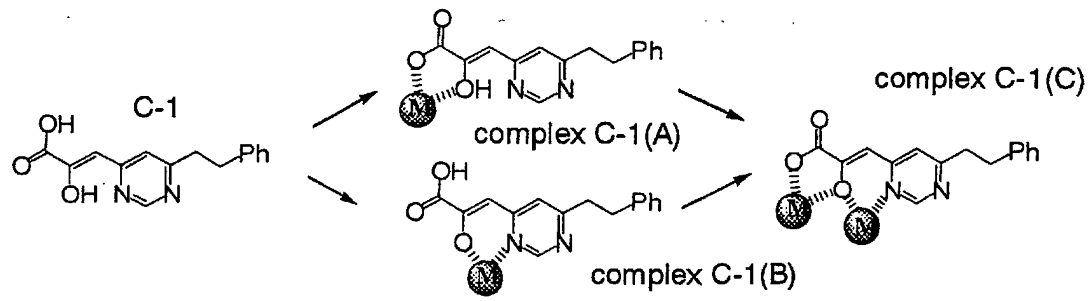


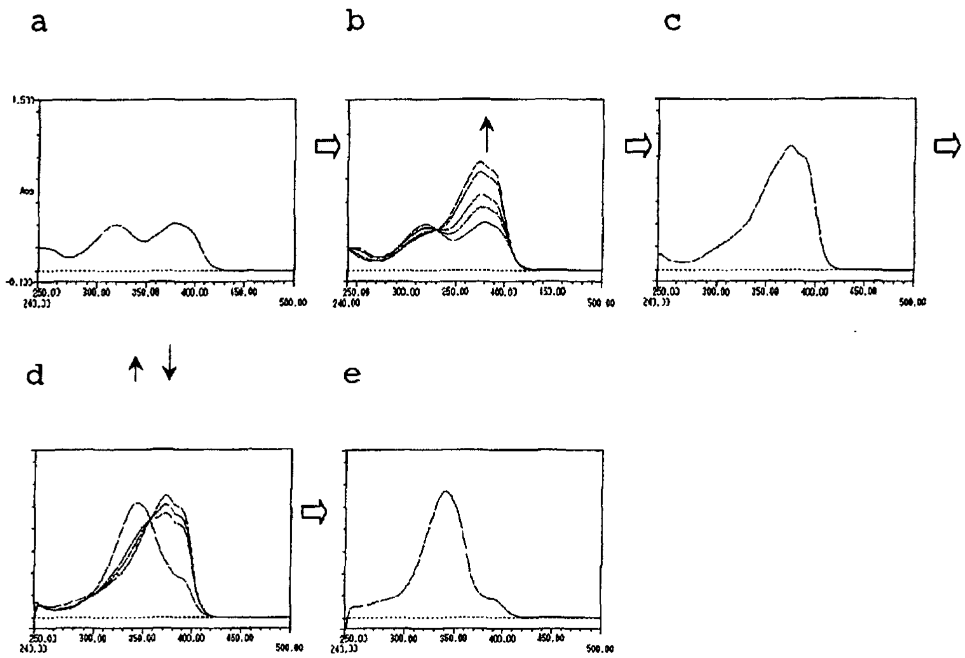


("a" is the UV spectrum curve of Sample 1, "b" shows the change in the UV spectrum curve from Sample 1 to 6, "c" is the UV spectrum curve of Sample 6, "d" shows the change in the UV spectrum curve from Sample 6 to 10, "e" is the UV spectrum curve of Sample 10, and the abscissa represents the wavelength (nm), and the ordinate represents the absorption (abs).)

The results described above indicate that Compound C-1 forms the chelate as shown below.

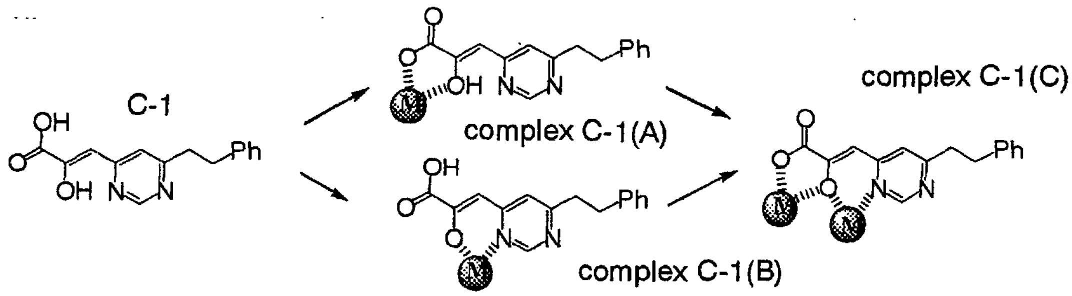

C-1

complex C-1(A)

complex C-1(B)

complex C-1(C)

Compounds D-2, D-5 and D-6

Compounds D-2, D-5 and D-6 exhibited the change in the UV spectrum curve as shown in Figure 2 (Compound D-2), Figure 3 (Compound D-3) and Figure 4 (Compound D-4). Thus, unlike to Compound C-1, the increase in the metal ion concentration did not result in the 2-step change. Compound D-2 exhibited no change in the UV spectrum curve even when the metal ion concentration was increased. This reflects the fact that Compound D-2 does not form a chelate. Each of Compounds D-5 and D-6 exhibited a 1-step change as the metal ion concentration was increased. This reflects the fact that each of Compounds D-5 and D-6 form one chelate but cannot form two chelates. Such findings are well in consistency with the fact that three heteroatoms are coordinating to two divalent metal ions as is the case of Complex C-1 (C).

$Mn^{2+}$ and $Ca^{2+}$ were tested similarly employing the chlorides and proven to exhibits the similar results. Furthermore, it was also verified that monovalent ions $Na^+$ and $K^+$ caused almost no change in the UV spectrum curve of Compound C-1.

The results of the similar test using various inventive compound also supported that a compound causing a 2-step change in the UV spectrum in the presence of a metal ion at a low concentration or a compound forming a precipitation (for example, a 2:2 complex of the compound and the divalent metal ions) in the presence of a metal ion at a low concentration is preferred especially as an inventive compound.

As an HIV integrase inhibitor, a compound which shows high affinity to Mg2+ is preferred especially.

Formulation Example

It is to be noted that the following Formulation Examples 1 to 8 are mere illustration, but not intended to limit the scope of the invention. The term "active ingredient" means the compounds of the present invention, the prodrugs thereof, their pharmaceutical acceptable salts, or their hydrates.

Formulation Example 1

Hard gelatin capsules are prepared using of the following ingredients:

| | Dose (mg/capsule) |
|---|---|
| Active ingredient | 250 |
| Starch, dried | 200 |
| Magnesium stearate | 10 |
| Total | 460 mg |

Formulation Example 2

A tablet is prepared using of the following ingredients:

| | Dose (mg/tablet) |
|---|---|
| Active ingredient | 250 |

(continued)

| | Dose (mg/tablet) |
|---|---|
| Cellulose, microcrystals | 400 |
| Silicon dioxide, fumed | 10 |
| Stearic acid | 5 |
| Total | 665 mg |

The components are blended and compressed to form tablets each weighing 665 mg.

Formulation Example 3

An aerosol solution is prepared containing the following components:

| | Weight |
|---|---|
| Active ingredient | 0.25 |
| Ethanol | 25.75 |
| Propellant 22 (chlorodifluoromethane) | 74.00 |
| Total | 100.00 |

The active ingredient is mixed with ethanol and the admixture added to a portion of the propellant 22, cooled to -30 °C and transferred to a filling device. The required amount is then fed to a stainless steel container and diluted with the reminder of the propellant. The valve units are then fitted to the container.

Formulation Example 4

Tablets, each containing 60 mg of active ingredient, are made as follows.

| | |
|---|---|
| Active ingredient | 60 mg |
| Starch | 45 mg |
| Microcrystals cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch, and cellulose are passed through a No. 45 mesh U.S. sieve, and the mixed thoroughly. The aqueous solution containing polyvinylpyrrolidone is mixed with the resultant powder, and the admixture then is passed through a No. 14 mesh U.S. sieve. The granules so produced are dried at 50°C and passed through a No. 18 mesh U.S. sieve. The sodium carboxymethyl starch, magnesium stearate, and talc, previously passed through No. 60 mesh U.S. sieve, are then added to the granules which, after mixing, are compressed on a tablet machine to yield tablets each weighing 150 mg.

Formulation Example 5

Capsules, each containing 80 mg of active ingredient, are made as follows:

| | |
|---|---|
| Active ingredient | 80 mg |
| Starch | 59 mg |
| Microcrystals cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

Formulation Example 6

Suppositories, each containing 225 mg of active ingredient, are made as follows:

| | |
|---|---|
| Active ingredient | 225 mg |
| Saturated fatty acid glycerides | 2000 mg |
| Total | 2225 mg |

The active ingredient is passed through a No. 60 mesh U.S. sieve and suspended in the saturated fatty acid glycerides previously melted using the minimum heat necessary. The mixture is then poured into a suppository mold of nominal 2g capacity and allowed to cool.

Formulation Example 7

Suspensions, each containing 50 mg of active ingredient per 5 ml dose, are made as follows:

| | |
|---|---|
| Active ingredient | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The active ingredient is passed through a No. 45 U.S. sieve, and mixed with the sodium carboxymethyl cellulose and syrup to form a smooth paste. The benzoic acid solution, flavor and color are diluted with a portion of the water and added, with stirring. Sufficient water is then added to produce the required volume.

Formulation Example 8

An intravenous formulation may be prepared as follows:

| | |
|---|---|
| Active ingredient | 100 mg |
| Isotonic saline | 1000 ml |

The solution of the above ingredients is generally administered intravenously to a subject at a rate of 1 ml per minute.

INDUSTRIAL APPLICABILITY

A compound which can coordinate to both of two divalent metal ions present in the active center of an enzyme at the same time can inhibit the action of the enzyme having two divalent metal ions as the active center and thus is useful as the inhibitor of the enzyme.

## Claims

1. A pharmaceutical composition for use as an inhibitor of an enzyme having two divalent metal ions as an active center, which comprises a compound capable of chelating both of the two divalent metal ions at the same time, a prodrug thereof, a pharmaceutically acceptable salt thereof or a solvate thereof as an active ingredient.

2. A pharmaceutical composition for use as an inhibitor according to claim 1, wherein the compound is capable of chelating both of the two divalent metal ions present in the active center of an enzyme at the same time.

3. A pharmaceutical composition for use as an inhibitor according to claim 1, wherein the compound is capable of chelating both of the two divalent metal ions at the same time and has an inhibitory activity on an enzyme having two divalent metal ions as an active center.

4. A pharmaceutical composition for use as an inhibitor according to any of claims 1 to 3, wherein the compound has atom $A^1$ capable of coordinating to one divalent metal ion, atom $A^3$ capable of coordinating to the other divalent metal ion and atom $A^2$ capable of coordinating to the both divalent metal ions at the same time.

5. A pharmaceutical composition for use as an inhibitor according to claim 4, wherein atom A1, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion.

6. A pharmaceutical composition for use as an inhibitor according to claim 4 or 5, wherein the distance between atom $A^1$ and atom $A^2$ (distance $A^1$-$A^2$) is 2.4 to 3.6 angstrom, the distance between atom $A^2$ and atom $A^3$ (distance $A^2$-$A^3$) is 2.4 to 3.6 angstrom and the angle defined by atom $A^1$, atom $A^2$ and atom $A^3$ (angle $A^1$-$A^2$-$A^3$) is 110 to 180°.

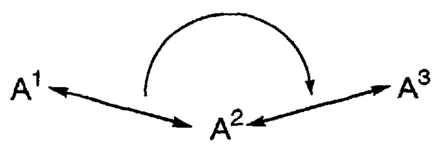

7. A pharmaceutical composition for use as an inhibitor according to any one of claims 1 to 6, wherein the compound is capable of forming a 5- or 6-membered chelate ring by chelating one divalent metal ion and also capable of forming a 5- or 6-membered chelate ring by chelating the other divalent metal ion.

8. A pharmaceutical composition for use as an inhibitor according to claim 7, wherein the compound has a substructure represented by Formula (I):

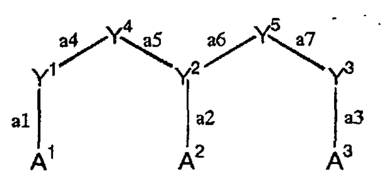

(I)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
atom $Y^4$ and atom $Y^5$ are each independently a carbon atom or nitrogen atom,
bond a1 to bond a7 are each independently a single bond or a double bond,
one of bond a2, bond a5 and bond a6 is a double bond, and the other two are single bonds,
bond a1 and bond a3 to bond a7 may each independently constitute a part of the ring,
provided that any adjacent bonds of bond a1 to bond a7 are not double bonds at the same time.

9. A pharmaceutical composition for use as an inhibitor according to claim 7, wherein the compound has a substructure represented by Formula (II):

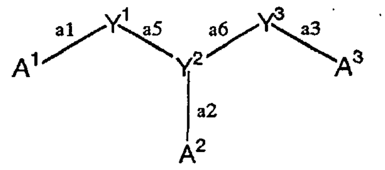

(II)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
bond a1 to bond a3, bond a5 and bond a6 are each independently a single bond or a double bond,
one of bond a2, bond a5 and bond a6 is a double bond, and the other two are single bonds,
bond a1, bond a3, bond a5 and bond a6 may each independently constitute a part of the ring,
provided that any adjacent bonds of bond a1 to bond a3, bond a5 and bond a6 are not double bonds at the same time.

**10.** A pharmaceutical composition for use as an inhibitor according to claim 7, wherein the compound has a substructure represented by Formula (III):

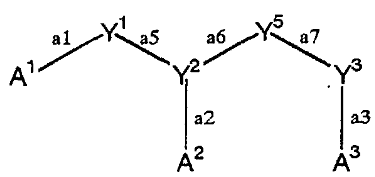

(III)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,
atom $Y^5$ is a carbon atom or nitrogen atom,
bond a1 to bond a3 and bond a5 to bond a7 are each independently a single bond or a double bond,
one of bond a2, bond a5 and bond a6 is a double bond, and the other two are single bonds,
bond a1, bond a3 and bond a5 to bond a7 may each independently constitute a part of the ring,
provided that any adjacent bonds of bond a1 to bond a3 and bond a5 to bond a7 are not double bonds at the same time.

**11.** A pharmaceutical composition for use as an inhibitor according to claim 7, wherein the compound has a substructure represented by Formula (IV):

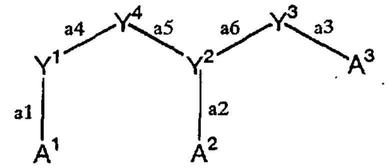

(IV)

wherein atom $A^1$, atom $A^2$ and atom $A^3$ are each independently a nitrogen atom capable of coordinating to a divalent metal ion, oxygen atom capable of coordinating to a divalent metal ion or sulfur atom capable of coordinating to a divalent metal ion,
atom $Y^1$ and atom $Y^3$ are each independently a carbon atom, nitrogen atom or sulfur atom,
atom $Y^2$ is a carbon atom,

atom $Y^4$ is a carbon atom or nitrogen atom,
bond a1 to bond a6 are each independently a single bond or a double bond,
one of bond a2, bond a5 and bond a6 is a double bond, and the other two are single bonds,
bond a1 and bond a3 to bond a6 may each independently constitute a part of the ring,
provided that any adjacent bonds of bond a1 to bond a6 are not double bonds at the same time.

**12.** A pharmaceutical composition for use as an inhibitor according to any of claims 8 to 11, wherein the structure containing a substructure represented by Formula:

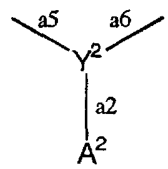

is a structure represented by Formula:

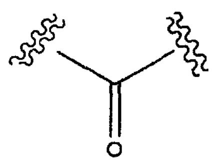
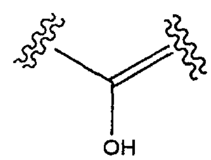
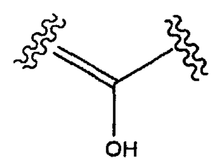
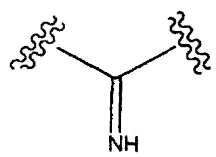
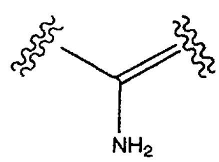
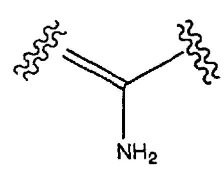
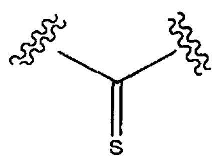
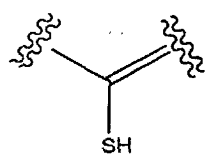
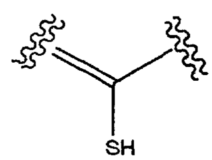

**13.** A pharmaceutical composition for use as an inhibitor according to claim 1, wherein said compound chelates two divalent metal ions and forms a complex when the compound contacts with a divalent metal salt.

**14.** A pharmaceutical composition for use as an inhibitor according to claim 1, wherein said compound chelates two divalent metal ions and forms a complex when the compound contacts with a base and a divalent metal salt.

**15.** A pharmaceutical composition for use as an inhibitor according to claim 1, wherein said compound chelates two divalent metal ions and forms a complex when the compound contacts with two equivalents or more of a base and one equivalent or more of a divalent metal salt.

**16.** A pharmaceutical composition for use as an inhibitor according to claim 1, wherein said complex is a complex in which the compound and the metal ions are present in the ratio of 2:2.

**17.** A pharmaceutical composition for use as an inhibitor according to any of claims 13 to 16, wherein said complex can be identified by the change in an elemental analysis, mass spectroscopy, X-ray crystal analysis and/or UV spectrum.

**18.** A pharmaceutical composition for use as an inhibitor according to any of claims 1 to 17, wherein the distance between the two divalent metal ions is 3.2 to 4.5 angstrom.

**19.** A pharmaceutical composition for use as an inhibitor according to any of claims 1 to 18, wherein each of the two divalent metal ions is $Mg^{2+}$ or $Mn^{2+}$.

**20.** A pharmaceutical composition for use as an inhibitor according to any of claims 1 to 19, wherein the molecular weight of the compound is 700 or less.

**21.** A pharmaceutical composition for use as an inhibitor according to any of claims 1 to 20, wherein the enzyme having two divalent metal ions as an active center is enzymes which catalyze nucleic acid related reactions.

**22.** A pharmaceutical composition for use as an inhibitor according to claim 21, wherein the an enzyme which catalyzes nucleic acid related reactions is an HIV integrase.

**23.** A pharmaceutical composition for use as an HIV integrase inhibitor which comprises a compound having a substructure according to any of claims 8 to 12 and having moiety T which satisfies the following criteria:

(1) the distance between the center of moiety T and the position of atom $A^2$ (distance T-$A^2$) is 6.0 to 11.0 angstrom,
(2) the angle defined by the center of moiety T, the position of atom $A^2$ and the position of atom $A^3$ (angle T-$A^2$-$A^3$) is 5.0 to 30.0°, and
(3) the torsional angle defined by the center of moiety T, position of atom $A^2$, position of atom $A^3$ and position of atom $A^1$ (torsional angle T-$A^2$-$A^3$-$A^1$) is 30.0 to 100.0°,

a prodrug thereof, a pharmaceutically acceptable salt thereof or a solvate thereof.

**24.** A pharmaceutical composition for use as an HIV integrase inhibitor which comprises a compound having a substructure according to any of claims 8 to 12 and having moiety T which satisfies the following criteria:

(1) the distance between the center of moiety T and the position of atom $A^3$ (distance T-$A^3$) is 3.0 to 8.0 angstrom,
(2) the angle defined by the center of moiety T, the position of atom $A^3$ and the position of atom $A^2$ (angle T-$A^3$-$A^2$) is 130.0 to 165.0°, and
(3) the torsional angle defined by the center of moiety T, position of atom $A^3$, position of atom $A^2$ and position of Atom $A^1$ (torsional angle T-$A^3$-$A^2$-$A^1$) is 240.0 to 320.0°,

a prodrug thereof, a pharmaceutically acceptable salt thereof or a solvate thereof.

**25.** A pharmaceutical composition for use as an HIV integrase inhibitor which comprises a compound having a substructure according to any of claims 8 to 12 and having moiety T which satisfies the following criteria:

(1) the distance between the center of moiety T and the position of divalent metal ion $M^2$ (distance T-$M^2$) is 3.5 to 8.5 angstrom,
(2) the angle defined by the center of moiety T, the position of divalent metal ion $M^2$ and the position of atom $A^2$ (angle T-$M^2$-$A^2$) is 110.0 to 140.0°, and
(3) the torsional angle defined by the center of moiety T, position of divalent metal ion $M^2$, position of atom $A^2$ and position of divalent metal ion $M^1$ (torsional angle T-$M^2$-$A^2$-$M^1$) is 130.0 to 165.0°,

a prodrug thereof, a pharmaceutically acceptable salt thereof or a solvate thereof.

**26.** A pharmaceutical composition for use as an HIV integrase inhibitor according to any of claims 23 to 25, wherein moiety T is a group whose volume is 50 cubic angstrom or more.

**27.** A pharmaceutical composition for use as an HIV integrase inhibitor according to any of claims 23 to 25, wherein moiety T is a group represented by Formula:

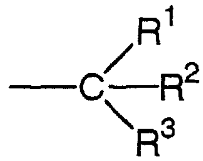

wherein 1) $R^1$, $R^2$ and $R^3$ are hydrogen, optionally substituted alkyl, optionally substituted alkenyl or optionally substituted alkynyl, and two of $R^1$, $R^2$ and $R^3$ are optionally substituted alkyl, optionally substituted alkenyl or optionally substituted alkynyl, 2) $R^1$ is hydrogen, optionally substituted alkyl, optionally substituted alkenyl or optionally substituted alkynyl, and $R^2$ and $R^3$ are taken together to form an optionally substituted 3- to 8-membered ring consisting of hydrogen atoms, carbon atoms, nitrogen atoms, sulfur atoms and oxygen atoms, or 3) $R^1$ and $R^2$ are taken together with $R^3$ to form an optionally substituted 3- to 8-membered ring consisting of hydrogen atoms, carbon atoms, nitrogen atoms, sulfur atoms and oxygen atoms, or a group represented by Formula:

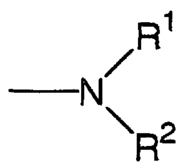

wherein 1) $R^1$ and $R^2$ are optionally substituted alkyl, optionally substituted alkenyl or optionally substituted alkynyl, or 2) $R^2$ and $R^3$ are taken together to form an optionally substituted 3- to 8-membered ring consisting of hydrogen atoms, carbon atoms, nitrogen atoms, sulfur atoms and oxygen atoms).

**28.** A pharmaceutical composition for use as an HIV integrase inhibitor according to any of claims 23 to 25, wherein moiety T is an optionally substituted carbocyclic group, an optionally substituted heterocyclic group, an optionally substituted branched alkyl or an optionally substituted branched alkenyl.

**29.** A pharmaceutical composition for use as an HIV integrase inhibitor according to any of claims 23 to 25, wherein moiety T is optionally substituted phenyl.

**30.** A method for inhibiting an enzyme having two divalent metal ions as an active center, which comprises bringing a compound capable of chelating both of the two divalent metal ions at the same time, a prodrug thereof, a pharmaceutically acceptable salt thereof or a solvate thereof into contact with said enzyme.

**31.** A method for inhibiting an enzyme having two divalent metal ions as an active center, which comprises bringing a compound capable of chelating both of the two divalent metal ions present in the active center of the enzyme at the same time, a prodrug thereof, a pharmaceutically acceptable salt thereof or a solvate thereof into contact with said enzyme.

**32.** A method for inhibiting an enzyme having two divalent metal ions as an active center, which comprises bringing a compound which is capable of chelating both of the two divalent metal ions at the same time and which has an inhibitory activity on the enzyme having two divalent metal ions as an active center, a prodrug thereof, a pharmaceutically acceptable salt thereof or a solvate thereof into contact with said enzyme.

**33.** A method for inhibiting an enzyme having two divalent metal ions as an active center, which comprises the following steps:

(1) a step for bringing a compound into contact with said enzyme, and
(2) a step for chelating said compound with both of said two divalent metal ion at the same time.

**34.** A method for inhibiting an enzyme according to any of claims 30 to 33, wherein the compound is a compound according to any of claims 1 to 12.

**35.** A method for inhibiting an enzyme according. to any of claims 30 to 33, wherein the compound is a compound according to any of claims 13 to 17.

**36.** A method for searching for an inhibitor of an enzyme having two divalent metal ions as an active center, which comprises using a search prerequisite that the distance between atom $A^1$ and atom $A^2$ (distance $A^1$-$A^2$) is 2.4 to 3.6 angstrom, the distance between atom $A^2$ and atom $A^3$ (distance $A^2$-$A^3$) is 2.4 to 3.6 angstrom and the angle defined by atom $A^1$, atom $A^2$ and atom $A^3$ (angle $A^1$-$A^2$-$A^3$) is 110 to 180°.

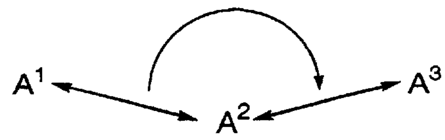


**37.** A method for searching for an inhibitor of an enzyme having two divalent metal ions as an active center, which comprises using a substructure data according to any of claims 8 to 12.

**38.** A method for searching for an inhibitor according to claim 37, which comprises using the substructure data as a search prerequisite.

**39.** A pharmaceutical composition for use as an inhibitor of an enzyme having two divalent metal ions as an active center, which comprises a compound obtained by a searching method according to any of claims 36 to 38, a prodrug thereof, a pharmaceutically acceptable salt thereof or a solvate thereof.

## INTERNATIONAL SEARCH REPORT

International application No.
PCT/JP01/04886

**A. CLASSIFICATION OF SUBJECT MATTER**

Int.Cl[7] A61K31/41, 42, 422, 44, 4409, 4433, 4709, 472, 4965, 497, 505, 506, A61P31/18, 43/00, C07B61/00 // C07D213/79, 217/18, 239/26, 241/12, 249/02, 401/06, 403/06, 405/06, 413/06, 417/06

According to International Patent Classification (IPC) or to both national classification and IPC

**B. FIELDS SEARCHED**

Minimum documentation searched (classification system followed by classification symbols)
Int.Cl[7] A61K31/00-506, A61P31/00-18, 43/00, CD07213/00-79, 217/00-18, 239/00-26, 241/00-12, 249/00-02, 401/00-06, 403/00-06, 405/00-06, 413/00-06, 417/00-06, C07B61/00

Documentation searched other than minimum documentation to the extent that such documents are included in the fields searched

Electronic data base consulted during the international search (name of data base and, where practicable, search terms used)
REGISTRY (STN), CAPLUS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | NEAMATI, N., HONG, H., SUNDER, S., MILNE, G. W., POMMIER, Y., "Potent inhibitors of human immunodeficiency virus type 1 integrase: identification of a novel four-point pharmacophore and tetracyclines as novel inhibitors", Mol. Pharmacol., (1997), Vol.52, No.6, pages 1041 to 1055 | 1-29,39<br>1-29,36-39 |
| PX<br>PY | WO 01/017968 A1 (Shionogi & Co., Ltd.), 15 March, 2001 (15.03.01), Full text (Family: none) | 1-29,39<br>1-29,36-39 |
| X<br>Y | US 5935982 A (The University of North Carolina), 10 August, 1999 (10.08.99), Full text & WO 98/38170 A1 & EP 973750 A | 1-29,39<br>1-29,36-39 |
| X<br>Y | US 5939414 A (Merck & Co., Inc.), 17 August, 1999 (17.08.99), Full text & WO 98/18473 A1 & EP 949926 A & GB 9624375 A | 1-29,39<br>1-29,36-39 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 August, 2001 (10.08.01) | 28 August, 2001 (28.08.01) |
| Name and mailing address of the ISA/<br>Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP01/04886

C (Continuation). DOCUMENTS CONSIDERED TO BE RELEVANT

| Category* | Citation of document, with indication, where appropriate, of the relevant passages | Relevant to claim No. |
|---|---|---|
| Y | KATRITZKY, A. R., GHIVIRIGA, I., ONICIU, D. C., O'FERRALL, R. A. M., WALSH, S. M., "Study of the enol-enaminone tautomerism of .alpha.-heterocyclic ketones by deuterium effects on 13C chemical shifts", J. Chem. Soc., Perkin Trans. 2, (1997), No.12, pages 2605 to 2608 especially, page 2606, chemical compounds 2, 4 | 1-29,36-39 |
| Y | ZUPET, R., TISLER, M. S., "Transformations of Alkyl Heteroarylpyruvates", J. Org. Chem., (1994), Vol.59, No.2, pages 507 to 508, especially, page 507, chemical compounds 1, 2, 3 | 1-29,36-39 |
| Y | GUDEPATI, M. S., "Excited-state behavior of 1,2-di-2-pyridyl-1,2-ethenediol (.alpha.-pyridoin) and its boric acid complexes", J. Phys. Chem., (1993), Vol.97, No.33, pages 8602 to 8607 Full text | 1-29,36-39 |
| Y | HOPFINGER, A. J., DUCA, J. S., "Extraction of pharmacophore information from high-throughput screens", Curr. Opin. Biotechnol., February, 2000, Vol.11, No.1, pages 97 to 103 Full text | 1-29,36-39 |
| A | WO 99/50245 A1 (Shionogi & Co., Ltd.), 07 October, 1999 (07.10.99), Full text & EP 1069111 A | 1-29,36-39 |

Form PCT/ISA/210 (continuation of second sheet) (July 1992)

## INTERNATIONAL SEARCH REPORT

International application No.
PCT/JP01/04886

**Box I Observations where certain claims were found unsearchable (Continuation of item 1 of first sheet)**

This international search report has not been established in respect of certain claims under Article 17(2)(a) for the following reasons:

1. ☒ Claims Nos.: 30-35
because they relate to subject matter not required to be searched by this Authority, namely:
Claim 30 to 35 relate to methods for the treatment of human disease by therapy which this International Searching Authority is not required to search, under the provisions of PCT Rule 39.1 (iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)